# EUROPEAN PATENT SPECIFICATION

(11) **EP 1 108 039 B1**
(45) Date of publication and mention of the grant of the patent: **16.04.2008**
(21) Application number: 99943978.9
(22) Date of filing: 30.08.1999
(51) Int. Cl.: C12N 15/54, C12N 9/10, C12N 1/15, C12N 1/19, C12N 1/21, C12N 5/10, A01K 67/027, A23D 9/00, A23L 1/29, A61K 31/185, A61K 8/00

(54) **ELONGASE GENES AND USES THEREOF**
ELONGASE GENE UND IHRE VERWENDUNG
GENES D'ELONGASE ET UTILISATION DE CES GENES

(30) Priority: 02.09.1998 US 145828
(43) Date of publication of application: 20.06.2001
(73) Proprietor: ABBOTT LABORATORIES, Abbott Park, IL 60064-6050 (US)
(72) Inventor: MUKERJI, Pradip, Gahanna, OH 43230 (US); LEONARD, Amanda, Eun-Yeong, Gahanna, OH 43230 (US); HUANG, Yung-Sheng, Upper Arlington, OH 43220 (US); THURMOND, Jennifer, Columbus, OH 43231 (US); KIRCHNER, Stephen, J., Westerville, OH 43081 (US); PARKER-BARNES, Jennifer, M., New Albany, OH 43054 (US); DAS, Tapas, Worthington, OH 43085 (US)
(74) Representative: Modiano, Micaela Nadia
(86) International application number: PCT/US1999/019715
(87) International publication number: WO 2000/012720

(56) References cited:
- EP-A- 0 296 751
- WO-A-88/07577
- FR-A- 2 648 347
- PATENT ABSTRACTS OF JAPAN vol. 1998, no. 08, 30 June 1998 (1998-06-30) & JP 10 070992 A (SUNTORY LTD), 17 March 1998 (1998-03-17)
- DATABASE EMBL [Online] Accession Number R63251, 30 May 1995 (1995-05-30) HILLIER L, ET AL.: "Homo sapiens clone 138518 (EST)" XP002131823
- SALEM N, ET AL.: "Arachidonic and docosahexaenoic acids are biosynthesized from their 18-carbon precursors in human infants " PROCEEDINGS OF THE NATIONAL ACADEMY OF SCIENCES, USA, vol. 93, January 1996 (1996-01), pages 49-54, XP002131822

## Description

### BACKGROUND OF THE INVENTION

### Technical Field

The subject invention relates to the identification of several genes involved in the elongation of long-chain polyunsaturated fatty acids (i.e., "elongases") and to uses thereof. In particular, the elongase enzyme is utilized in the conversion of one fatty acid to another. For example, elongase catalyzes the conversion of gamma linolenic acid (GLA) to dihomo-γ-linolenic acid (DGLA, 20:3n-6) and the conversion of stearidonic acid (STA, 18:4n-3) to (n-3)-eicosatetraenoic acid (20:4n-3). Elongase also catalyzes the conversion of arachidonic acid (AA, 20:4n-6) to adrenic acid (ADA, 22:4n-6), the conversion of eicosapentaenoic acid (EPA, 20:5n-3) to ω3-docosapentaenoic acid (22:5n-3), and the conversation of α-linolenic acid (ALA, 18:3n-3) to 20:3n-3. DGLA, for example, may be utilized in the production of other polyunsaturated fatty acids (PUFAs), such as arachidonic acid (AA) which may be added to pharmaceutical compositions, nutritional compositions, animal feeds, as well as other products such as cosmetics.

### Background Information

The elongases which have been identified in the past differ in terms of the substrates upon which they act. Furthermore, they are present in both animals and plants. Those found in mammals have the ability to act on saturated, monounsaturated and polyunsaturated fatty acids. In contrast, those found in plants are specific for saturated or monounsaturated fatty acids. Thus, in order to generate polyunsaturated fatty acids in plants, there is a need for a PUFA-specific elongase.

In both plants and animals, the elongation process is believed to be the result of a four-step mechanism (Lassner et al., The Plant Cell 8:281-292 (1996)). CoA is the acyl carrier. Step one involves condensation of malonyl-CoA with a long-chain acyl-CoA to yield carbon dioxide and a β-ketoacyl-CoA in which the acyl moiety has been elongated by two carbon atoms. Subsequent reactions include reduction to β-hydroxyacyl-CoA, dehydration to an enoyl-CoA, and a second reduction to yield the elongated acyl-CoA. The initial condensation reaction is not only the substrate-specific step but also the rate-limiting step.

As noted previously, elongases, more specifically, those which utilize PUFAs as substrates, are critical in the production of long-chain polyunsaturated fatty acids which have many important functions. For example, PUFAs are important components of the plasma membrane of a cell where they are found in the form of phospholipids. They also serve as precursors to mammalian prostacyclins, eicosanoids, leukotrienes and prostaglandins. Additionally, PUFAs are necessary for the proper development of the developing infant brain as well as for tissue formation and repair. In view of the biological significance of PUFAs, attempts are being made to produce them, as well as intermediates leading to their production, efficiently.

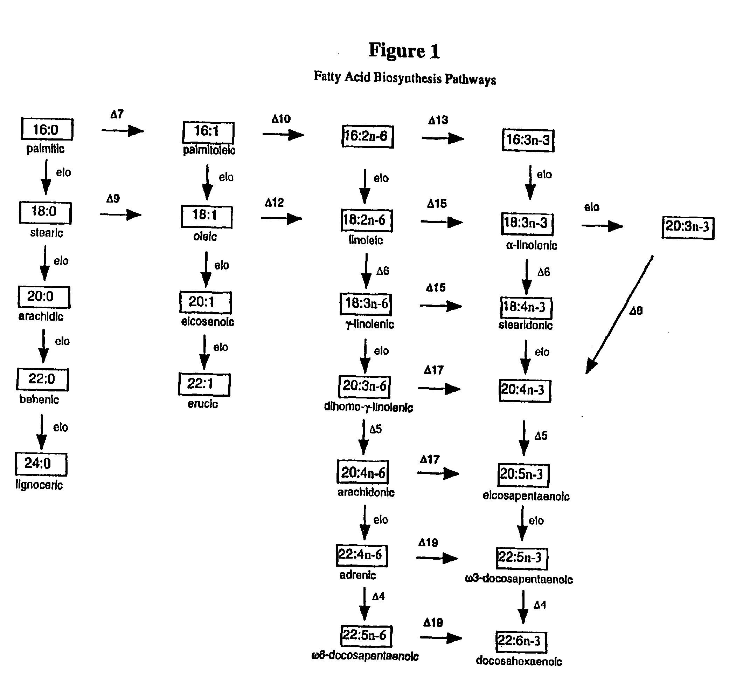

A number of enzymes are involved in PUFA biosynthesis including elongases (elo) (see Figure 1). For example, linoleic acid (LA, 18:2-Δ9,12 or 18:2n-6) is produced from oleic acid (OA, 18:1-Δ9 or 18:1n-9) by a Δ12 desaturase. GLA (18:3-Δ6,9,12) is produced from linoleic acid by a Δ6-desaturase. AA (20:4-Δ5,8,11,14) is produced from dihomo-γ-linolenic acid (DGLA, 20:3-Δ8,11,14) by a Δ5-desaturase. As noted above, DGLA is produced from GLA by an elongase.

It must be noted that animals cannot desaturate beyond the Δ9 position and therefore cannot convert oleic acid into linoleic acid. Likewise, α-linolenic acid (ALA, 18:3-Δ9,12,15 or 18:3n-3) cannot be synthesized by mammals, since they lack Δ15 desaturase activity. However, α-linolenic acid can be converted to stearidonic acid (STA, 18:4-Δ6,9,12,15) by a Δ6-desaturase (see PCT publication WO 96/13591; see also U.S. Patent No. 5,552,306), followed by elongation to (n-3)-eicosatetraenoic acid (20:4-Δ8,11,14,17 or 20:4n-3) in mammals and algae. This polyunsaturated fatty acid (i.e., 20:4-Δ8,11,14,17) can then be converted to eicosapentaenoic acid (EPA, 20:5-Δ5,8,11,14,17) by a Δ5-desaturase. Other eukaryotes, including fungi and plants, have enzymes which desaturate at carbons 12 (see PCT publication WO 94/11516 and U.S. Patent No. 5,443,974) and 15 (see PCT publication WO 93/11245). The major polyunsaturated fatty acids of animals therefore are either derived from diet and/or from desaturation and elongation of linoleic acid or α-linolenic acid. In view of the inability of mammals to produce these essential long chain fatty acids, it is of significant interest to isolate genes involved in PUFA biosynthesis from species that naturally produce these fatty acids and to express these genes in a microbial, plant or animal system which can be altered to provide production of commercial quantities of one or more PUFAs. Consequently, there is a definite need for the elongase enzyme, the gene encoding the enzyme, as well as recombinant methods of producing this enzyme. Additionally, a need exists for oils containing levels of PUFA beyond those naturally present as well as those enriched in novel PUFAs. Such oils can only be made by isolation and expression of the elongase gene.

One of the most important long chain PUFAs, noted above, is arachidonic acid (AA). AA is found in filamentous fungi and can also be purified from mammalian tissues including the liver and the adrenal glands. As noted above, AA production from DGLA is catalyzed by a Δ5-desaturase, and DGLA production from γ-linolenic acid (GLA) is catalyzed by an elongase. However, until the present invention, no elongase had been identified which was active on substrate fatty acids in the pathways for the production of long chain PUFAs and, in particular, AA, eicosapentaenoic acid (EPA), adrenic acid, docosahexaenoic acid (DHA, 22:6n-3), ω3-docosapentaenoic acid (22:5n-3) or ω6-docosapentaenoic acid (22:5n-6).

Two genes appeared to be of interest in the present search for the elongase gene. In particular, the jojoba β-ketoacyl-coenzyme A synthase (KCS), or jojoba KCS (GenBank Accession # U37088), catalyzes the initial reaction of the fatty acyl-CoA elongation pathway (i.e., the condensation of malonyl-CoA with long-chain acyl-CoA (Lassner et al., The Plant Cell 8:281-292 (1996)). Jojoba KCS substrate preference is 18:0, 20:0, 20:1, 18:1, 22:1, 22:0 and 16:0. Saccharomcyes cerevisiae elongase (ELO2) also catalyzes the conversion of long chain saturated and monounsaturated fatty acids, producing high levels of 22:0, 24:0, and also 18:0, 18:1, 20:0, 20:1, 22:0, 22:1, and 24:1 (Oh et al., The Journal of Biological Chemistry 272 (28):17376-17384 (1997); see also U.S. Patent No. 5,484,724 for a nucleotide sequence which includes the sequence of ELO2; see PCT publication WO 88/07577 for a discussion of the sequence of a glycosylation inhibiting factor which is described in Example V). The search for a long chain PUFA-specific elongase in Mortierella alpina began based upon a review of the homologies shared between these two genes and by expression screening for PUFA-elongase activity.

### SUMMARY OF THE INVENTION

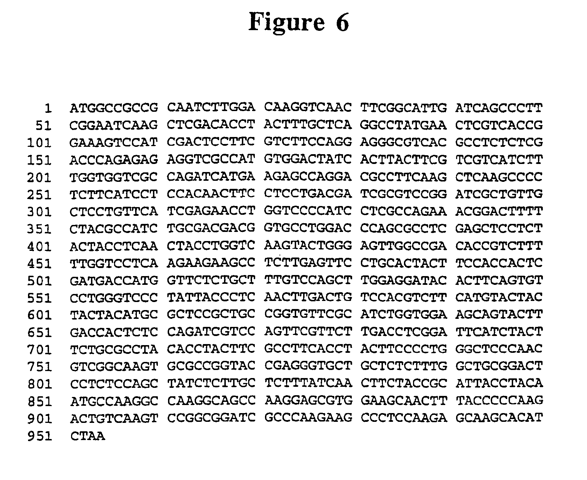

The present invention relates to an isolated nucleotide sequence corresponding to or complementary to at least about 50% of the nucleotide sequence shown in Figure 6.

The sequence encodes a functionally active elongase which utilizes a polyunsaturated fatty acid or a monounsaturated fatty acid as a substrate. In particular, the sequence may be derived from a fungus of the genus Mortierella and may specifically be isolated from Mortierella alpina.

The present invention also includes a purified protein encoded by the above nucleotide sequence.

Additionally, the present invention encompasses a method of producing an elongase enzyme comprising the steps of : a) isolating the nucleotide sequence represented by Figure 6; b constructing a vector comprising: i) the isolated nucleotide sequence operably linked to ii) a promoter; and c) introducing the vector into a host cell under time and conditions sufficient for expression of the elongase enzyme. The host cell may be a eukaryotic cell or a prokaryotic cell.

The prokaryotic cell may be, for example an E. coli cell, a cyanobacterial cell, or a B. subtilis cell. The eukaryotic cell may be, for example, a mammalian cell, an insect cell, a plant cell or a fungal cell. The fungal cell may be, for example, Saccharomyces spp., Candida spp., Lipomyces spp., Yarrowia spp., Kluyveromyces spp., Hansenula spp., Aspergillus spp., Penicillium spp., Neurospora spp., Trichoderma spp. or Pichia spp. In particular, the fungal cell may be a yeast cell such as Saccharomyces spp., in particular, Saccharomyces cerevisiae, Candida spp., Hansenula spp. or Pichia spp.

The invention also includes a vector comprising: a) a nucleotide sequence as represented by Figure 6 operably linked to b) a promoter, as well as a host cell comprising this vector. The host may be a prokaryotic cell or a eukaryotic cell. Suitable examples of prokaryotic cells include E. coli, Cyanobacteria, and B. subtilis cells. Suitable examples of eukaryotic cells include a mammalian cell, an insect cell, a plant cell and a fungal cell. The fungal cell may be, for example, Saccharomyces spp., Candida spp., Lipomyces spp., Yarrowia spp. , Kluyveromyces spp., Hansenula spp., Aspergillus spp., Penicillium spp., Neurospora spp., Trichoderma spp. and Pichia spp. In particular, the fungal cell may be, for example, a yeast cell such as, for example, Saccharomyces spp., in particular, Saccharomyces cerevisiae, Candida spp., Hansenula spp. and Pichia spp.

The present invention includes a plant cell, plant or plant tissue comprising the above-described vector, wherein expression of the nucleotide sequence of the vector results in production of at least one fatty acid selected from the group consisting of a monounsaturated fatty acid and a polyunsaturated fatty acid by the plant cell, plant or plant tissue. The polyunsaturated fatty acid may be, for example, dihomo-γ-linolenic acid (DGLA), 20:4n-3, and adrenic acid (ADA). The invention also includes one or more plant oils or fatty acids expressed by the plant cell, plant or plant tissue. Additionally, the present invention encompasses a transgenic plant comprising the above-described vector, wherein expression of the nucleotide sequence of the vector results in production of a polyunsaturated fatty acid in seeds of the transgenic plant.

Furthermore, the present invention includes a transgenic, non-human mammal whose genome comprises a DNA sequence encoding an elongase operably linked to a promoter. The DNA sequence may be represented by SEQ ID NO:1 (Figure 6).

A fluid (e.g., milk) may be produced by the transgenic, non-human wherein the fluid comprises a detectable level of at least one elongase or products thereof such as, for example, DGLA, ω6-docosapexntaenoic, acid, ADA and/or 20:4n-3 (see Figure 1).

Additionally, the present invention includes a method for producing a polyunsaturated fatty acid comprising the steps of: a) isolating said nucleotide sequence represented by SEQ ID NO:1 (Figure 6); b) constructing a vector comprising the isolated nucleotide sequence; c) introducing the vector into a host cell under time and conditions sufficient for expression of elongase enzyme encoded by the isolated nucleotide sequence; and d) exposing the expressed elongase enzyme to a "substrate" polyunsaturated fatty acid in order to convert the substrate to a "product" polyunsaturated fatty acid. The substrate polyunsaturated fatty acid may be selected from the group consisting of, for example, γ-linolenic acid (GLA), stearidonic acid (STA) and arachidonic acid (AA), and the product polyunsaturated fatty acid may be selected from the group consisting of, for example, DGLA, 20:4n-3, and ADA, respectively. The method may further comprise the step of exposing the product polyunsaturated fatty acid to at least one desaturase in order to convert the product polyunsaturated fatty acid to "another" polyunsaturated fatty acid. The product polyunsaturated fatty acid may be selected from the group consisting of, for example, DGLA, 20;4n-3, and ADA. The another polyunsaturated fatty acid may be selected from the group consisting of, for example, AA, eicosapentaenoic acid (EPA), ω6-docosapentaenoic acid, respectively, and the at least one desaturase is Δ5-desaturase, with respect to production of AA or EPA, and Δ4-desaturase, with respect to production of ω6-docosapentaenoic acid. The method may further comprise the step of exposing the another polyunsaturated fatty acid to one or more enzymes selected from the group consisting of at least one elongase and at least one additional desaturase in order to convert the another polyunsaturated fatty acid to a "final" polyunsaturated fatty acid. The final polyunsaturated fatty acid may be, for example, docosahexaenoic acid (DHA), AA, ω6-docosapentaenoic acid, or ω3-docosapentaenoic acid.

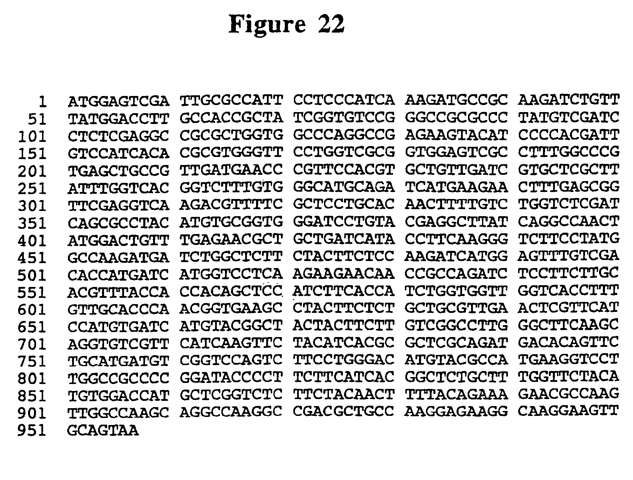

The present invention also includes an isolated nucleotide sequence corresponding to or complementary to at least about 35% of the nucleotide sequence shown in Figure 22. The sequence encodes a functionally active elongase which utilizes a polyunsaturated fatty acid as a substrate. This sequence may also be derived, for example, from a fungus of the genus Mortierella. In particular, it may be derived from M. alpina.

Additionally, the present invention includes a purified protein encoded by the above nucleotide sequence.

The present invention also includes a method of producing an elongate enzyme as described above. The sequence inserted in the vector is represented by Figure 22. The host cell may be prokaryotic or eukaryotic. Suitable examples are described above.

The present invention also includes a vector comprising: a) a nucleotide sequence as represented by Figure 22 operably linked to b) a promoter, as well as a host cell comprising this vector. Again, the host cell may be eukaryotic or prokaryotic. Suitable examples are described above.

The invention also includes a plant cell, plant or plant tissue Comprising the above vector, wherein expression of the nucleotide sequence of the vector results in production of a polyunsaturated fatty acid by the plant cell, plant or plant tissue. The polyunsaturated fatty acid may be, for example, DGLA, 20:4n-3, or ADA. Additionally, the invention includes one or more plant oils or fatty acids expressed by the plant cell, plant or plant tissue.

Furthermore, the present invention also includes a transgenic plant comprising the above vector, wherein expression of the nucleotide sequence of figure 22 of the vector results in production of a polyunsaturated fatty acid in seeds of the transgenic plant.

The invention also includes a transgenic, non-human mammal whose genome comprises a DNA sequence of figure 22 encoding an elongase operably linked to a promoter. A fluid may be produced by this transgenic, non-human mammal wherein the fluid comprises a detectable level of at least one elongase or products thereof.

The present invention also includes a method for producing a polyunsaturated fatty acid comprising the steps of: a) isolating the nucleotide sequence represented by Figure 22; b) constructing a vector comprising the isolated nucleotide sequence; c) introducing the vector into a host cell under time and conditions sufficient for expression of an elongase enzyme encoded by the isolated nucleotide sequence; and d) exposing the expressed elongase enzyme to a substrate polyunsaturated fatty acid in order to convert the substrate to a product polyunsaturated fatty acid. The substrate polyunsaturated fatty acid may be, for example, GLA, STA, or AA, the product polyunsaturated fatty acid may be, for example, DGLA, 20:4n-3, or ω6-docosapentaenoic acid, respectively. The method may further comprise the step of exposing the expressed elongase enzyme to at least one desaturase in order to convert the product polyunsaturated fatty acid to another polyunsaturated fatty acid. The product polyunsaturated fatty acid may be, for example, DGLA, 20:4n-3, or ADA, the another polyunsaturated fatty acid may be, for example, AA, EPA, or ω6-docosapentaenoic acid, respectively, and the at least one desaturase is Δ5-desaturase with respect to production of AA or EPA, and Δ4-desaturase with respect to production of ω6-docosapentaenoic acid. The method may further comprise the step of exposing the another polyunsaturated fatty acid to one or more enzymes selected from the group consisting of at least one elongase and at least one additional desaturase in order to convert the another polyunsaturated fatty acid to a final polyunsaturated fatty acid. The final polyunsaturated fatty acid may be, for example, docosahexaenoic acid, AA, ω6-docosapentaenoic acid, or ω3-docosapentaenoic acid.

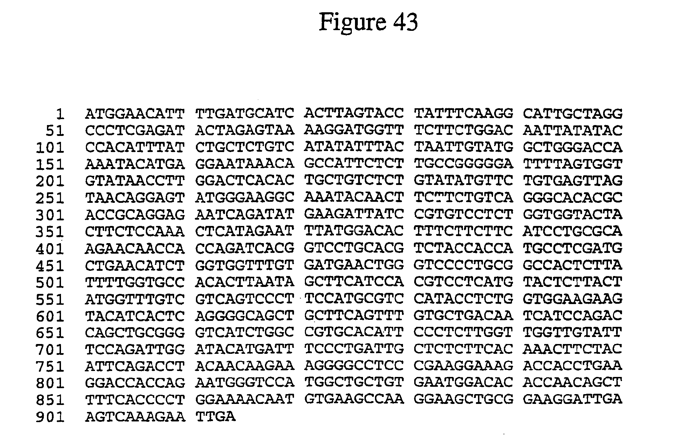

Furthermore, the present invention includes an isolated nucleotide sequence corresponding to or complementary to the nucleotide sequence shown in Figure 43.

This sequence encodes a functionally active elongase which utilizes a polyunsaturated fatty acid or a monounsaturated fatty acid as a substrate. The sequence is derived from a mammal such as, for example, a human.

The invention also includes a purified protein encoded by this nucleotide sequence.

Additionally, the invention includes method of producing an elongase enzyme comprising the steps of: a) isolating the nucleotide sequence represented by Figure 43; b) constructing a vector comprising: i) the isolated nucleotide sequence operably linked to ii) a promoter; and c) introducing said vector into a host cell under time and conditions sufficient for expression of the elongase enzyme. The host cell may be the same as that described above with respect to the corresponding methods utilizing Figures 6 or 22. The invention also includes a vector comprising: a) a nucleotide sequence as represented by Figure 43 operably linked to b) a promoter, as well as a host cell comprising this vector. The host cell may be the same as that described above.

The invention also includes a plant cell, plant or plant tissue comprising the above-described vector comprising the sequence of Figure 43 wherein expression of the nucleotide sequence of the vector results in production of at least one fatty acid selected from the group consisting of a monounsaturated fatty acid and a polyunsaturated fatty acid by said plant cell, plant or plant tissue. The polyunsaturated fatty acid may be, for example, DGLA, 20:4n-3 or ADA. The invention also includes one or more plant oils or acids expressed by the plant cell, plant or plant tissue.

The invention also includes a transgenic plant comprising the vector comprising the sequence of figure 43, wherein expression of the nucleotide sequence of the vector results in production of a polyunsaturated fatty acid in seeds of the transgenic plant.

Additionally, the present invention includes a transgenic, non-human mammal whose genome comprises a human DNA sequence encoding an elongase operably linked to a promoter. The DNA sequence is represented by Figure 43. A fluid may be produced by said transgenic, non-human mammal wherein said fluid comprises a detectable level of at least one elongase or products thereof.

The invention also encompasses a method for producing a polyunsaturated fatty acid comprising the steps of: a) isolating the nucleotide sequence represented by Figure 43; b) constructing a vector comprising said nucleotide sequence; c) introducing the vector into a host cell under time and conditions sufficient for expression of elongase enzyme encoded by the isolated nucleotide sequence; and d) exposing the expressed e7.angase enzyme to a substrate polyunsaturated fatty acid in order to convert the substrate to a product polyunsaturated fatty acid. The substrate polyunsaturated fatty acid may be, for example, GLA, STA or AA, and the product polyunsaturated fatty acid may be, for example, DGLA, 20:4n-3, or ADA, respectively. The method may further comprise the step of exposing the product polyunsaturated fatty acid to at least one desaturase in order to convert the product polyunsaturated fatty acid to another polyunsaturated fatty acid. The product polyunsaturated fatty acid may be, for example, DGLA, 20:4n-3 and ADA, the another polyunsaturated fatty acid may be, for example, AA, EPA, and ω6-docosapentaenoic acid, respectively, and the at least one desaturase is Δ5-desaturase with respect to production of AA or EPA and Δ4-desaturase with respect to production of ω6-docosapentaenoic acid. The method may further comprise the step of exposing the another polyunsaturated fatty acid to one or more enzymes selected from the group consisting of at least one elongase and at least one additional desaturase in order to convert the another polyunsaturated fatty acid to a final polyunsaturated fatty acid. The final polyunsaturated fatty acid may be, for example, DHA, ADA, ω6-docosapentaenoic acid, and ω3-docosapentaenoic acid.

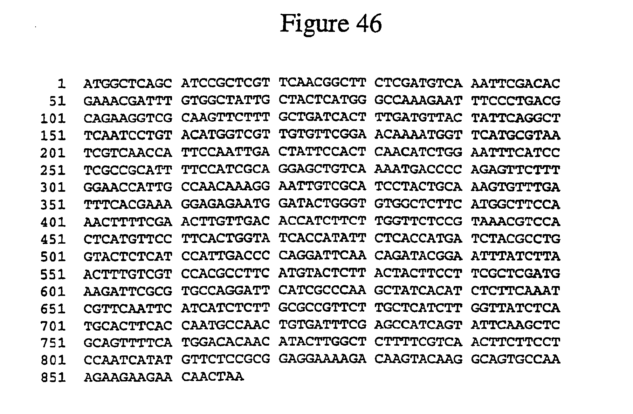

Additionally, the present invention includes an isolated nucleotide sequence corresponding to or complementary to the nucleotide sequence shown in Figure 46. It encodes a functionally active elongase which utilizes a polyunsaturated fatty acid as a substrate. The sequence may be derived or isolated from a nematode of the genus Caenorhabditis and, in particular, may be isolated from C. elegans.

Additionally, the present invention includes a method of producing an elongase enzyme comprising the steps of: a) isolating the nucleotide sequence represented by Figure 46 ; b) constructing a vector comprising: i) the isolated nucleotide sequence operably linked to ii) a promoter; and c) introducing the vector into a host cell under time and conditions sufficient for expression of the elongase enzyme. The properties of the host cell are the same as those described above in connection with the sequences of Figures 6, 22 and 43.

The present include also encompasses a vector comprising: a) a nucleotide sequence as represented by Figure 46 operably linked to b) a promoter, as well as a host cell comprising this vector. The host cell has the same properties as those recited above in connection with the host cell recited above for the sequences of Figures 6, 22 and 43.

Moreover, the present invention includes a plant cell, plant or plant tissue comprising the above vector comprising the sequence of Figure 46, wherein expression of said nucleotide sequence of the vector results in production of a polyunsaturated fatty acid by the plant cell, plant or plant tissue. The polyunsaturated fatty acid may be, for example, DGLA, 20:4n-3, or ADA. The invention also includes one or more plant oils or fatty acids expressed by this plant cell, plant or plant tissue.

The invention also includes transgenic plant comprising the above vector including the nucleotide sequence corresponding to the sequence of Figure 46, wherein expression of the nucleotide sequence of the vector results in production of a polyunsaturated fatty acid in seeds of the transgenic plant.

Additionally, the present invention includes a transgenic, non-human mammal whose genome comprises a C. elegans DNA sequence encoding an elongase operably linked to a promoter. The DNA sequence may be represented by Figure 46. A fluid may be produced by the transgenic, non-human mammal wherein the fluid comprises a detectable level of at least one elongase or products thereof.

The invention also includes a method for producing a polyunsaturated fatty acid comprising the steps of: a) isolating the nucleotide sequence represented by Figure 46; b) constructing a vector comprising the isolated nucleotide sequence; c) introducing the vector into a host cell under time and conditions sufficient for expression of an elongase enzyme encoded by the isolated nucleotide sequence; and d) exposing the expressed elongase enzyme to a substrate polyunsaturated fatty acid in order to convert the substrate to a product polyunsaturated fatty acid. The substrate polyunsaturated fatty acid may be, for example, GLA, STA, or AA, and the product polyunsaturated fatty acid may be, for example, DGLA, 20:4n-3, or ADA, respectively. The method may further comprise the step of exposing the expressed elongase enzyme to at least one desaturase in order to convert said product polyunsaturated fatty acid to another polyunsaturated fatty acid. The product polyunsaturated fatty acid may be, for example, DGLA, 20:4n-3 or ADA, the another polyunsaturated fatty acid may be, for example, AA, EPA or ω6-docosapentaenoic acid, respectively, and the at least one desaturase is Δ5-desaturase with respect to production of AA or EPA, and Δ4-desaturase with respect to production of ω6-docosapentaenoic acid. The method may further comprise the step of exposing the another polyunsaturated fatty acid to one or more enzymes selected from the group consisting of at least one elongase and at least one additional desaturase in order to convert the another polyunsaturated fatty acid to a final polyunsaturated fatty acid. The final polyunsaturated fatty acid may be, for example, DHA, ADA, ω6-docosapentaenoic acid, or ω3-docosapentaenoic acid.

### BRIEF DESCRIPTION OF THE DRAWINGS

Figure 1 represents various fatty acid biosynthesis pathways. The role of the elongase enzyme (elo) should be noted.
Figure 2 represents the percent similarity and percent identity between the amino acid sequences of jojoba KCS and ELO2.
Figure 3 represents the S. cerevisiae ELO2 sequence homologous to the jojoba KCS sequence (primer sequence underlined) of Figure 2.
Figure 4A shows the physical map of pRAE-2 containing the MAELO cDNA. Figure 4B represents the physical map of the constitutive expression vector, pRAE-5, used for elongase enzyme production in yeast.
Figure 5 represents a comparison of the nucleotide sequences of clones pRAE-5 and pRAE-6.
Figure 6 illustrates the complete nucleotide sequence of Mortierella alpina elongase (MAELO).
Figure 7 represents the amino acid sequence of the Mortierella alpina elongase translated from MAELO (see Figure 6).
Figure 8 represents an amino acid sequence alignment among 3 elongases: S. cerevisiae ELO2 (GNS1), S. cerevisiae ELO3 (SUR4) and the translated MAELO sequence as shown in Figure 7.
Figure 9 represents a comparison between the nucleotide sequence MAELO and the nucleotide sequence of ELO2 from S. cerevisiae.
Figures 10A and 10B represents the PUFA elongase activity of MAELO expressed in baker's yeast.
Figure 11 illustrates the PUFA elongase activity of MAELO when co-expressed with the Δ5-desaturase cDNA from M. alpina to produce AA.
Figure 12 compares the PUFA elongase activity of MAELO to the overexpression of ELO2 from S. cerevisiae in baker's yeast.
Figures 13, 14 and 15 represent three separate comparisons of amino acid sequences derived from C. elegans nucleotide sequences in the GenEMBL database with the translated MAELO.
Figure 16 shows the comparison between amino acid translations of two different mammalian sequences in the GenEMBL database and the translated MAELO.
Figure 17 shows the comparison of a translated DNA sequence (see published PCT application WO 88/07577) with the amino acid sequence derived from MAELO, which was detected during a database search.
Figure 18 shows the complete nucleotide sequence of the Δ5-desaturase from M. alpina.
Figure 19 represents the initial GC-FAME analysis of MAD708 pool. The detection of a DGLA (C20:3n-6) peak should be noted.
Figure 20 represents the PUFA elongase activity of the five MAD708 clones in yeast with GLA as substrate. All clones have apparent elongase activity.
Figure 21 represents the DNA sequencing analysis of plasmid pRPB2. The analysis reveals an open reading frame of 957 bp in length.
Figure 22 shows the complete nucleotide sequence of the M. alpina cDNA, contained in the plasmid pRPB2, which is designated GLELO for its GLA elongase activity.
Figure 23 represents the amino acid sequence of the M. alpina elongase translated from GLELO (see Figure 22).
Figure 24 illustrates the n-6 PUFA elongase activity in an induced culture of 334(pRPB2) when supplemented with GLA.
Figure 25 represents the n-3 and n-6 PUFA elongase activity in an induced culture of 334(pRPB2) when supplemented with 25 µm of other fatty acid substrates.
Figure 26A illustrates the elongase activity of GLELO with GLA as a substrate when co-expressed with the M. alpina Δ5-desaturase cDNA to produce AA. Figure 26B illustrates the elongase activity of GLELO with STA as a substrate when co-expressed with the M. alpina Δ5-desaturas cDNA to produce EPA.
Figure 27 illustrates the comparison between the translated GLELO sequence (see Figure 23) and the translated MAELO sequence (see Figure 7).
Figure 28 represents a comparison of the amino acid sequence of 4 elongases: the translated amino acid sequence of GLELO (see Figure 23), MAELO (see Figure 7), S. cerevisiae EL02 (GNS1), and S. cerevisiae ELO3 (SUR4). The histidine box is underlined.
Figure 29 represents an alignment between translated MAELO sequence and translated putative human homologue HS1 sequence.
Figure 30 represents an alignment between the translated MAELO sequence and the translated putative human homologue HS2 sequence.
Figure 31 shows an alignment between the translated MAELO sequence and the translated putative mouse homologue MM2 sequence.
Figure 32 represents an alignment between the translated MAELO and the translated putative mouse homologue AI225632 sequence.
Figure 33 illustrates an alignment between the translated GLELO sequence and the translated human homologue AI815960 sequence.
Figure 34 shows an alignment between the translated GLELO sequence and the translated putative human homologue HS1 sequence.
Figure 35 represents an alignment between the translated GLELO sequence and the translated putative human homologue sequence from AC004050.
Figure 36 illustrates an alignment between the translated GLELO sequence and the translated putative mouse homologue MM2 sequence.
Figure 37 represents an alignment of the translated GLELO sequence and a translated putative mouse homologue AI225632 sequence.
Figure 38 illustrates an alignment of the translated GLELO sequence and a translated putative mouse homologue U97107.
Figure 39 represents an alignment of the translated GLELO sequence and a translated putative C. elegans U68749 (F56H11.4) homologue sequence.
Figure 40 shows an alignment between the translated MAELO sequence and a translated putative C. elegans U68749 (F56H11.4) homologue sequence.
Figure 41 represents an alignment between the translated GLELO sequence and a translated putative Drosophila melanogaster homologue sequence, DM1.
Figure 42 illustrates an alignment between the translated MAELO sequence and a translated putative Drosophila melanogaster homologue sequence, DM1.
Figure 43 illustrates the complete nucleotide sequence of a human elongase HSELO1.
Figure 44 represents the deduced amino acid sequence of the human elongase HSELO1.
Figure 45 illustrates the elongase activity (PUFA and others) of an induced culture of 334(pRAE-58-A1) when supplemented with GLA or AA.
Figure 46 shows the complete nucleotide sequence of the C. elegans elongase CEELO.
Figure 47 represents the deduced amino acid of C. elegans elongase CEELO.
Figure 48 illustrates the PUFA elongase activity of an induced culture of 334(pRET-21) and 334(pRET-22) when supplemented with GLA and AA.
Figure 49 represents the complete nucleotide sequence of the putative human elongase gene HS3.
Figure 50 illustrates the deduced amino acid sequence of the putative human elongase enzyme HS3.

### DETAILED DESCRIPTION OF THE INVENTION

The subject invention relates to nucleotide and corresponding amino acid sequences of two elongase cDNAs derived from Mortierella alpina, as well as to nucleotide and corresponding amino acid sequences of an elongase cDNA derived from a human and one derived from C. elegans. Furthermore, the subject invention also includes uses of the cDNAs and of the proteins encoded by the genes. For example, the genes and corresponding enzymes may be used in the production of polyunsaturated fatty acids and/or monounsaturated fatty acids such as DGLA, AA, ADA, EPA and/or DHA which may be added to pharmaceutical compositions, nutritional compositions and to other valuable products.

### The Elongase Genes and Enzymes Encoded Thereby

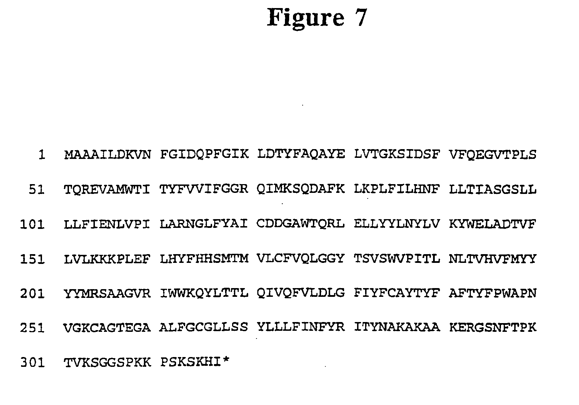

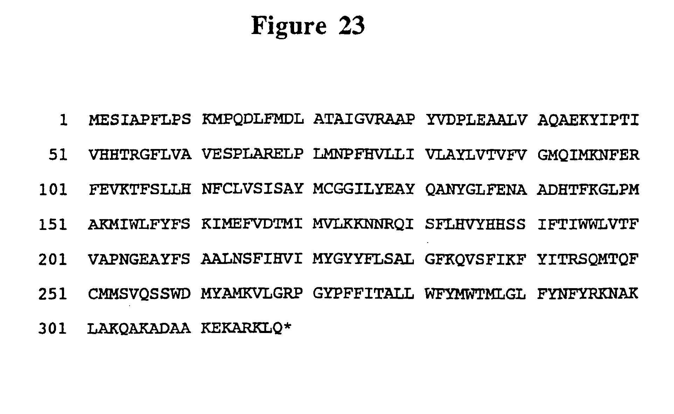

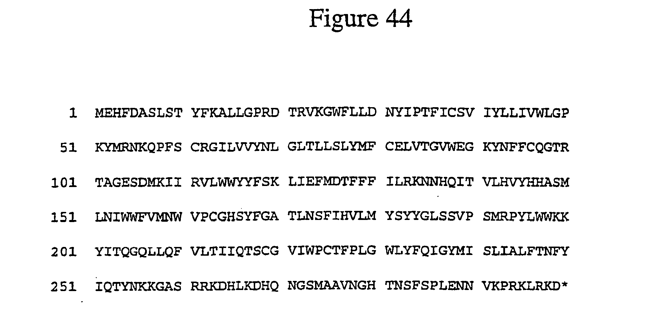

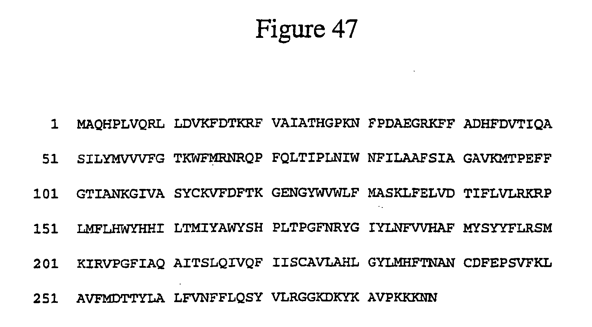

As noted above, an elongase enzyme encoded by an elongase cDNA is essential in the production of various polyunsaturated fatty acids, in particular, 20-24 carbon PUFAs. With respect to the present invention, the nucleotide sequence of the isolated M. alpina elongase cDNA (MAELO) is shown in Figure 6, and the amino acid sequence of the corresponding purified protein or enzyme encoded by this nucleotide sequence is shown in Figure 7. Additionally, the nucleotide sequence of the isolated GLA elongase cDNA (GLELO) is shown in Figure 22, and the amino acid sequence of the corresponding purified protein or enzyme encoded by this nucleotide sequence is shown in Figure 23. The nucleotide sequence of the isolated human sequence 1 (HSELO1) elongase is shown in Figure 43, and the amino acid sequence of the corresponding purified protein or enzyme encoded by this sequence is shown in Figure 44. Furthermore, the nucleotide sequence of the isolated C. elegans elongase cDNA (CEELO1) is shown in Figure 46, and the amino acid sequence of the corresponding purified protein or enzyme encoded thereby is shown in Figure 47.

As an example, the isolated elongases encoded by the cDNAs of the present invention elongate GLA to DGLA or elongate STA to 20:4n-3 or elongate AA to ADA. The production of arachidonic acid from DGLA, or EPA from 20:4n-3, is then catalyzed by a Δ5-desaturase. Thus, neither AA (or EPA), nor DGLA (or 20:4n-3) nor ADA (or ω3-docosapentaenoic acid), can be synthesized without at least one elongase cDNA and enzyme encoded thereby.

It should be noted that the present invention also encompasses nucleotide sequences (and the corresponding encoded proteins) having sequences corresponding to (i.e., having identity to) or complementary to at least about 50%, preferably at least about 60%, and more preferably at least about 70% of the nucleotides in the nucleotide sequence of the MAELO cDNA described herein (see Figure 6)). Furthermore, the present invention also includes nucleotide sequences (and the corresponding encoded proteins) having sequences corresponding to (i.e., having identity to) or complementary to at least about 35%, preferably at least about 45%, and more preferably at least about 55% of the nucleotides in the nucleotide sequence of the GLELO cDNA described herein (see Figure 22). Additionally, the present invention also includes nucleotide sequences (and the corresponding encoded proteins) having sequences corresponding to (i.e., having identity to) or complementary to the nucleotide sequence of the human sequence 1 (HSELO1) cDNA described herein (see Figure 43). In addition, the present invention also includes nucleotide sequences having sequences corresponding to (i.e., having identity to) or complementary to the nucleotides in the nucleotide sequence of the C. elegans cDNA, CEELO1, described herein (see Figure 46). Such sequences may be derived from non-Mortierella sources (e.g., a eukaryote (e.g., Thraustochytrium spp. (e.g., Thraustochytrium aureum and Thraustochytrium roseum), Schizochytrium spp. (e.g., Schizochytrium aggregatum), Conidiobolus spp. (e.g., Conidiobolus nanodes), Entomorphthora spp. (e.g., Entomorphthora exitalis), Sarolegnia spp. (e.g., Saproleania parasitica and Saprolegnia diclina), Leptomitus spp. (e.g., Leptomitus lacteus) , Entomophthora spp. , Pythium spp., Porphyridium spp. (e.g., Porohyridium cruentum), Conidiobolus spp., Phytophathora spp., Penicillium spp., Coidosporium spp., Mucor spp. (e.g., Mucor circinelloides and Mucor javanicus), Fusarium spp., Aspergillus spp. and Rhodotorula spp.), a yeast (e.g., Dipodascopsis uninucleata), a non-mammalian organism such as a fly (e.g., Drosophila melanogaster) or Caenorhabditis spp. (e.g., Caenorhabditis elegans), or a mammal (e.g., a human or a mouse). Such sequences may be derived from species within the genus Mortierella, other than the species alpina, for example, Mortierella elongata, Mortierella exigua, Mortierella isabellina, Mortierella hygrophila, and Mortierella ramanniana, va. angulispora. Furthermore, the present invention also encompasses fragments and derivatives of the nucleotide sequences of the present invention (i.e. the sequence of Figure 6 (MAELO), the sequence of Figure 22 (GLELO), the sequence of Figure 43 (HSELO1) and the sequence of Figure 46 (CEELO1)), as well as of the sequences derived from non Mortierella sources and having the above-described complementarity or correspondence/identity. Functional equivalents of the above-sequences (i.e., sequences having elongase activity) are also encompassed by the present invention.

For purposes of the present invention, "complementarity" is defined as the degree of relatedness between two DNA segments. It is determined by measuring the ability of the sense strand of one DNA segment to hybridize with the antisense strand of the other DNA segment, under appropriate conditions, to form a double helix. In the double helix, wherever adenine appears in one strand, thymine appears in the other strand. Similarly, wherever guanine is found in one strand, cytosine is found in the other. The greater the relatedness between the nucleotide sequences of two DNA segments, the greater the ability to form hybrid duplexes between the strands of two DNA segments.

"Identity" between two nucleotide sequences is defined as the degree of sameness, correspondence or equivalence between the same strands (either sense or antisense) of two DNA segments. The greater the percent identity, the higher the correspondence, sameness or equivalence between the strands.

"Similarity" between two amino acid sequences is defined as the presence of a series of identical as well as conserved amino acid residues in both sequences. The higher the degree of similarity between two amino acid sequences, the higher the correspondence, sameness or equivalence of the two sequences. ("Identity" between two amino acid sequences is defined as the presence of a series of exactly alike or invariant amino acid residues in both sequences.)

The definitions of "complementarity", "identity", and "similarity" are well known to those of ordinary skill in the art.

The invention also includes a purified polypeptide which elongates polyunsaturated and monounsaturated fatty acids (wherein the polypeptides (see, e.g., Figure 7 (MAELO)) are encoded by the above-described nucleotide sequences. Additionally, the present invention includes a purified polypeptide which elongates polyunsaturated fatty acids (wherein the polypeptides (see, e.g., Figure 23 (GLELO)) are encoded by the above-described nucleotide sequences. Furthermore, the invention also includes a purified polypeptide which elongates polyunsaturated and monounsaturated fatty acids (wherein the polypeptides (see, e.g., Figure 44 (HSELO1)) are encoded by the above-described nucleotide sequences.

The present invention also encompasses an isolated nucleotide sequence which encodes PUPA elongase activity and that is hybridizable, under moderately stringent conditions, to a nucleic acid having a nucleotide sequence corresponding or complementary to the nucleotide sequence represented by Figure 6 (MAELD) and/or Figure 22 (GLELO) and/or the sequence (HSELO1) shown in Figure 43 and/or the sequence (CEELO1) shown in Figure 46. A nucleic acid molecule is "hybridizable" to another nucleic acid molecule when a single-stranded form of the nucleic acid molecule can anneal to the other nucleic acid molecule under the appropriate conditions of temperature and ionic strength (see Sambrook et al., "Molecular Cloning: A Laboratory Manual, Second Edition (1989), Cold spring Harbor Laboratory Press, Cold Spring Harbor, New York)). The conditions of temperature and ionic strength determine the "stringency" of the hybridization. "Hybridization" requires that two nucleic acids contain complementary sequences. However, depending on the stringency of the hybridization, mismatches between bases may occur. The appropriate stringency for hybridizing nucleic acids depends on the length of the nucleic acids and the degree of complementarity. Such variables are well known in the art. More specifically, the greater the degree of similarity or homology between two nucleotide sequences, the greater the value of Tm, melting temperature, for hybrids of nucleic acids having those sequences. For hybrids of greater than 100 nucleotides in length, equations for calculating Tm have been derived (see Sambrook et al., supra). For hybridization with shorter nucleic acids, the position of mismatches becomes more important, and the length of the oligonucleotide determines its specificity (see Sambrook et al., supra).

### Production of the Elongase Enzyme

Once the gene encoding the elongase has been isolated, it may then be introduced into either a prokaryotic or eukaryotic host cell through the use of a vector, plasmid or construct.

The vector, for example, a bacteriophage, cosmid or plasmid, may comprise the nucleotide sequence encoding the elongase as well as any promoter which is functional in the host cell and is able to elicit expression of the elongase encoded by the nucleotide sequence. The promoter is in operable association with or operably linked to the nucleotide sequence. (A promoter is said to be "operably linked" with a coding sequence if the promoter affects transcription or expression of the coding sequence.) Suitable promoters include, for example, those from genes encoding alcohol dehydrogenase, glyceraldehyde-3-phosphate dehydrogenase, phosphoglucoisomerase, phosphoglycerate kinase, acid phosphatase, T7, TP1, lactase, metallothionein, cytomegalovirus immediate early, whey acidic protein, glucoamylase, and promoters activated in the presence of galactose, for example, GAL1 and GAL10. Additionally, nucleotide sequences which encode other proteins, oligosaccharides, lipids, etc. may also be included within the vector as well as other regulatory sequences such as a polyadenylation signal (e.g., the poly-A signal of SV-40T-antigen, ovalalbumin or bovine growth hormone). The choice of sequences present in the construct is dependent upon the desired expression products as well as the nature of the host cell.

As noted above, once the vector has been constructed, it may then be introduced into the host cell of choice by methods known to those of ordinary skill in the art including, for example, transfection, transformation and electroporation (see Molecular Cloning: A Laboratory Manual, 2nd ed., Vol. 1-3, ed. Sambrook et al., Cold Spring Harbor Laboratory Press (1989)). The host cell is then cultured under suitable conditions permitting expression of the PUFA which is then recovered and purified.

It should also be noted that one may design a unique triglyceride or oil if one utilizes one construct or vector comprising the nucleotide sequences of two or more cDNAs (e.g., MAELO, GLELO, HSELO1 and/or CEELO1). This vector may then be introduced into one host cell. Alternatively, each of the sequences may be introduced into a separate vector. These vectors may then be introduced into two host cells, respectively, or into one host cell.

Examples of suitable prokaryotic host cells include, for example, bacteria such as Escherichia coli, Bacillus subtilis as well as cyanobacteria such as Spirulina spp. (i.e., blue-green algae). Examples of suitable eukaryotic host cells include, for example, mammalian cells, plant cells, yeast cells such as Saccharomyces spp., Lipomyces spp., Candida spp. such as Yarrowia (Candida) spp., Kluyveromyces spp., Pichia spp., Trichoderma spp. or Hansenula spp., or fungal cells such as filamentous fungal cells, for example, Aspergillus, Neurospora and Penicillium. Preferably, Saccharomyces cerevisiae (baker's yeast) cells are utilized.

Expression in a host cell can be accomplished in a transient or stable fashion. Transient expression can occur from introduced constructs which contain expression signals functional in the host cell, but which constructs do not replicate and rarely integrate in the host cell, or where the host cell is not proliferating. Transient expression also can be accomplished by inducing the activity of a regulatable promoter operably linked to the gene of interest, although such inducible systems frequently exhibit a low basal level of expression. Stable expression can be achieved by introduction of a construct that can integrate into the host genome or that autonomously replicates in the host cell. Stable expression of the gene of interest can be selected for through the use of a selectable marker located on or transfected with the expression construct, followed by selection for cells expressing the marker. When stable expression results from integration, the site of the construct's integration can occur randomly within the host genome or can be targeted through the use of constructs containing regions of homology with the host genome sufficient to target recombination with the host locus. Where constructs are targeted to an endogenous locus, all or some of the transcriptional and translational regulatory regions can be provided by the endogenous locus.

A transgenic mammal may also be used in order to express the enzyme of interest (i.e., the elongase) encoded by one or both of the above-described nucleotide sequences. More specifically, once the above-described construct is created, it may be inserted into the pronucleus of an embryo. The embryo may then be implanted into a recipient female. Alternatively, a nuclear transfer method could also be utilized (Schnieke et al., Science 278:2130-2133 (1997)). Gestation and birth are then permitted to occur(see, e.g., U.S. Patent No. 5,750,176 and U.S. Patent No. 5,700,671). Milk, tissue or other fluid samples from the offspring should then contain altered levels of PUFAs, as compared to the levels normally found in the non-transgenic animal. Subsequent generations may be monitored for production of the altered or enhanced levels of PUFAs and thus incorporation of the gene or genes encoding the elongase enzyme into their genomes. The mammal utilized as the host may be selected from the group consisting of, for example, a mouse, a rat, a rabbit, a pig, a goat, a sheep, a horse and a cow. However, any mammal may be used provided it has the ability to incorporate DNA encoding the enzyme of interest into its genome.

For expression of an elongase polypeptide, functional transcriptional and translational initiation and termination regions are operably linked to the DNA encoding the elongase polypeptide. Transcriptional and translational initiation and termination regions are derived from a variety of nonexclusive sources, including the DNA to be expressed, genes known or suspected to be capable of expression in the desired system, expression vectors, chemical synthesis, or from an endogenous locus in a host cell. Expression in a plant tissue and/or plant part presents certain efficiencies, particularly where the tissue or part is one which is harvested early, such as seed, leaves, fruits, flowers, roots, etc. Expression can be targeted to that location with the plant by utilizing specific regulatory sequence such as those of U.S. Patent Nos. 5,463,174, 4,943,674, 5,106,739, 5,175,095, 5,420,034, 5,188,958, and 5,589,379, Alternatively, the expressed protein can be an enzyme which produces a product which may be incorporated, either directly or upon further modifications, into a fluid fraction from the host plant. Expression of an elongase gene or genes, or antisense elongase transcripts, can alter the levels of specific PUFAs, or derivatives thereof, found in plant parts and/or plant tissues. The elongase polypeptide coding region may be expressed either by itself or with other genes, in order to produce tissues and/or plant parts containing higher proportions of desired PUFAs or in which the PUFA composition more closely resembles that of human breast milk (Prieto et al., PCT publication WO 95/24494). The termination region may be derived from the 3' region of the gene from which the initiation region was obtained or from a different gene. A large number of termination regions are known to and have been found to be satisfactory in a variety of hosts from the same and different genera and species. The termination region usually is selected as a matter of convenience rather than because of any particular property.

As noted above, a plant (e.g., Glycine max (soybean) or Brassica napus (canola)), plant tissue, corn, potatoe, sunflower, safflower or flax may also be utilized as a host or host cell, respectively, for expression of the elongase enzyme(s) which may, in turn, be utilized in the production of polyunsaturated fatty acids. More specifically, desired PUFAs can be expressed in seed. Methods of isolating seed oils are known in the art. Thus, in addition to providing a source for PUFAs, seed oil components may be manipulated through the expression of the elongase genes, as well as perhaps desaturase genes, in order to provide seed oils that can be added to nutritional compositions, pharmaceutical compositions, animal feeds and cosmetics. Once again, a vector which comprises a DNA sequence encoding the elongase operably linked to a promoter, will be introduced into the plant tissue or plant for a time and under conditions sufficient for expression of the elongase gene. The vector may also comprise one or more genes which encode other enzymes, for example, Δ4-desaturase, Δ5-desaturase Δ6-desaturase, Δ8-desaturase, Δ9-desaturase, Δ10-desaturase, Δ12-desaturase, Δ13-desaturase, Δ15-desaturase, Δ17-desaturase and/or Δ19-desaturase. The plant tissue or plant may produce the relevant substrate (e.g., DGLA, GLA, STA, AA, ADA, EPA, 20:4n-3, etc.) upon which the enzymes act or a vector encoding enzymes which produce such substrates may be introduced into the plant tissue, plant cell, plant, or host cell of interest. In addition, substrate may be sprayed on plant tissues expressing the appropriate enzymes. Using these various techniques, one may produce PUFAs (e.g., n-6 unsaturated fatty acids such as DGLA, AA or ADA, or n-3 fatty acids such as EPA or DHA) by use of a plant cell, plant tissue, plant, or host cell of interest. It should also be noted that the invention also encompasses a transgenic plant comprising the above-described vector, wherein expression of the nucleotide sequence of the vector results in production of a polyunsaturated fatty acid in, for example, the seeds of the transgenic plant.

The substrates which may be produced by the host cell either naturally or transgenically, as well as the enzymes which may be encoded by DNA sequences present in the vector, which is subsequently introduced into the host cell, are shown in Figure 1.

In view of the above, the present invention also encompasses a method of producing one of the elongase enzymes described above comprising the steps of: 1) isolating the desired nucleotide sequence of the elongase cDNA; 2) constructing a vector comprising said nucleotide sequence; and 3) introducing said vector into a host cell under time and conditions sufficient for the production of the elongase enzyme.

The present invention also encompasses a method of producing polyunsaturated fatty acids comprising exposing an acid to the elongase(s) produced as above such that the elongase converts the acid to a polyunsaturated fatty acid. For example, when GLA is exposed to elongase, it is converted to DGLA. DGLA may then be exposed to Δ5-desaturase which converts the DGLA to AA. The AA may then be converted to EPA by use of Δ17-desaturase which may be, in turn, converted to DHA by use of elongase and a Δ4-desaturase. Alternatively, elongase may be utilized to convert 18:4n-3 to 20:4n-3 which may be exposed to Δ5-desaturase and converted to EPA. Elongase may also be used to convert 18:3n-3 to 20:3n-3, which may be, in turn, converted to 20:4n-3 by a Δ8-desaturase. Thus, elongase may be used in the production of polyunsaturated fatty acids which may be used, in turn, for particular beneficial purposes. (See Figure 1 for an illustration of the many critical roles elongase plays in several biosynthetic pathways.)

### Uses of the Elongase Gene and Enzyme Encoded Thereby

As noted above, the isolated elongase cDNAs and the corresponding elongase enzymes (or purified polypeptides) encoded thereby have many uses. For example, each cDNA and corresponding enzyme may be used indirectly or directly in the production of polyunsaturated fatty acids, for example, DGLA, AA, ADA, 20:4n-3 or EPA. ("Directly" is meant to encompass the situation where the enzyme directly converts the acid to another acid, the latter of which is utilized in a composition (e.g., the conversion of GLA to DGLA)). "Indirectly" is meant to encompass the situation where a fatty acid is converted to another fatty acid (i.e., a pathway intermediate) by elongase (e.g., GLA to DGLA) and then the latter fatty acid is converted to another fatty acid by use of a non-elongase enzyme (e.g., DGLA to AA by Δ5-desaturase)). These polyunsaturated fatty acids (i.e., those produced either directly or indirectly by activity of the elongase enzyme) may be added to, for example, nutritional compositions, pharmaceutical compositions, cosmetics, and animal feeds, all of which are encompassed by the present invention. These uses are described, in detail, below.

### Nutritional Compositions

One of the uses of the polyunsaturated fatty acid produced according to the methods of the present invention includes nutritional compositions. Such compositions, for purposes of the present invention, include any food or preparation for human consumption including for enteral or parenteral consumption, which when taken into the body (a) serve to nourish or build up tissues or supply energy and/or (b) maintain, restore or support adequate nutritional status or metabolic function.

The nutritional composition comprises at least one oil or acid produced by use of at least one elongase enzyme, produced using the respective elongase gene, and may either be in a solid or liquid form. Additionally, the composition may include edible macronutrients, vitamins and minerals in amounts desired for a particular use. The amount of such ingredients will vary depending on whether the composition is intended for use with normal, healthy infants, children or adults having specialized needs such as those which accompany certain metabolic conditions (e.g., metabolic disorders).

Examples of macronutrients which may be added to the composition include but are not limited to edible fats, carbohydrates and proteins. Examples of such edible fats include but are not limited to coconut oil, soy oil, and mono- and diglycerides. Examples of such carbohydrates include but are not limited to glucose, edible lactose and hydrolyzed starch. Additionally, examples of proteins which may be utilized in the nutritional composition of the invention include but are not limited to soy proteins, electrodialysed whey, electrodialysed skim milk, milk whey, or the hydrolysates of these proteins.

With respect to vitamins and minerals, the following may be added to the nutritional compositions : calcium, phosphorus, potassium, sodium, chloride, magnesium, manganese, iron, copper, zinc; selenium, iodine, and Vitamins A, E, D, C, and the B complex. Other such vitamins and minerals may also be added.

The components utilized in the nutritional compositions will be of semi-purified or purified origin. By semi-purified or purified is meant a material which has been prepared by purification of a natural material or by synthesis.

Examples of nutritional compositions include but are not limited to infant formulas, dietary supplements, dietary substitutes, and rehydration compositions. Nutritional compositions of particular interest include but are not limited to those utilized for enteral and parenteral supplementation for infants, specialist infant formulae, supplements for the elderly, and supplements for those with gastrointestinal difficulties and/or malabsorption.

The nutritional composition may also be added to food even when supplementation of the diet is not required. For example, the composition may be added to food of any type including but not limited to margarines, modified butters, cheeses, milk, yogurt, chocolate, candy, snacks, salad oils, cooking oils, cooking fats, meats, fish and beverages.

In a preferred embodiment, the nutritional composition is an enteral nutritional product, more preferably, an adult or pediatric enteral nutritional product. This composition may be administered to adults or children experiencing stress or having specialized needs due to chronic or acute disease states. The composition may comprise, in addition to polyunsaturated fatty acids produced in accordance with the present invention, macronutrients, vitamins and minerals as described above. The macronutrients may be present in amounts equivalent to those present in human milk or on an energy basis, i.e., on a per calorie basis.

Methods for formulating liquid or solid enteral and parenteral nutritional formulas are well known in the art. (See also the Examples below.)

The enteral formula, for example, may be sterilized and subsequently utilized on a ready-to-feed (RTF) basis or stored in a concentrated liquid or powder. The powder can be prepared by spray drying the formula prepared as indicated above, and reconstituting it by rehydrating the concentrate. Adult and pediatric nutrional formulas are well known in the art and are commercially available (e.g., Similac^{®}, Ensure^{®}, Jevity^{®} and Alimentum^{®} from Ross Products Division, Abbott Laboratories, Columbus, Ohio). An oil or fatty acid produced in accordance with the present invention may be added to any of these formulas.

The energy density of the nutritional compositions of the present invention, when in liquid form, may range from about 0.6 Kcal to about 3 Kcal per ml. When in solid or powdered form, the nutritional supplements may contain from about 1.2 to more than 9 Kcals per gram, preferably about 3 to 7 Kcals per gm. In general, the osmolality of a liquid product should be less than 700 mOsm and, more preferably, less than 660 mOsm.

The nutritional formula may include macronutrients, vitamins, and minerals, as noted above, in addition to the PUFAs produced in accordance with the present invention. The presence of these additional components helps the individual ingest the minimum daily requirements of these elements. In addition to the provision of PUFAs, it may also be desirable to add zinc, copper, folic acid and antioxidants to the composition. It is believed that these substance boost a stressed immune system and will therefore provide further benefits to the individual receiving the composition. A pharmaceutical composition may also be supplemented with these elements.

The nutritional composition comprises, in addition to antioxidants and at least one PUFA, a source of carbohydrate wherein at least 5 weight % of the carbohydrate is indigestible oligosaccharide. The nutritional composition additionally may comprise protein, taurine, and carnitine.

As noted above, the PUFAs produced in accordance with the present invention, or derivatives thereof, may be added to a dietary substitute or supplement, particularly an infant formula, for patients undergoing intravenous feeding or for preventing or treating malnutrition or other conditions or disease states. As background, it should be noted that human breast milk has a fatty acid profile comprising from about 0.15% to about 0.36% as DHA, from about 0.03% to about 0.13% as EPA, from about 0.30% to about 0.88% as AA, from about 0.22% to about 0.67% as DGLA, and from about 0.27% to about 1.04% as GLA. Thus, fatty acids such as DGLA, AA, EPA and/or docosahexaenoic acid (DHA), produced in accordance with the present invention, can be used to alter, for example, the composition of infant formulas in order to better replicate the PUFA content of human breast milk or to alter the presence of PUFAs normally found in a non-human mammal's milk. In particular, a composition for use in a pharmacologic or food supplement, particularly a breast milk substitute or supplement, will preferably comprise one or more of AA, DGLA and GLA. More preferably, the oil blend will comprise from about 0.3 to 30% AA, from about 0.2 to 30% DGLA, and/or from about 0.2 to about 30% GLA.

Parenteral nutritional compositions comprising from about 2 to about 30 weight percent fatty acids calculated as triglycerides are example of uses of the polyunsaturated acids of the present invention. The preferred composition has about 1 to about 25 weight percent of the total PUFA composition as GLA (U.S. Patent No. 5,196,198). Other vitamins, particularly fat-soluble vitamins such as vitamin A, D, E and L-carnitine can optionally be included. When desired, a preservative such as alpha-tocopherol may be added in an amount of about 0.1% by weight.

In addition, the ratios of AA, DGLA and GLA can be adapted for a particular given end use. When formulated as a breast milk supplement or substitute, a composition which comprises one or more of AA, DGLA and GLA will be provided in a ratio of about 1:19:30 to about 6:1:0.2, respectively. For example, the breast milk of animals can vary in ratios of AA:DGLA:GLA ranging from 1:19:30 to 6:1:0.2, which includes intermediate ratios which are preferably about 1:1:1, 1:2:1, 1:1:4. When produced together in a host cell, adjusting the rate and percent of conversion of a precursor substrate such as GLA and DGLA to AA can be used to precisely control the PUFA ratios. For example, a 5% to 10% conversion rate of DGLA to AA can be used to produce an AA to DGLA ratio of about 1:19, whereas a conversion rate of about 75% to 80% can be used to produce an AA to DGLA ratio of about 6:1. Therefore, whether in a cell culture system or in a host animal, regulating the timing, extent and specificity of elongase expression, as well as the expression of other desaturases, can be used to modulate PUFA levels and ratios. The PUFAs/acids produced in accordance with the present invention (e.g., AA and DGLA) may then be combined with other PUFAs/acids (e.g., GLA) in the desired concentrations and ratios.

Additionally, PUFA produced in accordance with the present invention or host cells containing them may also be used as animal food supplements to alter an animal's tissue or milk fatty acid composition to one more desirable for human or animal consumption.

### Pharmaceutical Compositions

Another use of the polyunsatured acids of the present invention is in the production of a pharmaceutical composition comprising one or more of the fatty acids and/or resulting oils produced using at least one of the elongase cDNAs (i.e., MAELO, GLELO, HSELO1, or CEELO), in accordance with the methods described herein. More specifically, such a pharmaceutical composition may comprise one or more of the acids and/or oils as well as a standard, well-known, non-toxic pharmaceutically acceptable carrier, adjuvant or vehicle such as, for example, phosphate buffered saline, water, ethanol, polyols, vegetable oils, a wetting agent or an emulsion such as a water/oil emulsion. The composition may be in either a liquid or solid form. For example, the composition may be in the form of a tablet, capsule, ingestible liquid or powder, injectible, or topical ointment or cream. Proper fluidity can be maintained, for example, by the maintenance of the required particle size in the case of dispersions and by the use of surfactants. It may also be desirable to include isotonic agents, for example, sugars, sodium chloride and the like. Besides such inert diluents, the composition can also include adjuvants, such as wetting agents, emulsifying and suspending agents, sweetening agents; flavoring agents and perfuming agents.

Suspensions, in addition to the active compounds, may comprise suspending agents such as, for example, ethoxylated isostearyl alcohols, polyoxyethylene sorbitol and sorbitan esters, microcrystalline cellulose, aluminum metahydroxide, bentonite, agar-agar and tragacanth or mixtures of these substances.

Solid dosage forms such as tablets and capsules can be prepared using techniques well known in the art. For example, PUFAs produced in accordance with the present invention can be tableted with conventional tablet bases such as lactose, sucrose, and cornstarch in combination with binders such as acacia, cornstarch or gelatin, disintegrating agents such as potato starch or alginic acid, and a lubricant such as stearic acid or magnesium stearate. Capsules can be prepared by incorporating these excipients into a gelatin capsule along with antioxidants and the relevant PUFA(s). The antioxidant and PUFA components should fit within the guidelines presented above.

For intravenous administration, the PUFAs produced in accordance with the present invention or derivatives thereof may be incorporated into commercial formulations such as Intralipids^{™}. The typical normal adult plasma fatty acid profile comprises 6.64 to 9.46% of AA, 1.45 to 3.11% of DGLA, and 0.02 to 0.08% of GLA. These PUFAs or their metabolic precursors can be administered alone or in combination with other PUFAs in order to achieve a normal fatty acid profile in a patient. Where desired, the individual components of the formulations may be provided individually, in kit form, for single or multiple use. A typical dosage of a particular fatty acid is from 0.1 mg to 20 g (up to 100 g) daily and is preferably from 10 mg to 1, 2, 5 or 10 g daily.

Possible routes of administration of the pharmaceutical compositions of the present invention include, for example, enteral (e.g., oral and rectal) and parenteral. For example, a liquid preparation may be administered, for example, orally or rectally. Additionally, a homogenous mixture can be completely dispersed in water, admixed under sterile conditions with physiologically acceptable diluents, preservatives, buffers or propellants in order to form a spray or inhalant.

The route of administration will, of course, depend upon the desired effect. For example, if the composition is being utilized to treat rough, dry, or aging skin, to treat injured or burned skin, or to treat skin or hair affected by a disease or condition, it may perhaps be applied topically.

The dosage of the composition to be administered to the patient may be determined by one of ordinary skill in the art and depends upon various factors such as weight of the patient, age of the patient, immune status of the patient, etc.

With respect to form, the composition may be, for example, a solution, a dispersion, a suspension, an emulsion or a sterile powder which is then reconstituted.

The above pharmaceutical and/or nutritional compositions are useful in the treatment of various disorders. In particular, these compositions may be used to treat restenosis after angioplasty. Furthermore, symptoms of inflammation, rheumatoid arthritis, asthma and psoriasis may also be treated with these compositions. Evidence also indicates that PUFAs may be involved in calcium metabolism; thus, the compositions may, perhaps, be utilized in the treatment or prevention of osteoporosis and of kidney or urinary tract stones.

Additionally, these compositions may also be used in the treatment of cancer. Malignant cells have been shown to have altered fatty acid compositions. Addition of fatty acids has been shown to slow their growth, cause cell death and increase their susceptibility to chemotherapeutic agents. Moreover, the compositions of the present invention may also be useful for treating cachexia associated with cancer.

These compositions may also be used to treat diabetes (see U.S. Patent No. 4,826,877 and Horrobin et al., Am. J. Clin. Nutr. Vol. 57 (Suppl.) 732S-737S). Altered fatty acid metabolism and composition have been demonstrated in diabetic animals.

Furthermore, these compositions, comprising PUFAs produced either directly or indirectly through the use of the elongase enzyme(s), may also be used in the treatment of eczema, in the reduction of blood pressure, and in the improvement of mathematics examination scores. Additionally, these compositions may be used in inhibition of platelet aggregation, induction of vasodilation, reduction in cholesterol levels, inhibition of proliferation of vessel wall smooth muscle and fibrous tissue (Brenner et al., Adv. Exp. Med. Siol. Vol. 83, p.85-101, 1976), reduction or prevention of gastrointestinal bleeding and other side effects of non-steroidal anti-inflammatory drugs (see U.S. Patent No. 4,666,701), prevention or treatment of endometriosis and premenstrual syndrome (see U.S. Patent No. 4,758,592), and treatment of myalgic encephalomyelitis and chronic fatigue after viral infections (see U.S. Patent No. 5,116,871).

Further uses of the compositions include use in the treatment of AIDS, multiple sclerosis, and inflammatory skin disorders, as well as for maintenance of general health.

Additionally, these composition may be utilized for cosmetic purposes. It may be added to pre-existing cosmetic compositions such that a mixture is formed or may be used as a sole composition.

### Veterinary Applications

It should be noted that the above-described pharmaceutical and nutritional compositions may be utilized in connection with animals (i.e., domestic or non-domestic), as well as humans, as animals experience many of the same needs and conditions as humans. For example, the oil or acids of the present invention may be utilized in animal feed supplements, animal feed substitutes, animal vitamins or in animal topical ointments.

The present invention may be illustrated by the use of the following non-limiting examples:

### Example I

### Determination of Codon Usage in Mortierella alpina

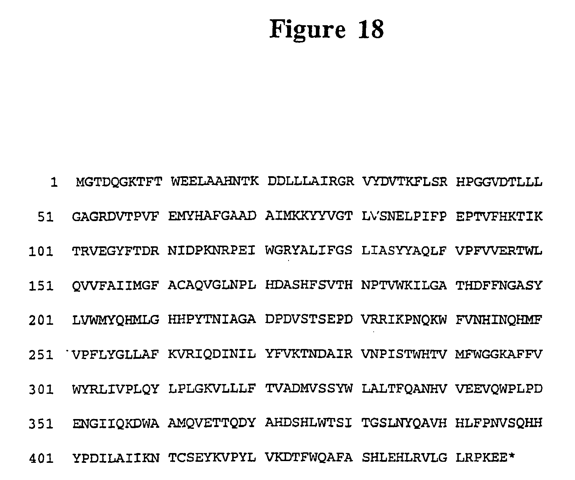

The 5' end of 1000 random cDNA clones were sequenced from Mortierella alpina cDNA library. The sequences were translated in six reading frames using GCG (Genetics Computer Group (Madison, Wisconsin)) with the FastA algorithm (Pearson and Lipman, Proc. Natl. Acad. Sci. USA 85:2444-2448 (1988)) to search for similarity between a query sequence and a group of sequences of the same type (nucleic acid or protein), specifically with the Swissprot database (GeneBio, Geneva, Switzerland). Many of the clones were identified as a putative housekeeping gene based on protein sequence homology to known genes. Twenty-one M. alpina cDNA sequences which matched with known, housekeeping genes in the database were selected (see Table 1 below). M. alpina codon bias table (see Table 2) was generated based on these 21 sequences as well as the full length M. alpina Δ5- (see Figure 18), Δ6-, and Δ12-desaturase sequences. Since the FastA alignment between the putative protein coded by the M. alpina cDNA sequence and the known protein sequence was weak in some areas, only the codons from areas of strong homology were used.

**Table 1**

| Clone # | Match | # of bp | # of aa |
|---|---|---|---|
| 193 | Elongation factor 1-alpha | 426 | 142 |
| 143 | 60S ribosomal protein L17 | 417 | 139 |
| 235 | Actin I | 360 | 120 |
| 299 | 40S ribosomal protein YS11 | 387 | 129 |
| 390 | Ras-related protein rab-1a | 342 | 114 |
| 65 | 40S ribosomal protein RP10 | 366 | 122 |
| 289 | Ubiquitin-conjugating enzyme E2-16 KD | 294 | 98 |
| 151 | Ubiquinol-cytochrome C reductase | 375 | 125 |
| 80 | Initiation factor 5A-2 | 183 | 61 |
| 33 | 60S ribosomal protein L15 | 252 | 84 |
| 132 | 60S ribosomal protein L3-2 | 300 | 100 |
| 198 | Histone H3 | 285 | 95 |
| 286 | 6-phosphogluconate dehydrogenase, decarboxylating | 363 | 121 |
| 283 | 40S ribosomal protein S22 | 261 | 87 |
| 127 | Elongation factor 2 | 231 | 77 |
| 197 | Actin, gamma | 252 | 84 |
| 496 | 40S ribosomal protein S16 | 270 | 90 |
| 336 | Histone H4 | 219 | 73 |
| 262 | Ubiquitin | 228 | 76 |
| 188 | Guanine nucleotide-binding protein beta subunit-like protein | 213 | 71 |
| 81 | Ubiquitin | 228 | 76 |
| 21 | TOTAL | 6252 | 2084 |

**Table 2**

| Amino acid | Codon Bias | % used | Amino acid | Codon Bias | % used |
|---|---|---|---|---|---|
| Ala | GCC | 63% | Lys | AAG | 96% |
| Arg | CGC | 50% | Met | ATG | 100% |
| Asn | AAC | 97% | Phe | TTC | 78% |
| Asp | GAC | 65% | Pro | CCC | 68% |
| Cys | TGC | 87% | Ser | TCC | 46% |
| Gln | CAG | 78% | Thr | ACC | 78% |
| Glu | GAG | 85% | Trp | TGG | 100% |
| Gly | GGT | 47% | Tyr | TAC | 95% |
| His | CAC | 91% | Val | GTC | 72% |
| Ile | ATC | 72% | Stop | TAA | 50% |
| Leu | CTC | 49% | | | |

### Example II

### Cloning of a Full-length Elongase-like cDNA from M. alpina

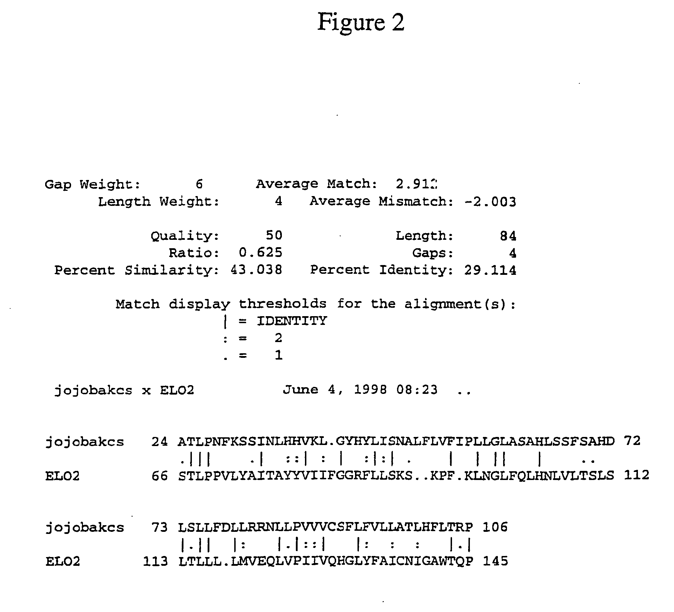

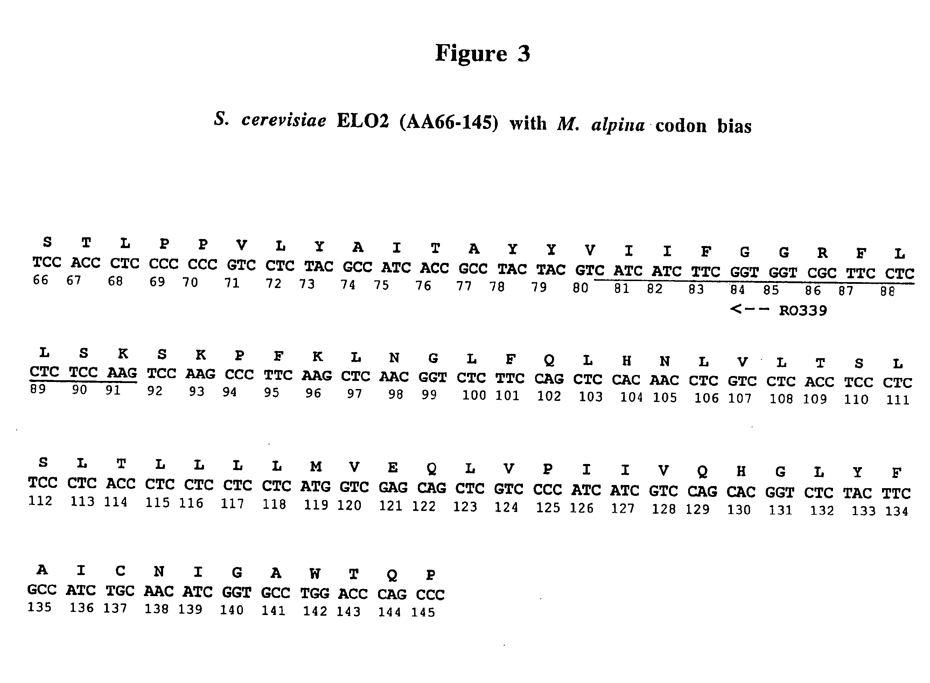

The β-ketoacyl-coenzyme A synthase (KCS) from jojoba and the Saccharomyces cerevisiae elongase (ELO2) were aligned to determine an area of amino acid homology (see Figure 2). The codon bias was applied to the area of sequence corresponding to the homologous amino acids between the two elongases, and primers were designed based on this biased sequence (see Figure 3). The cDNA was excised from the M11 M. alpina cDNA library (Knutzon et al., J. Biol. Chem. 273:29360-29366 (1998)), which contains approximately 6 X 10⁵ clones with an average insert size of 1.1 Kb. The excised cDNA was amplified with internal primer RO339 (5' -TTG GAG AGG AGG AAG CGA CCA CCG AAG ATG ATG- 3') and a vector forward primer RO317 (5'- CAC ACA GGA AAC AGC TAT GAC CAT GAT TAC G - 3'). Polymerase Chain Reaction (PCR) was carried out in a 100 µl volume containing: 300 ng of excised M. alpina cDNA library, 50 pmole each primer, 10 µl of 10X buffer, 1 µl 10 mM PCR Nucleotide Mix (Boehringer Mannheim Corp., Indianapolis, IN) and 1.0 U of Taq Polymerase. Thermocycler conditions in Perkin Elmer 9600 (Norwalk, CT) were as follows: 94°C for 2 mins., then 30 cycles of 94°C for 1 min., 58°C for 2 mins., and 72°C for 3 mins. PCR was followed by an additional extension at 72°C for 7 minutes.

The PCR amplified product was run on a gel, an amplified fragment of approximately 360 bp was gel purified, and the isolated fragment was directly sequenced using ABI 373A DNA Sequencer (Perkin Elmer, Foster City, CA). The sequence analysis package of GCG was used to compare the obtained sequence with known sequences. The sequence was translated in all six reading frames in the GCG Analysis Program using the FastA algorithm (Pearson and Lipman, supra). The Swissprot database (GeneBio, Geneva, Switzerland) of proteins was searched. This translated cDNA fragment was identified as a part of a putative elongase based on the homology of the putative protein sequence to the S. cerevisiae ELO2 (GNS1), having 41.3% identity in 63 amino acids.

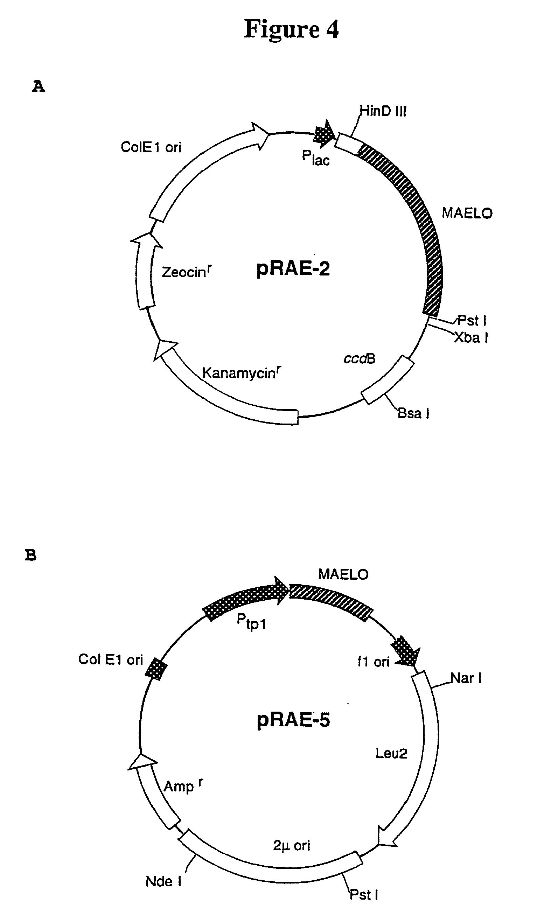

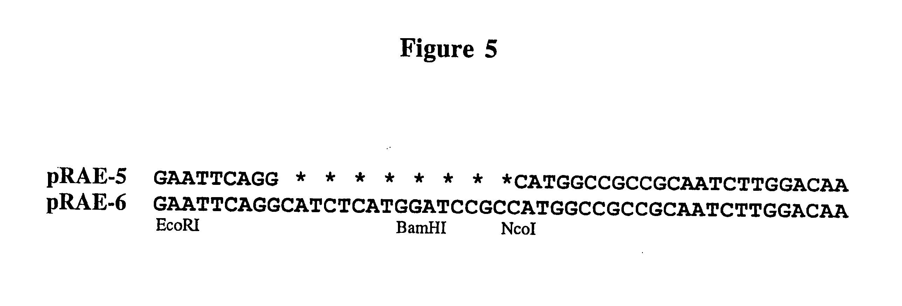

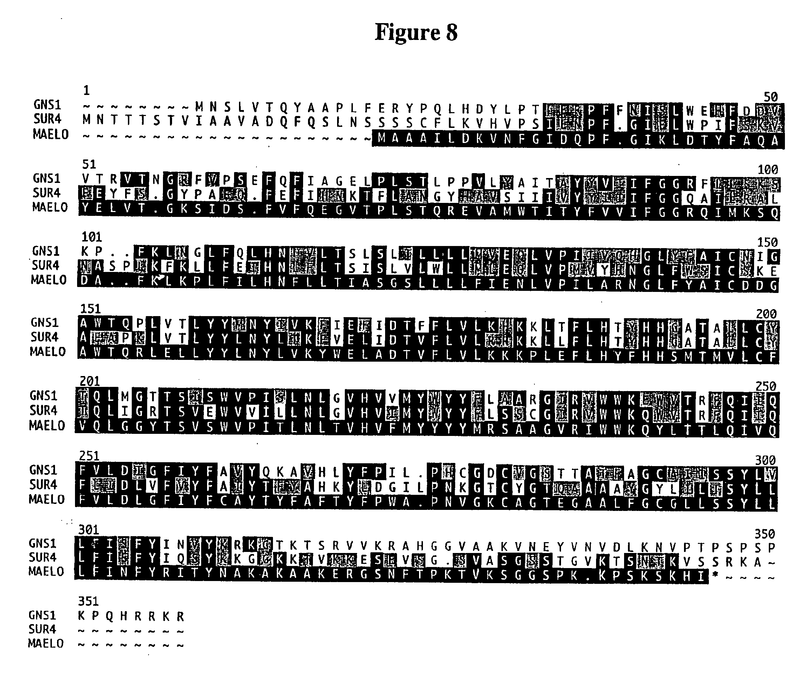

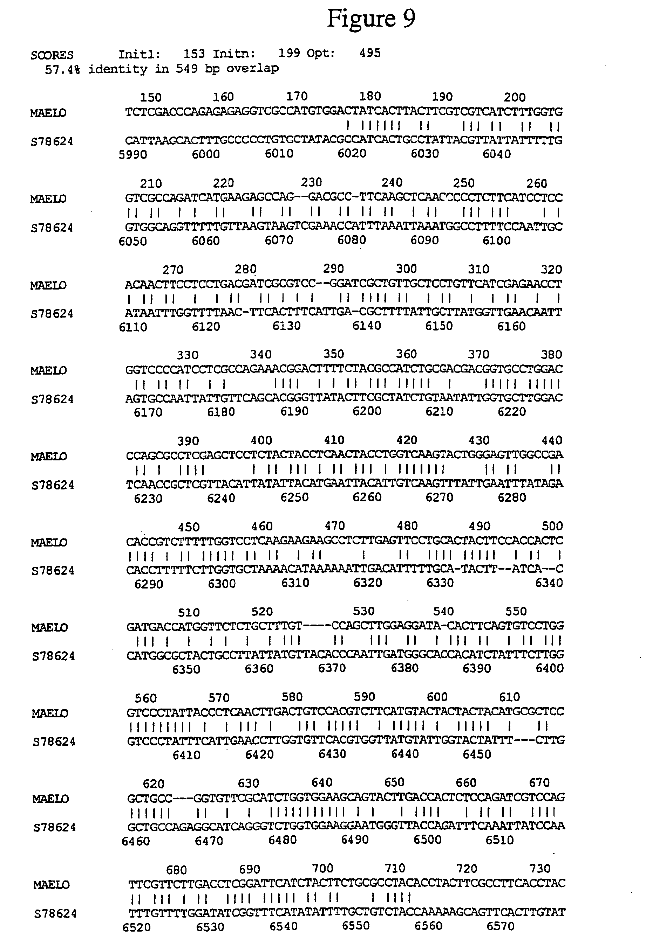

New primers were designed based on the putative elongase sequence and the vector, pZL1 (Life Technologies, Inc., Gaithersburg, MD) sequence used to construct M. alpina cDNA library. The M. alpina excised cDNA library was PCR amplified again using primers R0350 (5' -CAT CTC ATG GAT CCG CCA TGG CCG CCG CAA TCT TG- 3'), which has an added *BamH*I restriction site (underlined), and the vector reverse primer R0352 (5' -ACG CGT ACG TAA AGC TTG- 3') to isolate the full length M. alpina elongase cDNA, using previously described conditions. The termini of the approximately 1.5 Kb PCR amplified fragment was filled-in with T4 DNA polymerase (Boehringer Mannheim Corp., Indianapolis, IN) to create blunt ends and cloned into the pCR-blunt vector (Invitrogen Corp., Carlsbad, CA). This resulted in two clones, pRAE-1 and pRAE-2 (see Figure 4A). (Plasmid DNA pRAE-2 was deposited with the American Type Culture Collection, 10801 University Boulevard, Manassas, VA 20110-2209, on August 28, 1998, under the terms of the Budapest Treaty, and was accorded deposit number ATCC 203166.) The elongase cDNAs from these vectors were cut out as an *Eco*RI fragment and cloned into the *EcoR*I digested pYX242 (Novagen, Madison, WI) vector. The clones pRAE-5 and pRAE-6 (see Figure 4B) have the elongase cDNAs from pRAE-1 and pRAE-2, respectively. (Plasmid DNA pRAE-5 was deposited with the American Type Culture Collection, 10801 University Boulevard, Manassas, Virginia 20110-2209, on August 28, 1998, under the terms of the Budapest Treaty, and was accorded deposit number ATCC 203167.) The sequencing of pRAE-5 and pRAE-6 revealed that 5' untranslated region of the elongase gene in pRAE-5 is 16 bp shorter than that in pRAE-6 (see Figure 5). The complete M. alpina elongase cDNA sequence, designated MAELO was obtained from pRAE-2 (see Figure 6). Figure 7 is the amino acid sequence obtained from the translation of MAELO. The Swissprot database (GeneBio, Geneva, Switzerland) was searched again, as previously described, with the translated MAELO: MAELO has 44.3% identity in 317 amino acids with S. cerevisiae GNS1(ELO2) and 44.7% identity in 318 amino acids with S. cerevisiae SUR4(EL03). The FastA alignment among the three elongases is shown in Figure 8. At the nucleotide level (see Figure 9), MAELO has 57.4% identity in 549 bp overlap with S. cerevisiae GNS1(EL02) (GenBank Accession # S78624). However, the identity between the complete MAELO gene of 954 bp and S. cerevisiae GNS1(ELO2) is 33.0%.

### Example III

### Expression of M. alpina Elongase cDNA in Baker's Yeast

The constructs pRAE-5, and pRAE-6 were transformed into S. cerevisiae 334 (Hoveland et al., Gene 83:57-64 (1989)) and screened for elongase activity. The plasmid pCGN7875 (Calgene LLC, Davis, CA) containing jojoba KCS gene in pYES2 vector (Invitrogen Corp., Carlsbad, CA) was used as a positive control. The substrate used to detect elongase activity in M. alpina elongase (MAELO) was GLA and that in jojoba KCS was oleic acid (OA). The negative control strain was S. cerevisiae 334 containing pYX242 vector. The cultures were grown for 40-48 hours at 25°C, in selective media (Ausubel et al., Short Protocols in Molecular Biology, Ch. 13, p. 3-5 (1992)), in the presence of a particular substrate. The expression of the jojoba KCS gene cloned in pYES2 was under the control of GAL1 promoter, while the promoter in pYX242 is TP1, which is constitutive. Hence, the 334(pCGN7875) and 334(pYES2) cultures were induced with galactose. The GC-FAME analysis of the lipid fraction of each cell pellet was performed as previously described (Knutzon et al., supra).

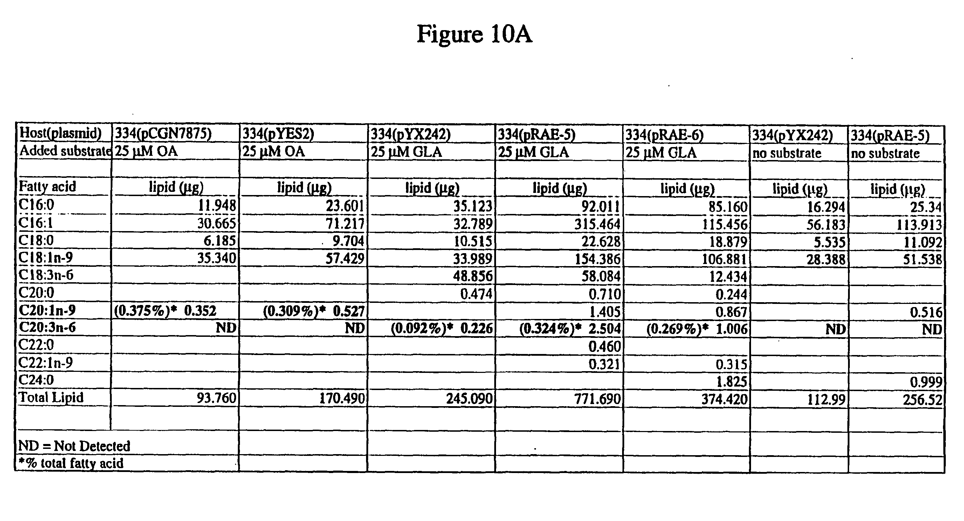

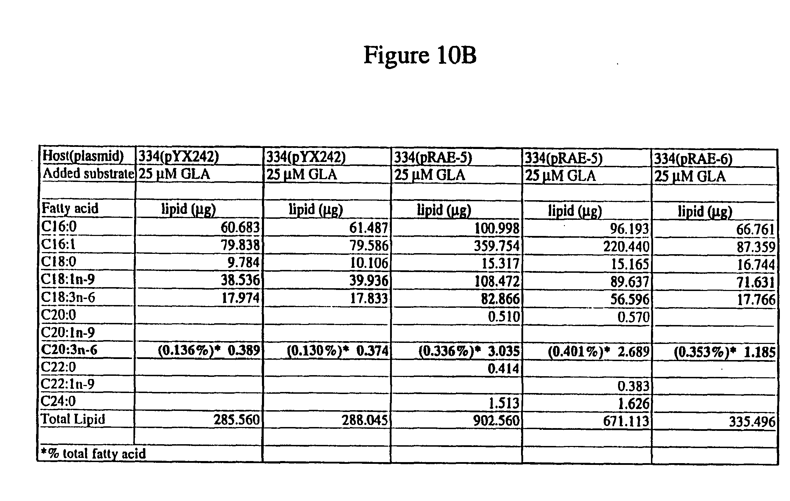

The elongase activity results from different experiments are provided in Figure 10A and 10B. The jojoba KCS elongates long chain monounsaturated fatty acids 18:1n-9 to 20:1n-9. The amino acid homology between the M. alpina elongase (MAELO) and the S. cerevisiae ELO2 and ELO3 suggested that the proteins encoded by these genes may have similar substrate specificity. The activity of the M. alpina elongase, elongation (MAELO) of long chain monounsaturated and saturated fatty acids, is seen in the conversion of 18:1n-9 to 20:1n-9 and also in the synthesis of 24:0. The control strain, 334(pYX242) has very little or no detectable amount of 20:1 and 24:0 (see Figure 10A). M. alpina elongase (MAELO) also acts on at least one PUFA, converting 18:3n-6(GLA) to 20:3n-6(DGLA). The percentage of the 20:3n-6 in total lipid is higher in the strain 334(pRAE-5) and 334(pRAE-6) with the M. alpina elongase (MAELO) cDNA when compared to that in the control 334(pYX242). The percentages of 20:3n-6 produced were 0.092% for 334(pYX242) vs. 0.324% for 334(pRAE-5) and 0.269% for 334(pRAE-6) (shown in parenthesis in Figures 10A and 10B). This difference in the fatty acid profile is also seen in the total amount of 20:3n-6 produced. Only 0.226 µg of 20:3n-6 was produced by 334(pYX242) while 334(pRAE-5) and 334(pRAE-6) produced 2.504 µg of 20:3n-6 and 1.006 µg of 20:3n-6, respectively. Also, when no substrate is added, the level of 20:3n-6 is not detectable.

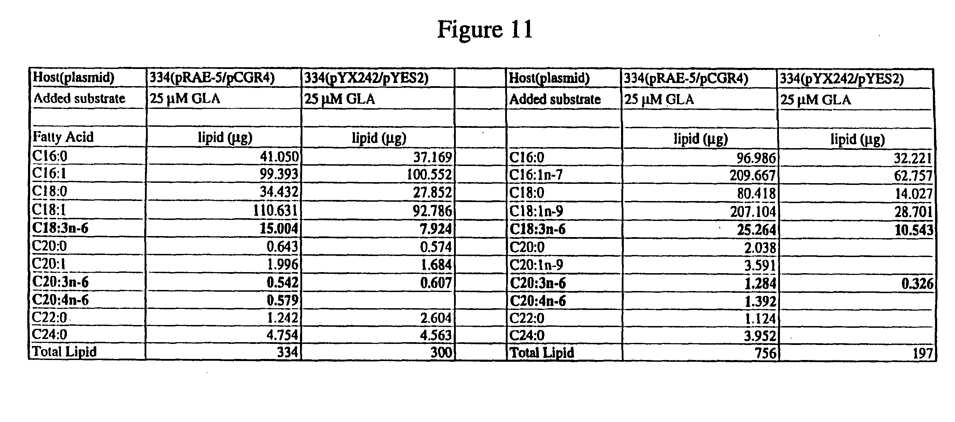

Once 20:3n-6 is generated by the M. alpina elongase (MAELO), the Δ5-desaturase can convert it to AA in the desired expression system. To test this hypothesis, the constructs pRAE-5 and pCGR-4 (a Δ5-desaturase containing plasmid) were co-transformed into S. cerevisiae 334 and screened for AA production. The substrate used was 25 µM GLA (18:3n-6). If the M. alpina elongase (MAELO) is active in yeast, then the substrate will be converted to DGLA(20:3n-6), which the Δ5-desaturase will convert to AA(20:4n-6). The results in Figure 11 confirm the production of AA and therefore, the activity of the M. alpina elongase (MAELO).

The expression of Δ5-, Δ6-, and Δ12-desaturases, in yeast, along with the elongase, should result in the production of AA (see Figure 1) without the need for an exogenous supply of fatty acids.

### Example IV

### A Comparison of the Expression of M. alpina Elongase cDNA MAELO and S. cerevisiae Elongase ELO2 in Baker's Yeast

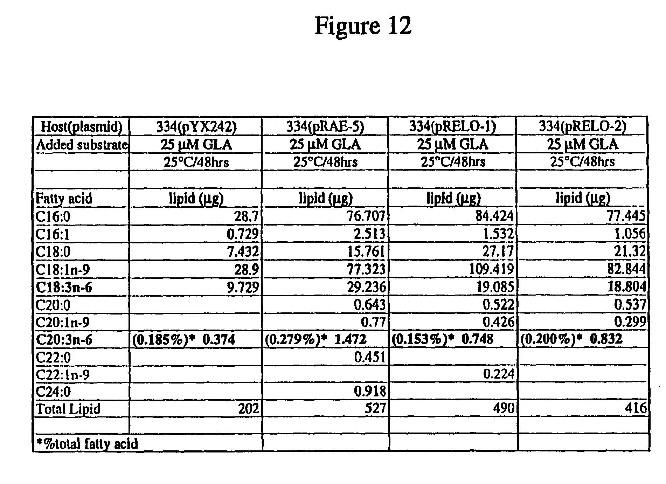

The ELO2 gene encoding for the yeast elongase was cloned from an S. cerevisiae genomic library (Origene, Rockville, MD) using the primers R0514 (5' -GGC TAT GGA TCC ATG AAT TCA CTC GTT ACT CAA TAT G-3') and RO515 (5' -CCT GCC AAG CTT TTA CCT TTT TCT TCT GTG TTG AG-3') incorporating the restriction sites (underlined) *BamH*I and *Hind*III (respectively). The ELO2 gene was cloned into the vector pYX242 at the *BamH*I and *Hind*III sites, designated pRELO, transformed into the S. cerevisiae host 334 (Hoveland et al., supra) and screened for PUFA elongase activity. The vector plasmid was used as a negative control and 334(pRAE-5) was grown to compare the PUFA elongase activity. The cultures were grown as previously described with no galactose in the media and 25 µM GLA added as a substrate. Figure 12 shows that amount of 20:3n-6 or DGLA produced (elongated from 18:3n-6 or GLA) by 334(pRAE-5) was approximately 4 times the negative control containing the unaltered vector pYX242, while the two individual clones 334(pRELO-1) and 334(pRELO-2) were only twice the negative control. Additionally, when DGLA produced is expressed as a percent of the total lipids (shown in parenthesis, Figure 12), the clones 334(pRELO-1) and 334 (pRELO-2) produced 0.153% and 0.2% DGLA respectively, while 334(pYX242) produced 0.185% DGLA. Hence all these strains produced comparable percentages of DGLA. The strain 334(pRAE-5), however, produced 0.279% DGLA, an increase of 50.8% over 334(pYX242) (negative control). These data show that the S. cerevisiae elongase gene EL02, even when overexpressed in yeast, does not elongate GLA to DGLA effectively. The M. alpina PUFA elongase activity is specific for this conversion as evidenced by the higher amount of DGLA produced compared to the control, 334(pYX242).

### Example V

### Identification of Elongases from Other Sources Using MAELO

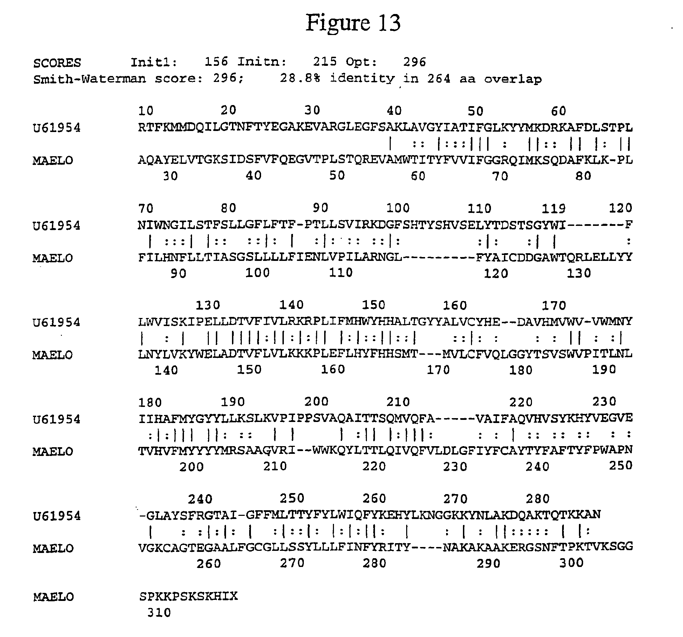

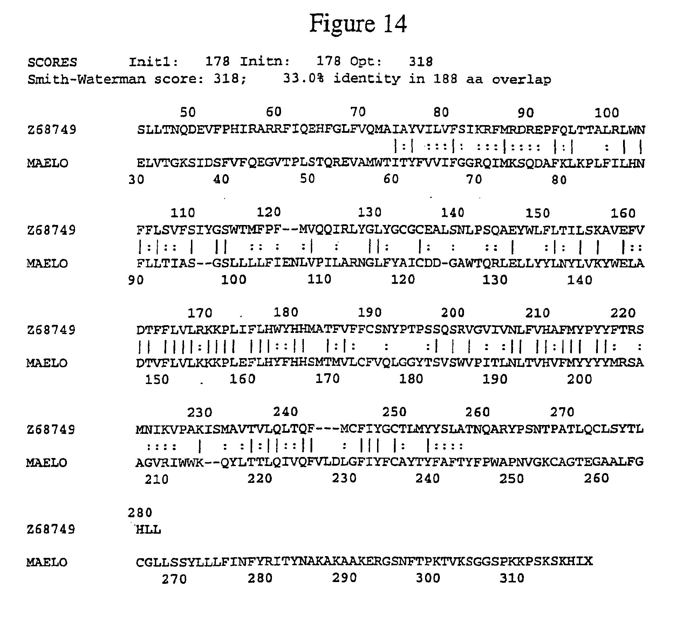

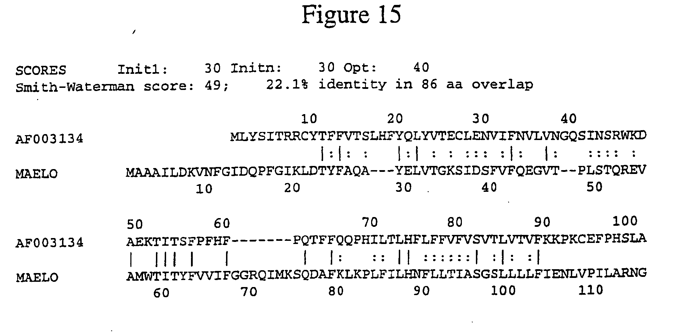

The TFastA algorithm (Pearson and Lipman, supra) is used to search for similarity between a query peptide sequence and the database DNA sequence translated in each of the six reading frames. Translated MAELO was used as the query for a TFastA search in GCG with the GenEMBL database (6/98) from GCG to identify other potential elongase sequences based on their amino acid similarity comparisons to translated MAELO. For example, in Figures 13 and 14, two alignments are shown between translations of two different C. elegans sequences from chromosome III and MAELO. C. elegans DNA sequence (GenBank accession # Z68749) was annotated denoting similarity with GNS1 (ELO2), while the additional C. elegans DNA sequence (GenBank accession # U61954) was noted as similar to both GNS1 and SUR4 (ELO3). These are spliced DNA fragments in which the introns have been removed from the genomic sequence, and the exons assembled and translated. The amount of amino acid identity between the putative PUFA elongases from C. elegans and translated MAELO are around 30%. This would point towards a common function in the fatty acid metabolism, e. g., a PUFA elongase. Figure 15 is another example of a translated C. elegans sequence (GenBank accession # AF003134) from chromosome III. The DNA sequence was identified that had DNA homology to the S. cerevisiae ELO2. Further inspection of this DNA sequence and its amino acid translation determined that there was homology to translated MAELO. C. elegans, therefore, may contain a PUFA elongase.

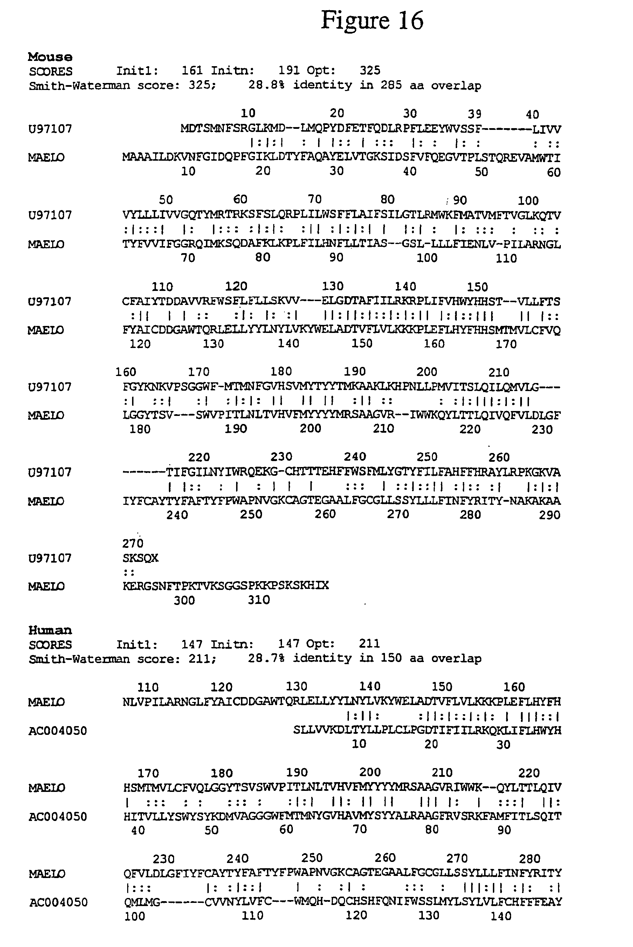

Figure 16 shows the alignments of translated DNA sequences from mouse and human, respectively, with translated MAELO. The mouse sequence CIG30, GenBank accession # U97107, was isolated from brown adipose tissue and reported as being "similar to yeast SUR4 protein". As shown in Figure 16, amino acids numbered 130 to 152 in the U97107 translation contain a high degree of similarity to the translated MAELO. The human sequence, GenBank accession # AC004050, from chromosome 4 was from an HTGS (High Throughput Genome Sequence). There were no annotations contained with this sequence. However, translated AC004050 had 28.7% identity in 150 amino acids with translated MAELO. This gene fragment could be a fragment of a human PUFA elongase based on its amino acid similarity to translated MAELO.

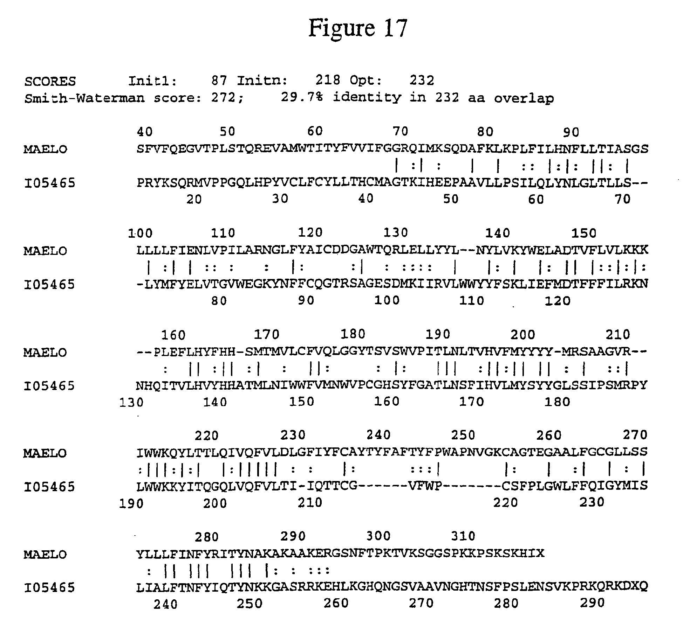

Figure 17 shows the amino acid alignment of translated MAELO and a mammalian sequence (GenBank accession # 105465, PCT# WO 88/ 07577) which claims that the protein derived from expression of this sequence is a glycoslylation inhibition factor. The amino acid identities between the two proteins, signifying that there could be related function, such as PUFA elongase activity.

These examples of other translated DNA sequences and their homology to the translated MAELO illustrate that any of the above examples could potentially be a PUFA elongase. These examples are not inclusive of all the possible elongases. However, use of MAELO or its amino acid translation as a query for database searches can identify other genes which have PUFA elongase activities.

### Example VI

### M. alpina cDNA Library Screening Using A Plaque Hybridization Method

In an effort to isolate additional PUFA elongase genes from M. alpina, a conventional plaque hybridization method was used to screen an M. alpina cDNA library made in a lambda vector. The DNA probe was generated based on MAELO nucleotide sequence and was used to screen the M7+8 M. alpina cDNA library made in a λZiplox vector (Knutzon et al., J. Biol. Chem. 273:29360-29366 (1998)).

To make the DNA probe for screening the library, the MAELO cDNA was digested with *Nsp*I and *Pvu*I restriction endonucleases. Three small DNA fragments, with an average size of approximately 300 bp, were produced and used as probes. The rationale for using a mixture of fragmented MAELO cDNA was based on the assumption that there might be a common region or domain in the amino acid sequence which is conserved among various PUFA elongases present in M. alpina. Using MAELO DNA probes, the cDNA library was screened by a plaque hybridization technique according to standard protocol (Sambrook et al., Molecular Cloning. 2nd Ed., Cold Spring Harbor, 1989).

Briefly, 50,000 primary clones were plated and transferred to nylon membranes. The membranes were denatured and hybridized with alpha ³²P-dCTP-labelled MAELO DNA probes overnight in the hybridization buffer which contained 20% formamide, 0.2% PVP, BSA, Ficoll, 0.1% SDS and 0.5 M NaCl. The filters were washed with 0.5X SSC at 37°C and exposed to X-ray film for autoradiography. This procedure was repeated three times. Four clones (designated as F1, F2, F3, and F4) which hybridized repeatedly were picked and suspended in SM buffer (Sambrook et al., supra) containing 7% DMSO.

The largest open reading frame of each candidate was subcloned into yeast expression vector pYX242 (Novagen, Inc., Madison, Wisconsin). The cDNA clones F1 and F3 were subcloned into pYX242 at the *Eco*RI site while F2 and F4 were subcloned at *Nco*I/*Hind*III sites. The recombinant pYX242 containing each candidate was transformed into SC334 (Hoveland et al., supra) for expression in yeast. To determine the elongase activity, as well as substrate specificity, SC334 containing each cDNA clone was grown in minimal media lacking leucine in the presence of 25 µM of GLA substrate as described in Example III. The fatty acid analysis was performed as described in Knutzon et al. (J. Biol. Chem. 273:29360-29366 (1998)). The results indicated that none of these four cDNA clones showed any significant activity in converting GLA to DGLA. Thus, the hybridization approach appeared to be unsuccessful in identifying additional PUFA elongases.

### Example VII

### Construction of Direct cDNA Expression Library of M. alpina in Yeast

To identify PUFA elongase genes other than MAELO, a different approach was taken to screen the M. alpina cDNA library. In particular, since Baker's yeast is incapable of producing long chain PUFAs due to the absence of respective desaturases and elongases, an attempt was made to construct an expression cDNA library of M. alpina in Saccharomyces cerevisiae. The vector pYES2 (Novagen, Inc., Madison, Wisconsin), containing the GALL promoter, was chosen for the expression of cDNA library in S. cerevisiae.

The conventional way by which a cDNA library is made (i.e. transformation of cDNA/vector ligated DNA mixture into host cells) is difficult in yeast because the transformation efficiency by direct electroporation of ligated DNA mix is very low compared to the efficiency of purified supercoiled plasmid DNA. However, the major advantage of this method is to avoid amplification of primary clones which happens when the library is made in E. coli as an intermediate. Due to the limitation in the number of colonies to be screened, it was decided to first optimize the efficiency of transformation in different S. cerevisiae strains using cDNA/vector ligated mix. The best results were obtained with a yield of 4-5 *x* 10⁵ transformants per µg of ligated DNA in S. cerevisiae strain SC334 (Hoveland et al., supra).

To make a direct M. alpina cDNA expression library in yeast total RNA was isolated from the fungus. M. alpina fungus (ATCC # 32221) was plated onto cornmeal agar (Difco Laboratories, Detroit, MI) and grown at room temperature for 3-4 days. Once fungus growth was visible, it was inoculated into 50 ml of potato dextrose broth and shaken at room temperature very slowly to formulate spores. Once spores were visible, the 50 ml culture was inoculated into a 1 liter culture of potato dextrose, and spores were grown for 72 hours. After filtering through sterile gauze, the cells were immediately frozen into liquid nitrogen for future RNA extraction. Total RNA was prepared from 36 g of cell pellet using the hot phenol/LiCl extraction method (Sambrook et al., supra). The cell pellets were homogenized in a 10 mM EDTA, 1% SDS and 200 mM sodium acetate, pH 4.8 solution. Phenol and chloroform were added to the homogenates, and the aqueous layer was extracted. The aqueous layer was back extracted one more time with phenol and chloroform. Then an equal volume of 4 M lithium chloride was added. The samples were ethanol precipitated on ice for 3 hours, and pellets were obtained by centrifugation. The RNA pellets were washed with 70% ethanol and resuspended in DEPC treated water. Total RNA was quantitated by spectrophotometry and visualized by agarose gel electrophoresis to confirm the presence of 28S and 18S ribosomal bands. Approximately, 15 mg of total RNA were obtained from 36 gram of cell pellet.

The library was constructed according to the standard protocol (Sambrook et. al., Molecular Cloning, 2nd Ed.. Cold Spring Harbor, 1989). Messenger RNA was prepared from the total RNA using oligo dT cellulose affinity purification. Messenger RNA was reverse transcribed with oligo dT primer containing a *Xho*I restriction site using AMV reverse transcriptase. Following first strand cDNA synthesis, the second strand of cDNA was synthesized by adding E. coli DNA polymerase, E. coli DNA ligase and RNAse H.

The *EcoR*I adaptor was ligated into the blunt-ended cDNA by T4 DNA ligase. The cDNA sample was kinased using T4 polynucleotide kinase and digested with *Xho*I, diluted with column buffer and passed through a Sephacryl S-400 column. The DNA samples were eluted by high salt buffer. Samples containing DNA from 400-5,000 bps were pooled and used for ligation into a pYES2 vector (Invitrogen Corp., Carlsbad, CA). The cDNA was ligated into the *EcoR*I/*Xho*I digested pYES2 vector using T4 DNA ligase. A large scale ligation reaction was carried out since a large amount of the ligated DNA (2-3 µg) is required in direct transformation of yeast.

To transform yeast cells directly with the cDNA/pYES2 ligated mixture, competent SC334 cells were prepared using the LiAc TRAFO method (Gietz et. al., Mol. Cell. Biol, 5: 255-269, 1995). Briefly, fresh culture of SC334 from the plate was inoculated into 50 ml YPD medium. The culture was grown at 30 °C with shaking until the OD at 600 had reached 1.0. Thirty ml of this starter was inoculated into 300 ml of YPD liquid medium and incubated with shaking until the cell number of the culture reached - 3-5 x 10⁶ cell/ml (approximately 3-4 h). The cells were harvested and washed with sterile water. The entire cell pellet was resuspended in 1.5 ml of freshly prepared 1X TE/LiAc (0.1M LiAc). These cells were used immediately for the transformations.

Seven hundred and fifty microliters of competent SC334 cells were aliquoted into 15 ml falcon tubes. Approximately 2 ug of cDNA/pYES2 ligated DNA were added to the cells along with carrier DNA and mixed gently. Three milliliters of sterile 40% PEG/LiAc was added to the cells and mixed gently but thoroughly. The cells were incubated at 30 °C for 30 min with shaking and subsequently given heat shock at 42 °C for 15 min. The cells were cooled, pelleted, and resuspended in 5 ml of 1X TE. A 100 *u*l aliquot of the above cells was plated onto fifty 150 mm selective agar plates lacking uracil (Ausubel et al., supra) and incubated at 30 °C for 3 days. A total of 8 *x* 10⁵ primary clones were obtained. Five colonies were pooled in 1 ml minimal media lacking uracil (Ausubel et al., supra) and glycerol added to prepare stocks. A total of 5,000 pools were made for screening.

### Example VIII

### MAD (M. alpina Direct) Screening in Yeast

The quality of the library was analyzed by determining the average size of the cDNAs in the library. Since the screening of the library was based on the expression of the cDNA, it was important to determine the average size of the cDNA present in the library. The expression library containing the longest cDNAs would be the best appropriate choice to isolate full-length cDNAs of interest. To this end, randomly selected pools were plated onto selective agar plates, as described in Example VII, to obtain individual colonies. Forty different yeast colonies were randomly picked, and each colony was inoculated into 5 ml of selective liquid medium lacking uracil (as described in Example VII) and grown, while shaking, for 24 hours at 30 °C. Plasmid DNA was extracted from these colonies by the bead beating method (Hoffman et al., Gene 57:267 (1987)) adapted as follows:

Pellets from 5 ml of culture were lysed in 0.5 ml of a 100 mM NaCl, 10 mM Tris, pH 8.0, 1 mM EDTA and 0.1% SDS solution. Sterile 0.5 mm glass beads of equal volume were added and manually vortexed for 3 minutes. Two hundred microliters of the same buffer were added, and the mixture was vortexed for an additional minute. The samples were centrifuged on high for 2 minutes, and cytoplasmic extract was then transferred to a fresh tube. An equal volume of phenol/CHCl₃ was added to the sample, vortexed and centrifuged again for 2 minutes. The aqueous layer was re-extracted twice and precipitated with 0.3 M sodium acetate and approximately 2.5 volumes of ethanol for 30 minutes at -20 °C. The precipitates were washed with 70% ethanol and resuspended in water. To eliminate RNA and any protein contamination, the plasmid DNAs isolated from 40 different samples were further purified using the QIAprep Spin Miniprep Kit according to the manufacturer's protocol (Qiagen Inc., Valencia, CA). The plasmid DNA samples were then restricted with *EcoR*I and *Xho*I restriction endonucleases to release the cDNA fragment, and the digest was analyzed on 1% agarose gel. The results indicated that the majority of the cDNAs of the direct library varied in length from 0.8 Kb to 1.5 Kb.

To screen the library, the glycerol stocks were thawed and approximately 0.5 ml was added to 5 ml of liquid selective media lacking uracil (Ausubel et al., supra) and grown at 30°C for 24 hours. The culture was then transferred into 50 ml of liquid selective medium lacking uracil with 2% galactose and 25 µM GLA (substrate for the elongase enzyme) for 24 hours at 25 °C with shaking. The GC-FAME analysis of the lipid content in the cell pellet of each induced culture was performed as previously described (Knutzon et al., supra). The MAELO (pRAE-5 in pYX242 grown in selective media lacking leucine) was used as a positive control in each batch run. MAELO had consistently been able to convert 1.5% of GLA to DGLA (see Example III).

### Example IX

### Identification of a cDNA Encoding a Potential PUFA Elongase

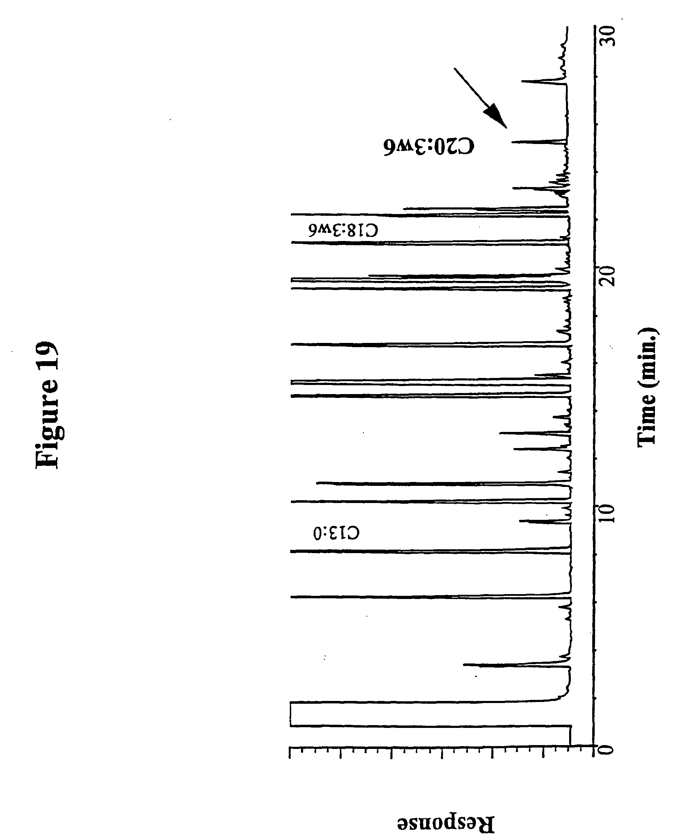

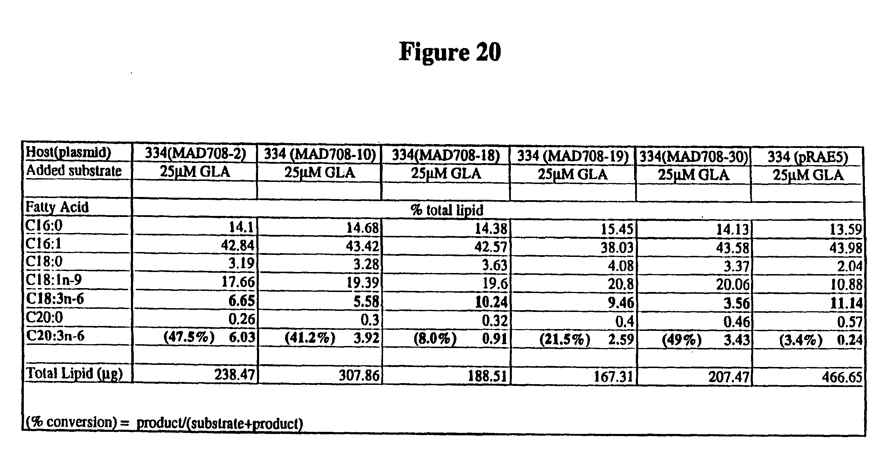

After screening and analyzing approximately 750 individual pools by GC-FAME analysis, as described in Example VIII, one pool of five colonies (i.e., MAD708) appeared to have significant enzymatic activity in converting GLA to DGLA. This activity was found to be approximately 5 fold higher than the M. alpina elongase activity (MAELO) in terms of DGLA/GLA ratio (Figure 19). This pool was tested again under identical assay conditions to confirm the initial findings. The repeat experiment showed 9.5% conversion of GLA to DGLA and was again around 5 fold higher than M. alpina elongase activity (MAELO). These results strongly indicated that the MAD708 pool contained an elongase candidate which was specific for GLA as substrate. Since MAD708 was a pool of five different clones, it was necessary to isolate the individual cDNA clone which encoded for elongase activity from this pool. To do this, the original MAD708 glycerol stock was plated onto a selective media agar plate lacking uracil (Ausubel et al., supra). Thirty individual colonies were picked and grown in liquid selective medium, lacking uracil with 2% galactose, as previously described in Example VIII, in the presence of GLA. The cell pellet obtained from each culture was then subjected to fatty GC-FAME analysis (Knutzon et al., supra) along with a positive control of 334 (pRAE-5)(MAELO in pYX242). The fatty acid analysis from the 30 individual clones from the MAD708 expression pool in yeast revealed that 5 of the 30 clones showed elongase activity in converting GLA to DGLA. The fatty acid profiles of the active clones MAD708-2, MAD708-10, MAD708-18, MAD708-19 and MAD708-30 are shown in Figure 20. As shown in this Figure, MAD708-2, 10, and 30 produced the most DGLA, approximately 25 fold more than MAELO (pRAE-5). These 3 converted in the range of 41% to 49% of GLA to DGLA. Other clones, MAD708-18 and MAD708-19, converted 8% and 21% of GLA to DGLA, respectively. All MAD708 clones converted a higher percentage of GLA to DGLA with respect to MAELO encoded elongase (3.4%).

### Example X

### Characterization of cDNAs Encoding Elongase

Plasmid DNA was extracted from SC334 yeast clones (MAD708 pool) that showed significant GLA specific elongase activity by the bead beating method, as described in Example VIII. To determine the size of the cDNA insert, PCR was performed using each plasmid DNA obtained from positive elongase clones as a template. The forward primer RO541 (5'- GAC TAC TAG CAG CTG TAA TAC -3') and the reverse primer R0540 (5'- GTG AAT GTA AGC GTG ACA TAA -3') are in the multicloning site of the pYES2 vector and were used to amplify the cDNA insert within the *Eco*RI and *Xho*I sites. PCR reaction was performed in a 50 µl volume containing 4 µl of plasmid DNA, 50 pmole of each primer, 5 µl of 10X buffer, 1 µl 10 µM PCR Nucleotide Mix (Boehringer Mannheim Corp., Indianapolis, IN) and 0.5 µl of High Five Taq polymerase (Boehringer Mannheim, Indianapolis, IN). The amplification was carried out as follows: 2 mins. denaturation at 94 °C, then 94 °C for 1 min, 55 °C for 2 mins., and 72°C for 3 mins. for 30 cycles, and 7 mins. extension at 72°C at the end of the amplification. Analysis of PCR amplified products on a 1% agarose gel showed the sizes of the elongase cDNAs to be around 1.0 - 1.2 Kb. The plasmid DNAs, containing the potential elongase cDNAs, were designated as pRPB2, pRPB10, pRPB18, pRPB19, and pRPB30. Since the cDNA library was made in the pYES2 vector at the *EcoR*I and *Xho*I sites, the size of the cDNA present in each plasmid was further confirmed by digesting the above plasmids with *EcoR*I and *Xho*I.

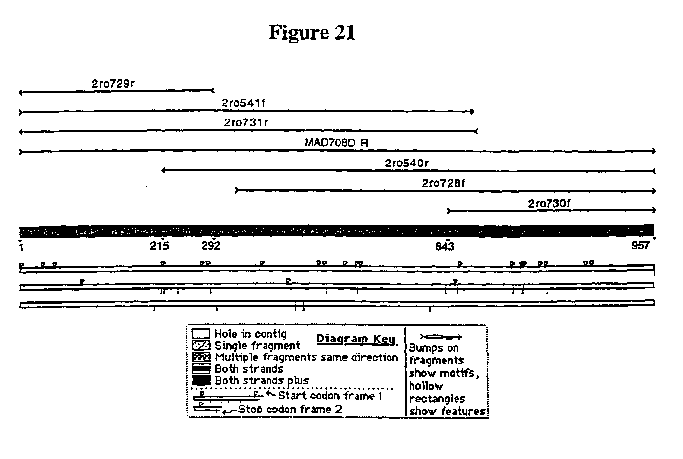

The plasmid DNAs isolated from yeast were re-amplified in E. coli for long-term storage of the cDNA clones as well as for DNA sequencing. E. coli TOP10 (Invitrogen Corp., Carlsbad, CA) cells were transformed with the pRPB recombinant plasmids according to the manufacturer's protocol. The transformants obtained from each plasmid DNA were inoculated into LB containing ampicillin (50 µg/ml) and grown overnight at 37 °C with shaking. Plasmid DNAs were isolated from these cultures by using QIAprep Spin Miniprep (Qiagen Inc., Valencia, CA) according to the manufacturer's protocol. The purified plasmid DNAs were then used for sequencing from both 5' and 3' ends. The DNA sequencing was performed by using a 373A Stretch ABI automated DNA sequencer (Perkin Elmer, Foster City, CA) according to the manufacturer's protocol. Primers used for sequencing were the forward primer RO541 (5'- GAT TAC TAG CAG CTG TAA TAC -3') and the reverse primer R0540 (5'- GTG AAT GTA AGC GTG ACA TAA -3') contained in the multicloning sites of the pYES2 vector. The obtained nucleotide sequences were transferred to Sequencher software program (Gene Codes Corporation, Ann Arbor, MI) for analysis. The DNA sequence analysis revealed that all five elongase cDNAs contained the identical nucleotide sequence with a common overlap of 301 nucleotides. Each DNA sequence contains a putative start site at the beginning of the 5' end and a stop codon with poly A tail at the end of the 3' site. To further confirm the DNA sequence, internal forward primers R0728 (5'- GAG ACT TTG AGC GGT TCG -3') and RO730 (5'- TCT CTG CTG CGT TGA ACT CG -3'), along with reverse primers RO729 (5'- AAA GCT CTT GAC CTC GAA C -3') and R0731 (5'- AAC TTG ATG AAC GAC ACG TG -3') were designed within the cDNA, and used for sequencing of pRPB2, since this candidate possessed the highest elongase activity. The entire nucleotide sequence was analyzed by the Sequencher program (Figure 21), and the longest open reading frame deduced from the entire cDNA sequence in pRPB2 appeared to be 957 bp in length (Figure 22). The deduced open reading frame was then translated into the corresponding amino acid sequence, and the predicted sequence is shown in Figure 23. The elongase encoded by the cDNA (pRPB2) identified from M. alpina appears to be a 318 amino acid long protein which is nearly identical in size with translated MAELO. This new elongase cDNA was designated as "GLELO" and its encoded protein has been named "GLA elongase".

Plasmid DNA pRPB2 was deposited with the American Type Culture Collection, 10801 University Boulevard, Manassas, Virginia 20110-2209 on July 22, 1999 under the terms of the Budapest Treaty. It was accorded ATCC Deposit # PTA-402.

### Example XI

### Biochemical Characterization of GLA Elongase (GLELO)

### A. Confirmation of GLA Elongase Activity

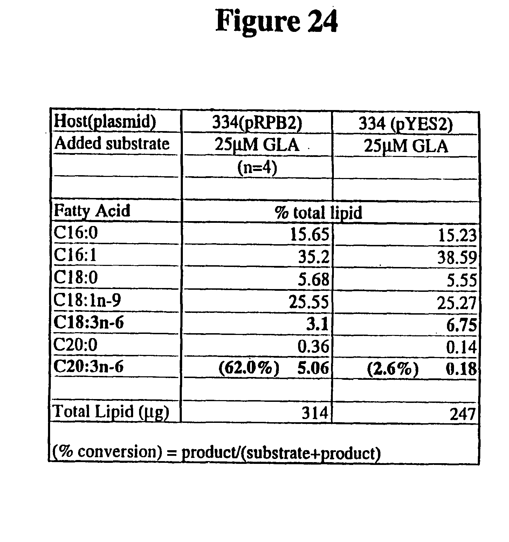

To further confirm the activity of the GLA elongase encoded by the pRPB2 recombinant plasmid, elongase activity screening was repeated on the yeast clone SC334 containing pRPB2 plasmid. This experiment was also conducted to assure consistent lipid extraction and to detect the activity of GLA elongase by averaging four independent experiments. The S. cerevisiae 334 glycerol stock containing pRPB2 was plated onto minimal media agar plates lacking uracil. Individual colonies were randomly picked and grown in minimal medium lacking uracil, as described in Example VIII. The four independent cultures were combined, and a 5 ml aliquot was used as an inoculum for four separate 50 ml cultures. The cultures were then grown in the presence of GLA and were subjected to fatty acid analysis along with a negative control of S. cerevisiae 334 containing pYES2, as described in Example VIII. The average elongase activity from four independent cultures of 334(pRPB2) with 25 µM GLA is shown in Figure 24. The GLA elongase activity of each of the four independent samples of 334(pRPB2) appeared to be consistent with an average conversion of 62% GLA to DGLA.

### B. Determination of GLELO Substrate Specificity for GLA Elongase

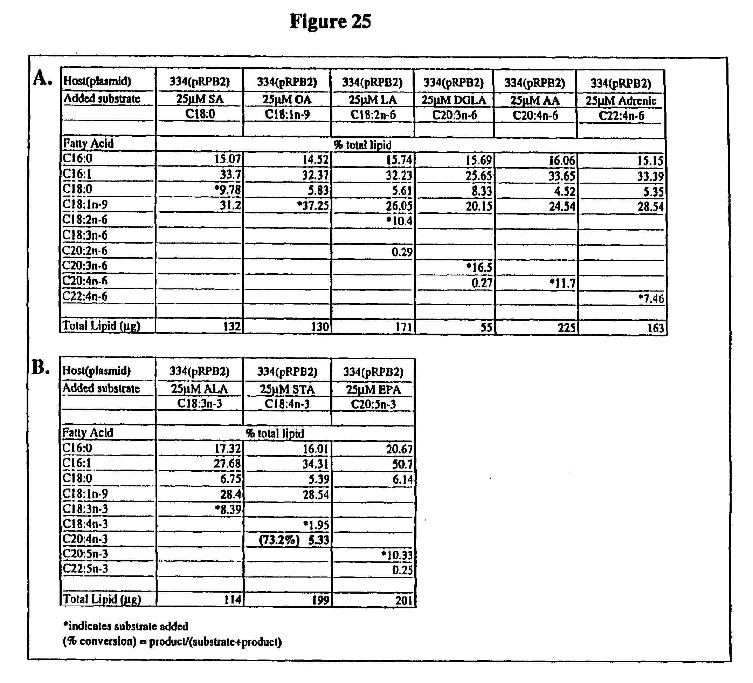

To analyze the substrate specificity of the GLA elongase, the culture of 334(pRPB2) was tested with different fatty acid substrates besides GLA (e.g., SA(18:0), OA(18:1), LA(18:2n-6), AA(20:4n-6), ADA(22:4n-6), ALA(18:3n-3), STA(18:4n-3), and EPA(20:5n-3)). Under identical assay conditions, the only other substrate utilized by the elongase enzyme was STA, a fatty acid from the n-3 pathway. GLA elongase was able to convert 73% of STA to 20:4n-3 (Figure 25). From these experiments, it can be concluded that the GLA elongase has substrate specificity for both GLA and STA, indicating that it possesses elongase activity along both the n-6 and n-3 pathways.

### C. Co-expression of Fungal GLELO and Δ5-Desaturase Gene in Yeast

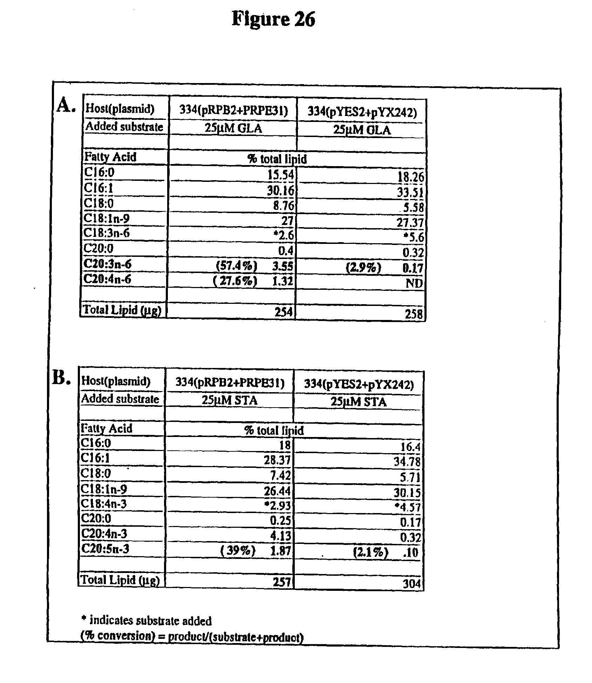

Once DGLA (20:3n-6) is produced by the GLA elongase, the Δ5-desaturase can convert it to AA (20:4n-6) in a desired co-expression system. This scheme, as depicted in Figure 1, can be tested by co-transforming S. cerevisiae 334 with plasmids pRPB2 and pRPE31 (the recombinant plasmid pYX242 containing a Δ5-desaturase cDNA (Figure 18) cloned at the *Eco*RI site. The co-transformed yeast cultures were supplemented with 25µM GLA and analyzed for AA synthesis. If both elongase and Δ5-desaturase enzymes are expressed, the GLA substrate will be converted to DGLA, which will then be converted to AA. The results in Figure 26A indicate that the sequential action of GLA elongase and Δ5-desaturase on GLA substrate resulted in an average conversion of 27% GLA to AA. Therefore, the GLA elongase has the ability to work with other enzymes in the n-6 PUFA synthetic pathway to produce desirable fatty acids.

To determine whether the above conversion is also true in n-3 pathways, the similar co-expression experiments were carried out in the presence of 25 µM STA. Again, if both enzymes are expressed, the STA substrate will be converted to 20:4n-3 which will then be converted to EPA (20:5n-3) by the Δ5-desaturase. Figure 26B shows the results in which the production of EPA (approx. 40%) is observed. Once again, the GLA elongase demonstrates its ability to work with Δ5-desaturase in the n-3 pathway to produce desirable fatty acids.

### Example XII

### Sequence Comparison Between GLELO and Other Fungal Elongases

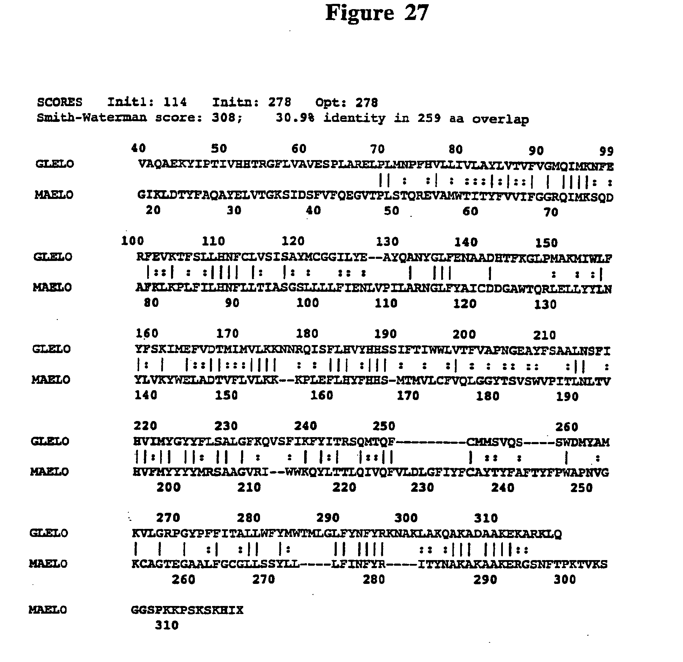

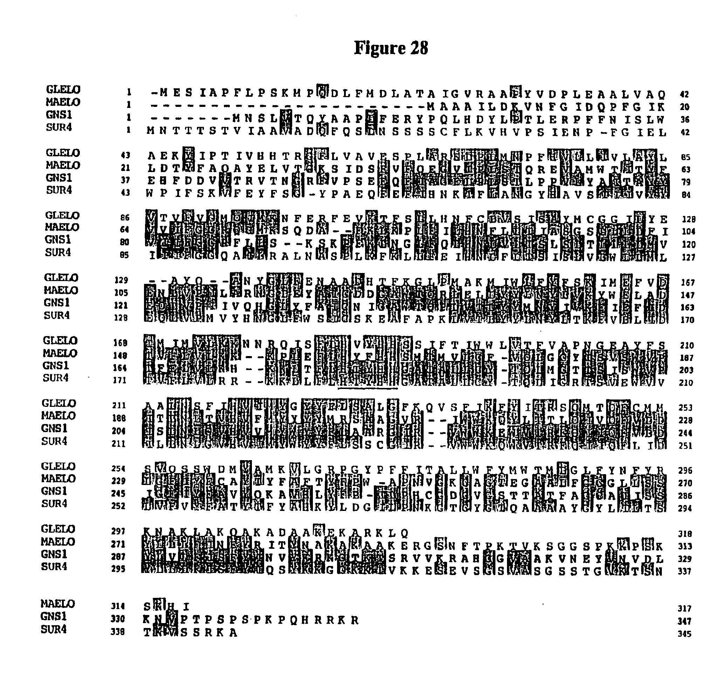

The sequence analysis package of GCG (see Example I) was used to compare the GLELO sequence with known protein sequences. The nucleotide sequence of GLELO open reading frame was first translated into amino acid sequence that was used as a query sequence to search Swissprot database (see Example I) using the FastA algorithm (see Example I). Based on amino acid sequence similarity, the best matches were found with S. cerevisiae YJT6 (an EST with unknown annotation) with 33.9% identity in 189 amino acid overlap, S. cerevisiae ELO2 (GNS1) with 25.8% identity in 295 amino acid overlap, and S. cerevisiae ELO3 (SUR4) with 25.2% identity in 313 amino acid overlap. The FastA alignment of GLELO with MAELO showed 30.9% identity in 275 amino acids (Figure 27). GCG Pileup program creates a multiple sequence alignment from a group of related sequences using progressive, pairwise alignments (see Example I), and was used with the elongases described above. The Pileup results indicate that there are many conserved regions among the elongases including a putative histidine box, which is underlined (Knutzon et. al., J. Biol. Chem. 273: 29360-29366, 1998) (Figure 28). Thus, although GLELO has similarity with MAELO, the difference in their encoded elongases may presumably be due to their substrate preference. GLA elongase can convert a higher percentage of GLA to DGLA than M. alpina elongase. In addition, MAELO expression in S. cerevisiae showed elongation of saturated and monounsaturated fatty acids in addition to GLA elongation to DGLA (see Example III).

### Example XIII

### Identification of M. alpina MAELO Homologues in Mammals

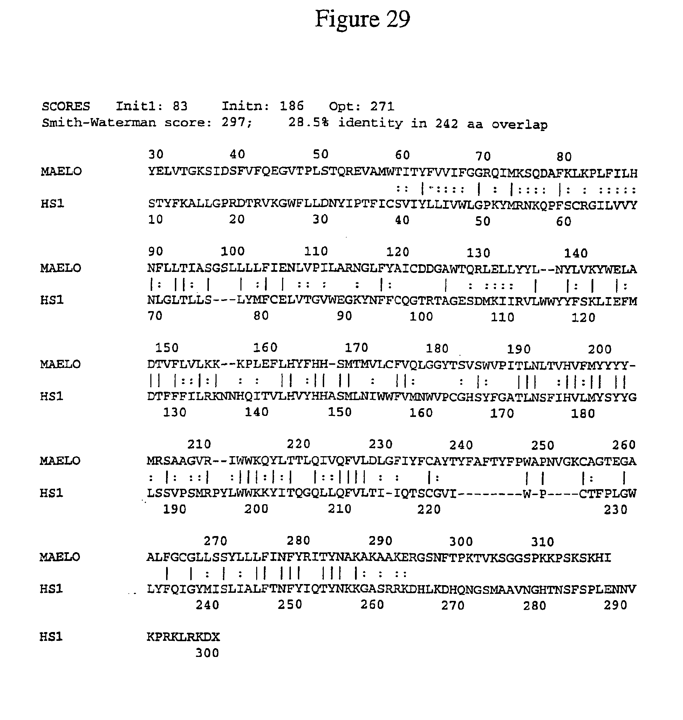

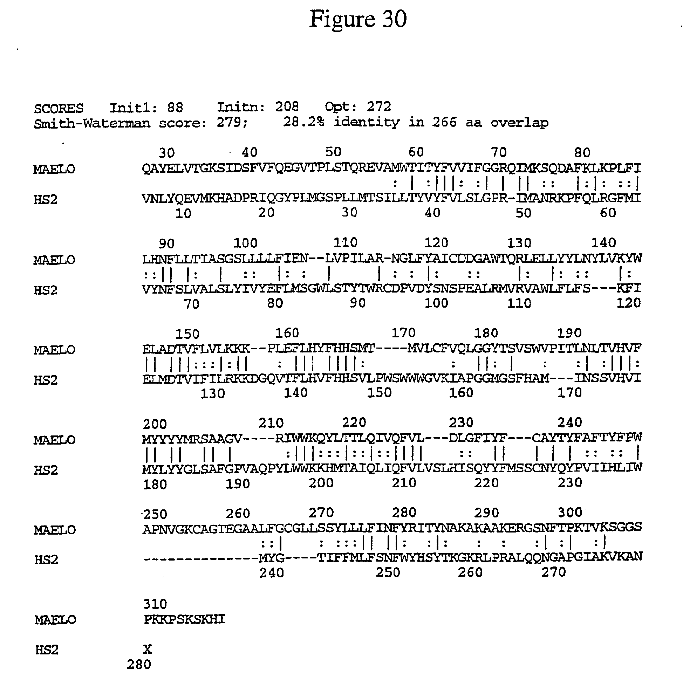

The MAELO translated sequence was used to search the Unified Human Transcript Database of Abbott Laboratories, 100 Abbott Park Rd., Abbott Park, Illinois 60064. This database was searched using Basic Local Alignment Search Tool (BLAST) (Altschul et al., Nuc. Acids Res. 25:3389-3402 (1997)) which "is a set of similarity search programs designed to explore all of the available sequence databases regardless of whether the query is a protein or DNA." Specifically, the tblastn algorithm was used (i.e., a protein query search to a nucleotide database translated in six reading frames). The contig (CC) sequences in the Unified Human Transcript Database are consensus sequences representing groups of expressed sequence tags (EST) cDNAs derived from the public domain and from the Incyte LIFESEQ^{™} database of ESTs (Incyte Pharmaceuticals, Inc., 3174 Porter Drive, Palo Alto, CA 94304) that are clustered together on the basis of defined sequence homology, and assembled on the basis of sequence overlap. Two sequences from this database, CC067284R1 and CC1484548T1 had 28% identity in 242 amino acid overlap and 28.6% identity in 266 amino acid overlap, respectively, with the translated MAELO sequence. The two derived and edited sequences were designated as hs1 and hs2, respectively, and copied into the sequence analysis software package of GCG (see Example I). The translated MAELO sequence was aligned with translated HS1 (28.5% identity in 242 amino acids) and HS2 (28.2% identity in 266 amino acids) cDNA sequences using the FastA algorithm, as shown in Figures 29 and 30, respectively. HS1 cDNA nucleotide sequence also had 86.9% identity in 844 bp with the I05465 nucleotide sequence (see Example V). The translated HS2 cDNA sequence had 100% identity with the amino acid sequence from GenBank with accession number W74824 (see published PCT application WO9839448).

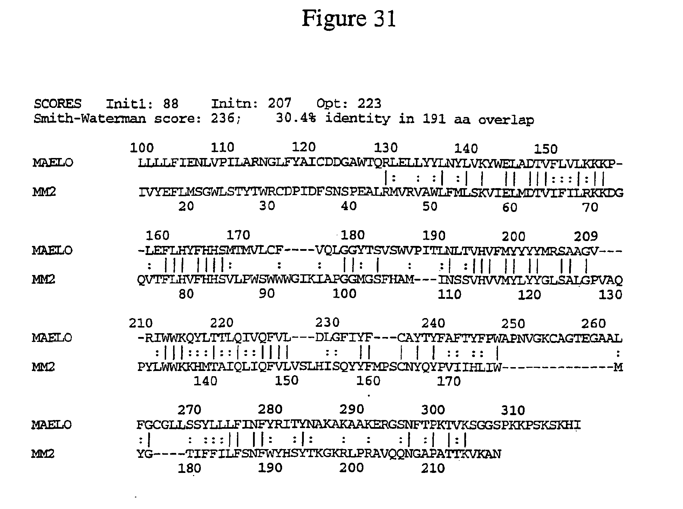

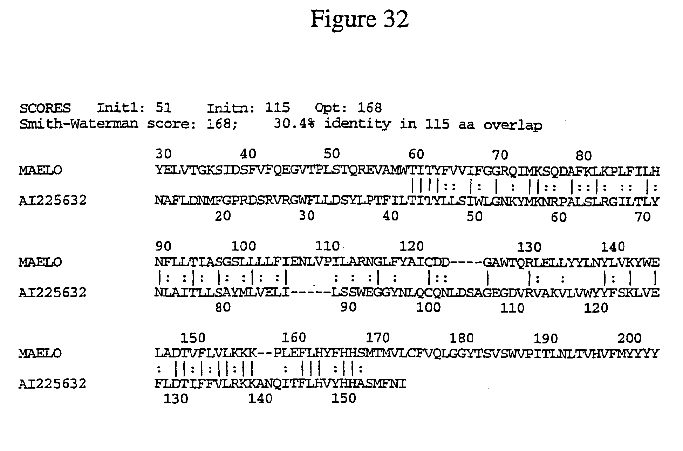

The National Center for Biotechnology Information (NCBI at http://www.ncbi.nlm.nih.gov/) was used to conduct database searches using tblastn with the 28 amino acid sequence (DTIFIILRKQKLIFLHWYHHITVLLYSW) translated from AC004050 (a human sequence identified in a TFastA search, see Example V). This amino acid sequence contains a histidine box (underlined), which has a noted motif of desaturases (Knutzon et al., supra), and both PUFA elongases, MAELO and GLELO (see Figure 28). A translated mouse sequence shown previously in Example V (GenBank Accession #U97107) and a translated C. elegans sequence (GenBank Accession # U41011) had the highest matches with this 28 amino acid query. The NCBI mouse EST database was searched again with tblastn, using translated U41011 as a query. An additional mouse sequence was identified (GenBank Accession #AF014033.1), annotated as "putative involvement in fatty acid elongation." Three longer sequences (GenBank Accession #'s AA591034, AA189549, and AA839346) were identified through a tblastn search of the mouse EST database with translated AF014033.1 and combined into one sequence designated as mm2. The FastA alignment (see Example I) of translated mm2 and MAELO is shown in Figure 31. Another related, but not identical mouse sequence (GenBank Accession #AI225632), was also identified in a tblastn search of the mouse EST database with AF014033.1. The FastA alignment with translated AI225632 to MAELO is shown in Figure 32. The percent identity for both translated MM2 and AI 225632 with translated MAELO is 30.4% in 191 and 115 amino acid overlap, respectively. The level of amino acid identity with translated MAELO with these two translated mouse sequences identifies them as putative homologues of PUFA elongases.

### Example XIV

### Identification of M. alpina GLELO Homologues in Mammals

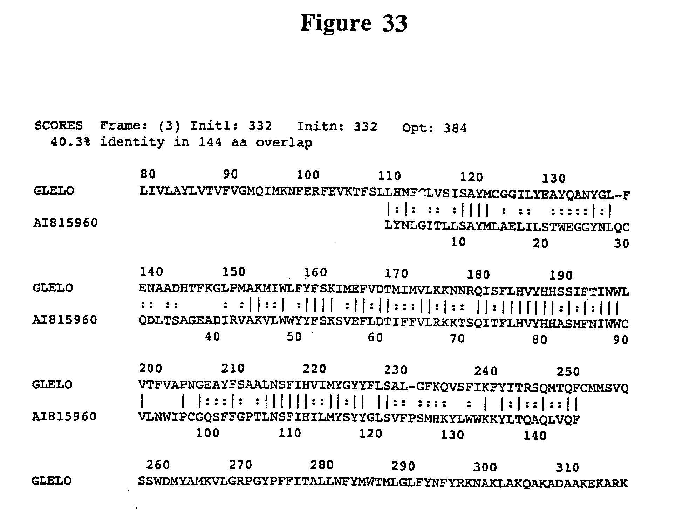

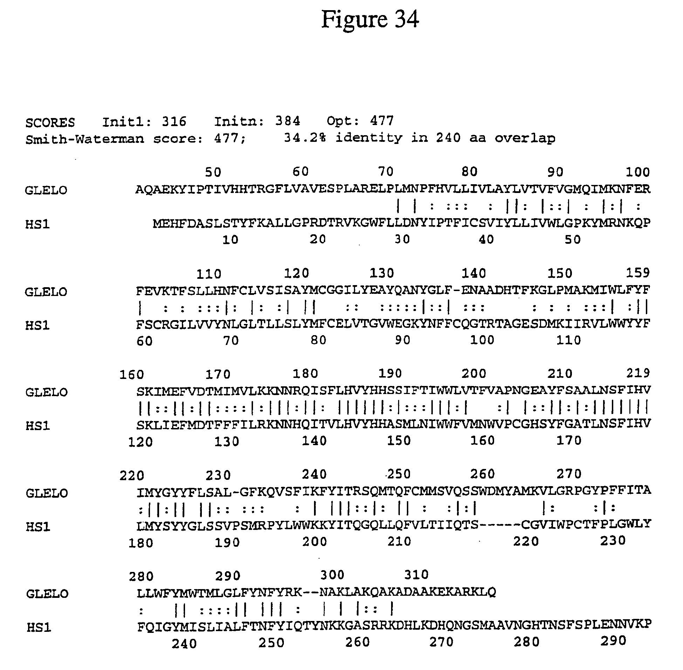

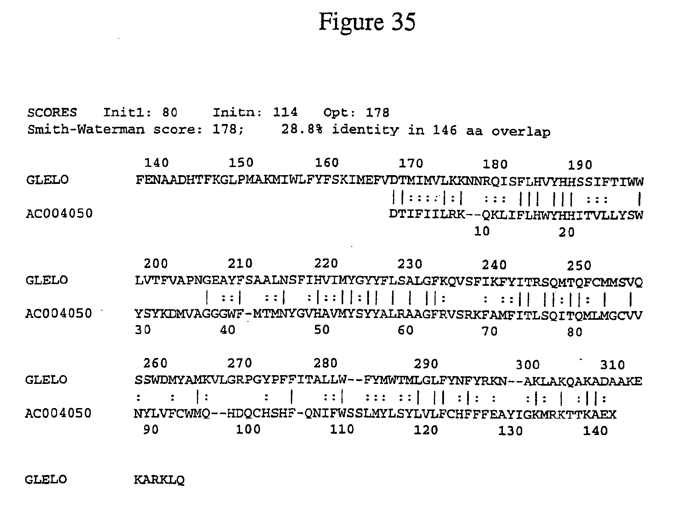

The TFastA algorithm, which compares a protein sequence to the database DNA sequence translated in each of the six reading frames, was used with translated GLELO as the query. The GenEMBL database from GCG was used to identify other potential elongase sequences based on their amino acid similarity to translated GLELO. Three human sequences were found to have matches with the GLELO amino acid sequence. These sequences have GenBank accession numbers 1) AI815960, 2) AL034374, and 3) AC004050. AI815960, a Homo sapien EST sequence, has 40.3% identity in 144 amino acid overlap with translated GLELO (see Figure 33). A translated region of the human genomic sequence AL034374, derived from chromosome VI has 46.7% identity in a 60 amino acid overlap with translated GLELO. This homologous region in AL034374 appeared to be a part of the HS1 amino acid sequence which was shown to have homology with translated MAELO (see Example XIII). Therefore, HS1 sequence has similarity with both MAELO (see Figure 29) as well as GLELO (see Figure 34). A translated region of a human genomic sequence AC004050 from chromosome IV has 34.8% identity in 89 amino acid overlap with translated GLELO (see Figure 35). The amino acid identities between GLELO and these human sequences indicate that the proteins dervied from these human sequences could have related function, such as PUFA elongase activity.

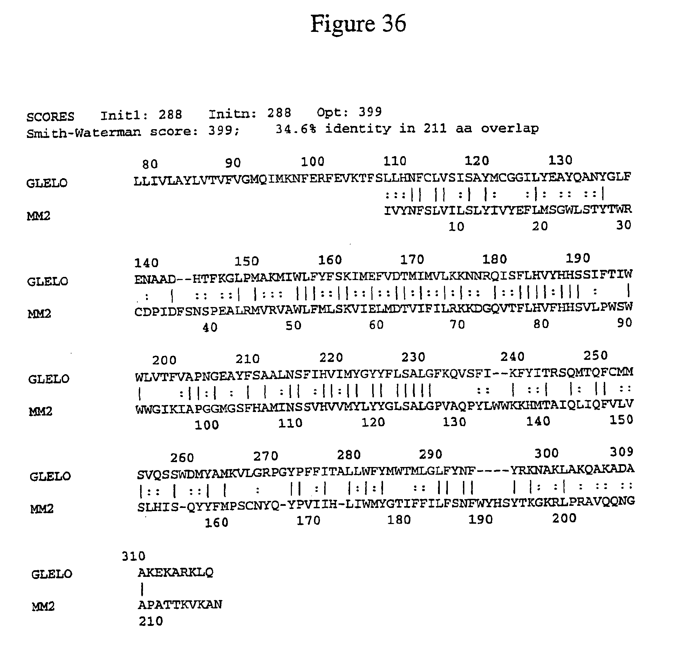

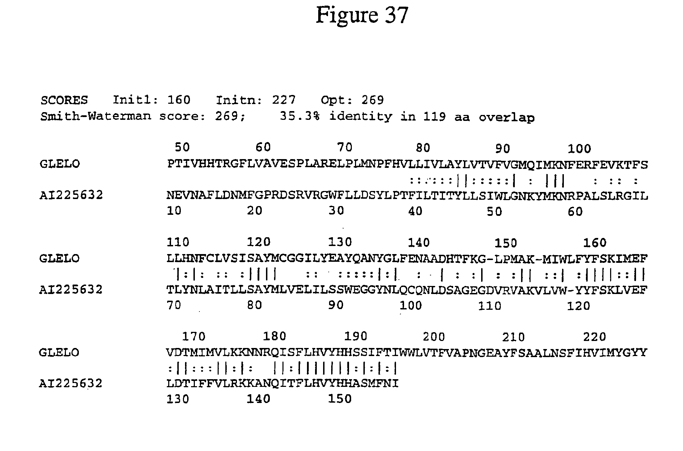

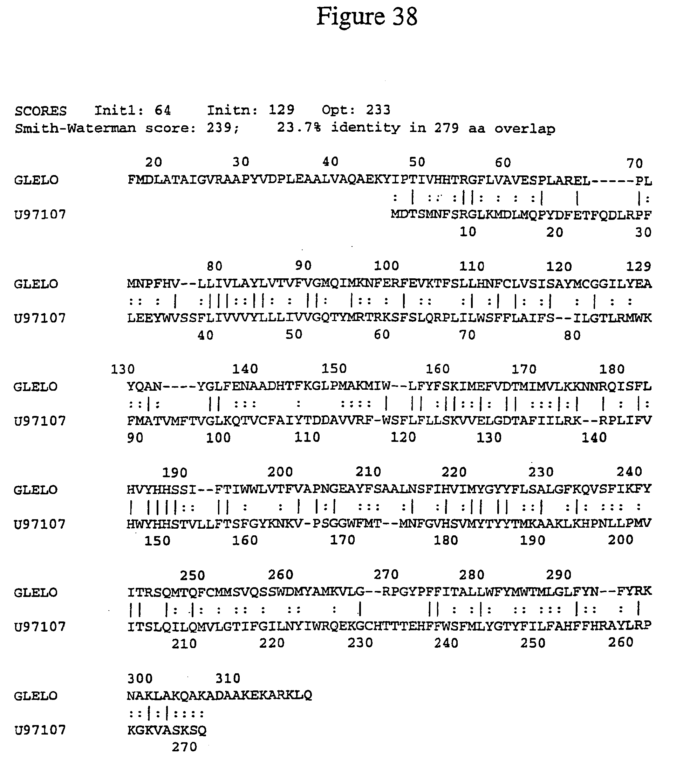

To identify a mouse cDNA similar to GLELO, TFastA searches were performed with the GenEMBL database using translated GLELO as a query. From the TFastA searches, the three mouse sequences with the highest matches to translated GLELO were identified: (GenBank accession numbers 1) AF104033, 2) AI595258, and 3) U97107). AF104033 is annotated as "MUEL protein having putative fatty acid elongase with homology to yeast ELO3 (SUR4)" and is a part of the sequence of MM2. The MM2 sequence was initially derived from AF104033 mouse sequence, but the entire MM2 sequence was finally obtained through further mouse EST database searches and also shown to have homology with translated MAELO (see Example XIII and Figure 31). When this MM2 amino acid sequence was aligned with translated GLELO sequence using FastA, a 34.6% identity in 211 amino acid overlap was found (see Figure 36) indicating that MM2 also has homology with GLELO. AI595258 is a mouse cDNA clone having 5' similarity with yeast ELO3 elongase and is part of mouse EST cDNA AI225632. The AI225632 mouse sequence, which is a longer sequence than AI595258, was shown to have similarity with translated MAELO (see Figure 32). The AI225632 was also aligned with the translated GLELO, and the FastA alignment is shown in Figure 37. A 35.3% identity in 199 amino acid overlap has been found. The third sequence, U97107, a mouse sequence, was annotated as "similar to yeast EL03 (SUR4) gene." The FastA alignment of translated GLELO with U97107 is shown in Figure 38 where a 23.7% identity in 279 amino acid overlap was found. Previously, a region of U97107 was also found to have a high degree of homology with MAELO based on a FastA alignment (see Example V and Figure 16).

The above searches clearly indicate that the same human and mouse sequences were obtained by using either MAELO or GLELO as a query.

### Example XV

### Identification of M. alpina GLELO and MAELO Homologues in Other PUFA Producing Organisms

### A) Caenorhabditis elegans:

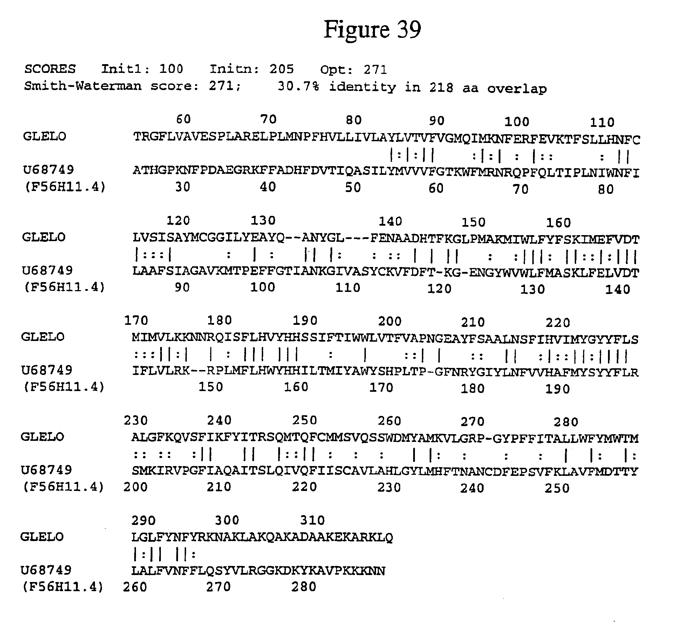

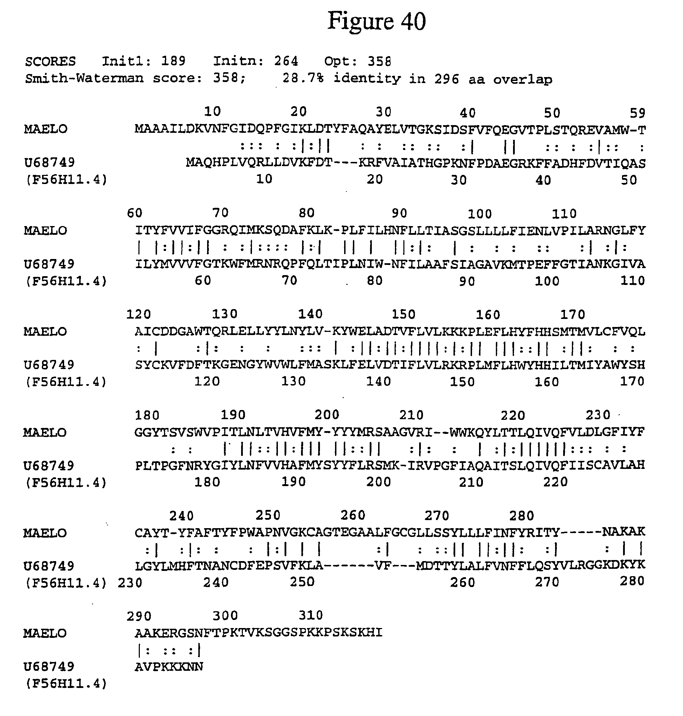

A putative amino acid sequence deduced from a chromosomal sequence of C. elegans (GenBank Accession # U41011) was able to identify a partial sequence contained in the mouse MM2 putative PUFA elongase which has amino acid similarity with both GLA elongase (GLELO) and M. alpina elongase (MAELO). It was therefore conceivable that C. elegans homologues of GLELO or MAELO might be present in the nematode database. The putative amino acid sequences derived from GLELO and MAELO sequences were used as queries independently to search the nematode databases. A BLAST search (see Example XIII) was performed on wormpep16 (blastp compares an amino acid query sequence against a nucleotide sequence database) and wormpep 16cDNAs (tblastn) databases which are predicted proteins and cDNAs obtained from the C. elegans genome sequencing project or EST's and their corresponding cDNA sequences, respectively. These sequence data were produced by the C. elegans Sequencing group, carried out jointly by the Sanger Centre and Genome Sequencing Center, and can be obtained from ftp://ftp.sanger.ac.uk/pub/ databases/wormpep/. At least seven putative C. elegans translated sequences were identified by their amino acid sequence homology to the translated amino acid sequence of both GLELO and MAELO. The GenBank Accession #'s of those genomic sequences containing the deduced amino acids were identified as Z19154, U68749 (2 deduced proteins (F56H11.4 and F56H11.3 (wormpep Accession #'s)), U41011, U61954 (2 deduced proteins (F41H10.7 and F41H10.8 (wormpep Accession #'s)), and Z81058. Those underlined were identified in a previous search using translated MAELO as query (see Example V). As an example, the FastA amino acid alignments of translated U68749 (F56H11.4) with translated GLELO and MAELO are shown in Figures 39 and 40. Translated U68749 (F56H11.4) has 25-30% identity with both M. alpina elongase and GLA elongase in approximately a 200 amino acid overlap (see Figures 39 and 40). For all seven translated putative C. elegans cDNAs, the FastA alignments to translated GLELO was between 25-30% identity in a 200 amino acid overlap, while the identity was 26-34% in at least a 188 amino acid overlap for translated MAELO. The alignment similarities indicate that either translated GLELO or MAELO can be used to identify potential genes from C. elegans with elongase activity.

### B) Drosophila melanogaster:

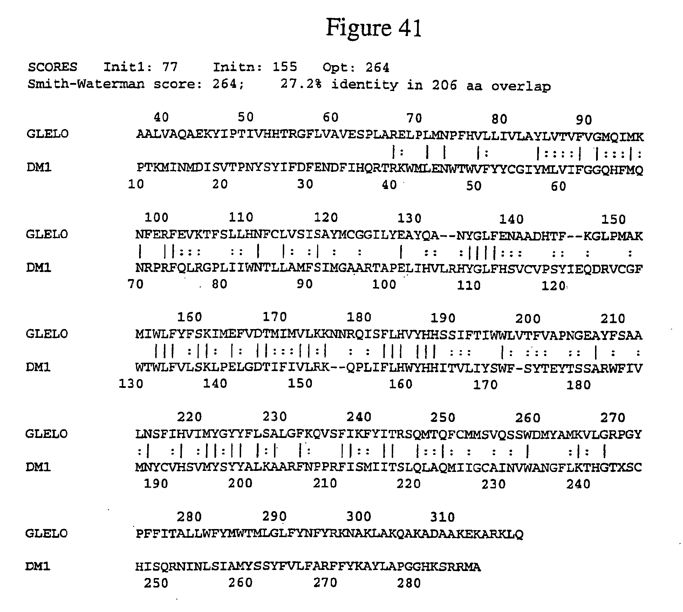

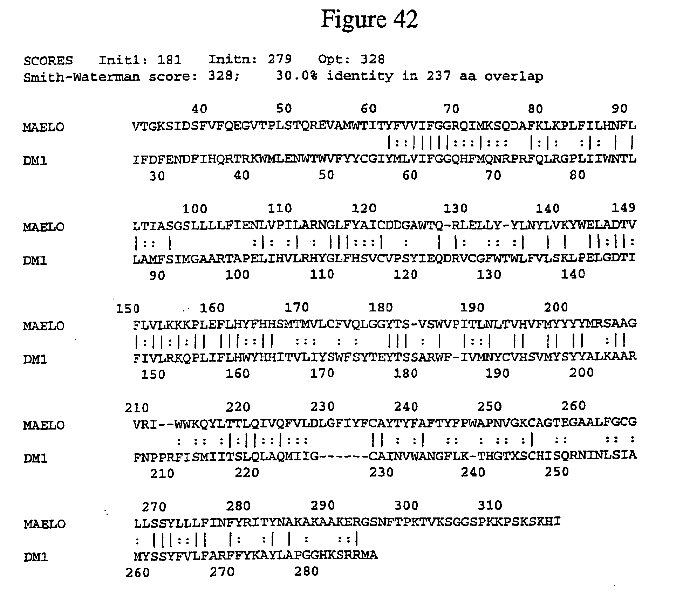

The translated deduced cDNA from the genomic sequence U41011 (C. elegans) had its highest match with a Drosophila melanogaster EST, accession number AI134173 in a blastn search (compares a nucleotide query sequence against a nucleotide database) of the "other ESTs" database through NCBI (see Example XIII) and was assembled with an overlapping DNA EST fragment, accession number AI517255. The translated DNA fragment DM1, derived from the two overlapping sequences was aligned with translated GLELO as well as MAELO (see Figures 41 and 42) using FastA in GCG (see Example I). The alignments showed 27.2% identity with GLA elongase in a 206 amino acid overlap and 30% identity with M. alpina elongase in a 237 amino acid overlap. Thus, based on amino acid similarity, the DM1 could be a potential homologue to GLELO or MAELO having PUFA elongase-like activity. Moreover, using DNA sequences of GLELO and MAELO as queries for database searches, homologues with PUFA elongase activity from Drosophila can be identified.

### Example XVI

### Cloning and Expression of A Human PUFA Elongase Homologue

Many potential PUFA elongase sequences were identified based on their amino acid similarities to translated GLELO and/or MAELO. To determine the potential elongase activities of these sequences, the cDNA encoding the full length protein is then identified, cloned, and expressed, as demonstrated in the present example.

Primers RO719 (5' -GGT TCT CCC ATG GAA CAT TTT GAT GCA TC-3') and RO720 (5' -GGT TTC AAA GCT TTG ACT TCA ATC CTT CCG- 3') were designed based on the putative HS1 sequence, and used to amplify the human liver Marathon-Ready cDNA (Clontech Laboratories, Inc., Palo Alto, California). The polymerase Chain Reaction (PCR) was carried out in a 50 µl volume containing: 5 µl of human liver Marathon-Ready cDNA, 50 pmole each primer, 1µl 10 mM PCR Nucleotide Mix (Boehringer Mannheim Corp., Indianapolis, IN), 5 µl 10 X buffer and 1.0 U of Advantage KlenTaq Polymerase Mix (Clontech Laboratories, Inc., Palo Alto, CA). Thermocycler conditions in Perkin Elmer 9600 (Norwalk, CT) were as follows: 94 °C for 2 mins, then 30 cycles of 94 °C for 1 min., 58 °C for 2 mins, and 72 °C for 3 mins. PCR was followed by an additional extension cycle at 72 °C for 7 minutes.

The PCR amplified product was run on a gel, an amplified fragment of approximately 960 bp was gel purified, the termini of the fragment filled-in with T4 DNA polymerase (Boehringer Mannheim, Corp., Indianapolis, IN), and cloned into pCR-Blunt Vector (Invitrogen Corp., Carlsbad, CA) following manufacturer's protocol. The new plasmid was designated as pRAE-52, and the putative PUFA elongase cDNA in this clone was sequenced using ABI 373A Stretch DNA Sequencer (Perkin Elmer, Foster City, CA). The putative PUFA elongase cDNA sequence in plasmid pRAE-52 is shown in Figure 43, and the translated sequence is shown in Figure 44.

The putative PUFA elongase cDNA from plasmid pRAE-52 was then digested with *Nco*I/*Hind*III*,* gel purified, and ligated into pYX242(*Nco*I/*Hind*III). The new plasmid was designated as pRAE-58-A1. (Plasmid 58-A1 was deposited with the American Type Culture Collection, 10801 University Boulevard, Manassas, VA 20110-2209 on August 19, 1999, under the terms of the Budapest Treaty and was accorded deposit number ___.)

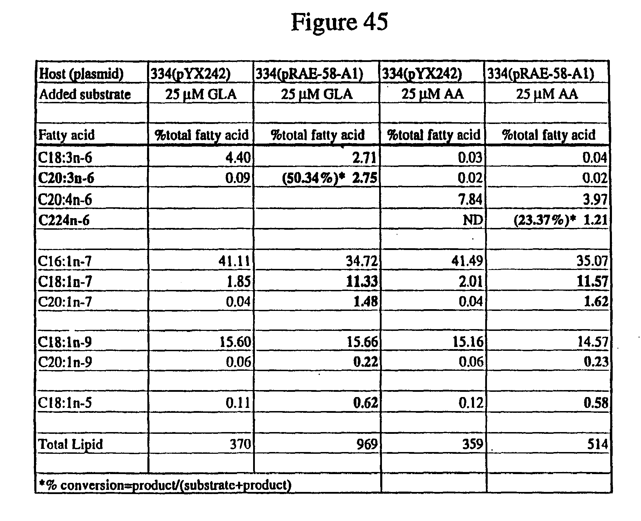

The construct pRAE-58-A1 was transformed into S. cerevisiae 334 (Hoveland et al., supra) and screened for elongase activity. The negative control strain was S. cerevisiae 334 containing pYX242 vector. The cultures were grown for 24 hours at 30°C, in selective media (Ausubel et al., supra), in the presence of 25 µM of GLA or AA. In this study, DGLA or adrenic acid (ADA, 22:4n-6), respectively, was the predicted product of human elongase activity. When GLA was used as a substrate, the yeast cells containing the human elongase cDNA contained elevated levels of DGLA compared to control cells, 2.75% vs. 0.09% of total fatty acids, respectively (see Figure 45). When AA was used as a substrate, the yeast cells containing the human elongase cDNA contained elevated levels of ADA compared to control cells, none detected vs. 1.21% of total fatty acids, respectively. Thus, the human elongase converts both 18 and 20 carbon chain long PUFAs to their respective elongated fatty acids.

The yeast cells containing the human elongase cDNA also had elevated levels of monounsaturated fatty acids including 18:1n-7, 20:1n-7, 20:1n-9, and 18:1n-5, compared to the control strain. Therefore, these results indicate that the identified human elongase is capable of utilizing PUFAs as well as monounsaturated fatty acids as substrates. Thus, this human sequence HSELO1, and its encoded protein, possess elongase activity independent of substrate specificity.

### Example XVII

### Cloning and Expression of a C. elegans PUFA Elongase

Several putative C. elegans elongases were identified with amino acid homology to both translated GLELO and MAELO. As with the human cDNA sequence, cloning of a cDNA and expression in yeast was used to determine if indeed it was a PUFA elongase. Primers RO738 (5' -AAT CAG GAA TTC ATG GCT CAG CAT CCG CTC GTT CAA C -3') and RO739 (5' -CCG CTT GTC GAC TTA GTT GTT CTT CTT CTT TGG CAC -3') with restriction sites *EcoR*I and *Sal*I (underlined), respectively, were based on the putative cDNA sequence contained in the genomic sequence U68749 (wormpep cDNA accession #F56H11.4.) A PCR amplification was performed in a 100 µl volume containing: 250 ng excised C. elegans library cDNA (OriGene Technologies Inc., Rockville, MD), 50 pmole each primer, 10 µl 10X reaction buffer (Boehringer Mannheim Corp., Indianapolis, IN), 1 µl 10 mM PCR Nucleotide mix (Boehringer Mannheim Corp., Indianapolis, IN), and 2.5 U Taq polymerase (Boehringer Mannheim Corp., Indianapolis, IN). Thermocycler conditions in a Perkin Elmer 9600 (Norwalk, CT) were as follows: 95 °C for 5 mins, then 25 cycles of 94 °C for 30 secs, 55 °C for 2 mins, and 72 °C for 2 mins. PCR was followed by an additional cycle of 72 °C for 7 minutes.

The PCR amplified product was purified from an agarose gel, cut with *Eco*RI and *Sal*I*,* ligated to pYX242 (Invitrogen Corp., Carlsbad, CA) (linearized with *Eco*RI and *Sal*I*)* using the Rapid Ligation kit (Boehringer Mannheim Corp., Indianapolis, IN), according to the manufacturer's protocol and transformed into E. coli Top10 cells (Invitrogen Corp., Carlsbad, CA). The new plasmids, designated pRET-21 and pRET-22 (two individual clones from the ligation), were sequenced with the 373A Stretch DNA sequencer ABI (Perkin Elmer, Foster City, CA), and the cDNA sequences were identical. The 867 base cDNA nucleotide sequence of the plasmid pRET-22 containing the putative elongase is shown in Figure 46 and the translated sequence of 288 amino acids is shown in Figure 47. (Plasmid pRET-22 was deposited with the American Type Culture Collection, 10801 University Boulevard, Manassas, VA 20110-2209 on August 19, 1999, under the terms of the Budapest Treaty and was accorded deposit number ___)

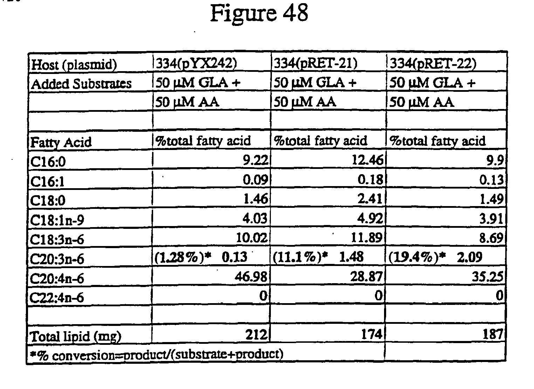

The plasmids pRET-21 and -22 were transformed into S. cerevisiae 334 as previously described (see Example III) and the resulting yeast cultures (334(pRET-21) and 334(pRET-22)) grown in 100 ml of selective media without leucine (Ausubel et al, supra) for 48 hours at 20°C in the presence of 50 µM GLA and AA. The cell pellets were collected and subjected to fatty acid analysis and the results shown in Figure 48. DGLA, the predicted product from GLA elongation, was found to be an average of 1.79% of the total lipid in the two samples, versus 0.13% for the negative control (334 containing plasmid pYX242) indicating that the enzyme encoded by both pRET-21 and pRET-22 possessed GLA elongase activity. The percent conversion of GLA to DGLA by 334(pRET-21) and 334(pRET-22) was 11.1% and 19.4% respectively with an average of 15.25%. Interestingly, almost no elongation of AA or any endogenous fatty acid was observed (Fig. 48). These results indicate that the elongase encoded by this newly identified C. elegans cDNA, CEELO1, is able to specifically elongate GLA to DGLA, suggesting that it may be a C. elegans homologue of GLA elongase.

### Example VIII

### Isolation of a Putative Human Elongase cDNA Based on AC004050 Sequence

To isolate the full length putative elongase cDNA based on the AC004050 sequence, primers RP735 (5' -CCT CCT GAA TTC CAQA CAC TAT TCA GCT TTC -3') and R073 (5' -TAA TAC GAC TCA CTA TAG GG -3') were used to PCR amplify the human liver Marathon-Ready cDNA (Clontech Laboratories, Inc., Palo Alto, CA). The PCR was carried out using the Advantage^{™} cDNA PCR Kit (Clontech Laboratories, Inc., Palo Alto, CA) with 5 µl of human liver Marathon-Ready cDNA and 50 pmole each primer following manufacturer's instructions. Thermocycler conditions in Perkin Elmer 9600 (Norwalk, CT) were as follows: 94 °C for 2 mins, then 30 cycles of 94 °C for 1 min., 58 °C for 2 mins., and 72 °C for 3 mins. PCR was followed by an additional extension at 72 °C for 7 mins.

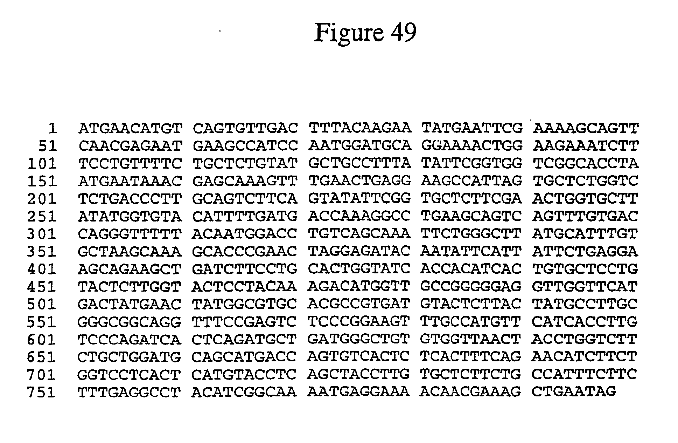

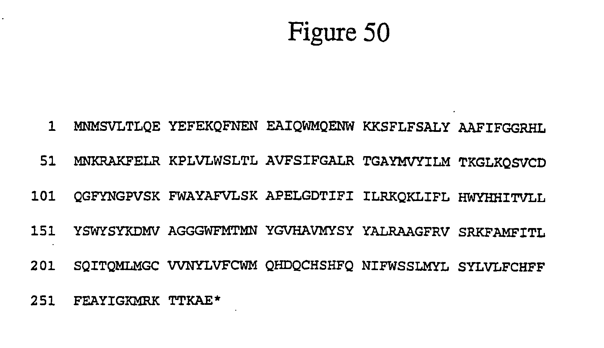

The PCR amplified product was run on a gel, an amplified fragment of approximately 1 Kb was gel purified, the termini of the fragment were filled in with T4DNA polymerase (Boehringer Mannheim, Corp., Carlsbad, CA) following manufacturer's instructions. The new plasmid was designated as pRAE-59, and the putative PUFA elongase cDNA in this plasmid, designated as HS3, was sequenced using the ABI 373A Stretch Sequencer (Perkin Elmer, Foster City, CA). The putative PUFA elongase cDNA sequence HS3 is shown in Figure 49, and the translated sequence is shown in Figure 50.

### Nutritional Compositions

The PUFAs described in the Detailed Description may be utilized in various nutritional supplements, infant formulations, nutritional substitutes and other nutritional solutions.

### I. INFANT FORMULATIONS

### A. Isomil^{®} Soy Formula with Iron:

Usage: As a beverage for infants, children and adults with an allergy or sensitivity to cows milk. A feeding for patients with disorders for which lactose should be avoided: lactase deficiency, lactose intolerance and galactosemia.

### Features:

- Soy protein isolate to avoid symptoms of cow's-milk-protein allergy or sensitivity.
- Lactose-free formulation to avoid lactose-associated diarrhea.
- Low osmolality (240 mOs/kg water) to reduce risk of osmotic diarrhea.
- Dual carbohydrates (corn syrup and sucrose) designed to enhance carbohydrate absorption and reduce the risk of exceeding the absorptive capacity of the damaged gut.
- 1.8 mg of Iron (as ferrous sulfate) per 100 Calories to help prevent iron deficiency.
- Recommended levels of vitamins and minerals.
- Vegetable oils to provide recommended levels of essential fatty acids.
- Milk-white color, milk-like consistency and pleasant aroma.

Ingredients: (Pareve) 85% water, 4.9% corn syrup, 2.6% sugar (sucrose), 2.1 % soy oil, 1.9% soy protein isolate, 1.4% coconut oil, 0.15% calcium citrate, 0. 11 % calcium phosphate tribasic, potassium citrate, potassium phosphate monobasic, potassium chloride, mono- and disglycerides, soy lecithin, carrageenan, ascorbic acid, L-methionine, magnesium chloride, potassium phosphate dibasic, sodium chloride, choline chloride, taurine, ferrous sulfate, m-inositol, alpha-tocopheryl acetate, zinc sulfate, L-carnitine, niacinamide, calcium pantothenate, cupric sulfate, vitamin A palmitate, thiamine chloride hydrochloride, riboflavin, pyridoxine hydrochloride, folic acid, manganese sulfate, potassium iodide, phylloquinone, biotin, sodium selenite, vitamin D3 and cyanocobalamin.

### B. Isomil^{®} DF Soy Formula For Diarrhea:

Usage: As a short-term feeding for the dietary management of diarrhea in infants and toddlers.

### Features:

- First infant formula to contain added dietary fiber from soy fiber specifically for diarrhea management.
- Clinically shown to reduce the duration of loose, watery stools during mild to severe diarrhea in infants.
- Nutritionally complete to meet the nutritional needs of the infant.
- Soy protein isolate with added L-methionine meets or exceeds an infant's requirement for all essential amino acids.
- Lactose-free formulation to avoid lactose-associated diarrhea.
- Low osmolality (240 mOsm/kg water) to reduce the risk of osmotic diarrhea.
- Dual carbohydrates (corn syrup and sucrose) designed to enhance carbohydrate absorption and reduce the risk of exceeding the absorptive capacity of the damaged gut.
- Meets or exceeds the vitamin and mineral levels recommended by the Committee on Nutrition of the American Academy of Pediatrics and required by the Infant Formula Act.
- 1.8 mg of iron (as ferrous sulfate) per 100 Calories to help prevent iron deficiency.
- Vegetable oils to provide recommended levels of essential fatty acids.

Ingredients: (Pareve) 86% water, 4.8% com syrup, 2.5% sugar (sucrose), 2.1% soy oil, 2.0% soy protein isolate, 1.4% coconut oil, 0.77% soy fiber, 0.12% calcium citrate, 0.11 % calcium phosphate tribasic, 0.10% potassium citrate, potassium chloride, potassium phosphate monobasic, mono and diglycerides, soy lecithin, carrageenan, magnesium chloride, ascorbic acid, L-methionine, potassium phosphate dibasic, sodium chloride, choline chloride, taurine, ferrous sulfate, m-inositol, alpha-tocopheryl acetate, zinc sulfate, L-carnitine, niacinamide, calcium pantothenate, cupric sulfate, vitamin A palmitate, thiamine chloride hydrochloride, riboflavin, pyridoxine hydrochloride, folic acid, manganese sulfate, potassium iodide, phylloquinone, biotin, sodium selenite, vitamin D3 and cyanocobalamin.

### C. Isomil^{®} SF Sucrose-Free Soy Formula With Iron:

Usage: As a beverage for infants, children and adults with an allergy or sensitivity to cow's-milk protein or an intolerance to sucrose. A feeding for patients with disorders for which lactose and sucrose should be avoided.

### Features:

- Soy protein isolate to avoid symptoms of cow's-milk-protein allergy or sensitivity.
- Lactose-free formulation to avoid lactose-associated diarrhea (carbohydrate source is Polycose^{®} Glucose Polymers).
- Sucrose free for the patient who cannot tolerate sucrose.
- Low osmolality (180 mOsm/kg water) to reduce risk of osmotic diarrhea.
- 1.8 mg of iron (as ferrous sulfate) per 100 Calories to help prevent iron deficiency.
- Recommended levels of vitamins and minerals.
- Vegetable oils to provide recommended levels of essential fatty acids.
- Milk-white color, milk-like consistency and pleasant aroma.

Ingredients: (Pareve) 75% water, 11.8% hydrolized cornstarch, 4.1% soy oil, 4.1 % soy protein isolate, 2.8% coconut oil, 1.0% modified cornstarch, 0.38% calcium phosphate tribasic, 0. 17% potassium citrate, 0.13% potassium chloride, mono- and diglycerides, soy lecithin, magnesium chloride, abscorbic acid, L-methionine, calcium carbonate, sodium chloride, choline chloride, carrageenan, taurine, ferrous sulfate, m-inositol, alpha-tocopheryl acetate, zinc sulfate,L-carnitine, niacinamide, calcium pantothenate, cupric sulfate, vitamin A palmitate, thiamine chloride hydrochloride, riboflavin, pyridoxine hydrochloride, folic acid, manganese sulfate, potassium iodide, phylloquinone, biotin, sodium selenite, vitamin D3 and cyanocobalamin.

### D. Isomil^{®} 20 Soy Formula With Iron Ready To Feed, 20 Cal/fl oz.:

Usage: When a soy feeding is desired.

Ingredients: (Pareve) 85% water, 4.9% corn syrup, 2.6% sugar(sucrose), 2.1 % soy oil, 1.9% soy protein isolate, 1.4% coconut oil, 0.15% calcium citrate, 0. 11% calcium phosphate tribasic, potassium citrate, potassium phosphate monobasic, potassium chloride, mono- and diglycerides, soy lecithin, carrageenan, abscorbic acid, L-methionine, magnesium chloride, potassium phosphate dibasic, sodium chloride, choline chloride, taurine, ferrous sulfate, m-inositol, alpha-tocopheryl acetate, zinc sulfate, L-carnitine, niacinamide, calcium pantothenate, cupric sulfate, vitamin A palmitate, thiamine chloride hydrochloride, riboflavin, pyridoxine hydrochloride, folic acid, manganese sulfate, potassium iodide, phylloquinone, biotin, sodium selenite, vitamin D3 and cyanocobalamin.

### E. Similac^{®} Infant Formula:

Usage: When an infant formula is needed: if the decision is made to discontinue breastfeeding before age 1 year, if a supplement to breastfeeding is needed or as a routine feeding if breastfeeding is not adopted.

### Features:

- Protein of appropriate quality and quantity for good growth; heat-denatured, which reduces the risk of milk-associated enteric blood loss.
- Fat from a blend of vegetable oils (doubly homogenized), providing essential linoleic acid that is easily absorbed.
- Carbohydrate as lactose in proportion similar to that of human milk.
- Low renal solute load to minimize stress on developing organs.
- Powder, Concentrated Liquid and Ready To Feed forms.

Ingredients: (-D) Water, nonfat milk, lactose, soy oil, coconut oil, mono- and diglycerides, soy lecithin, abscorbic acid, carrageenan, choline chloride, taurine, m-inositol, alpha-tocopheryl acetate, zinc sulfate, niacinamide, ferrous sulfate, calcium pantothenate, cupric sulfate, vitamin A palmitate, thiamine chloride hydrochloride, riboflavin, pyridoxine hydrochloride, folic acid, manganese sulfate, phylloquinone, biotin, sodium selenite, vitamin D3 and cyanocobalamin.

### F. Similac^{®} NeoCare Premature Infant Formula With Iron:

Usage: For premature infants' special nutritional needs after hospital discharge. Similac NeoCare is a nutritionally complete formula developed to provide premature infants with extra calories, protein, vitamins and minerals needed to promote catch-up growth and support development.

### Features:

- Reduces the need for caloric and vitamin supplementation. More calories (22 Cal/fl oz) than standard term formulas (20 Cal/fl oz).
- Highly absorbed fat blend, with medium-chain triglycerides (MCT oil) to help meet the special digestive needs of premature infants.
- Higher levels of protein, vitamins and minerals per 100 calories to extend the nutritional support initiated in-hospital.
- More calcium and phosphorus for improved bone mineralization.

Ingredients: -D Corn syrup solids, nonfat milk, lactose, whey protein concentrate, soy oil, high-oleic safflower oil, fractionated coconut oil (medium chain triglycerides), coconut oil, potassium citrate, calcium phosphate tribasic, calcium carbonate, ascorbic acid, magnesium chloride, potassium chloride, sodium chloride, taurine, ferrous sulfate, m-inositol, choline chloride, ascorbyl palmitate, L-carnitine, alpha-tocopheryl acetate, zinc sulfate, niacinamide, mixed tocopherols, sodium citrate, calcium pantothenate, cupric sulfate, thiamine chloride hydrochloride, vitamin A palmitate, beta carotene, riboflavin, pyridoxine hydrochloride, folic acid, manganese sulfate, phylloquinone, biotin, sodium selenite, vitamin D3 and cyanocobalamin.

### G. Similac Natural Care Low-Iron Human Milk Fortifier Ready To Use, 24 Cal/fl oz.:

Usage: Designed to be mixed with human milk or to be fed alternatively with human milk to low-birth-weight infants.

Ingredients: -D Water, nonfat milk, hydrolyzed cornstarch, lactose, fractionated coconut oil (medium-chain triglycerides), whey protein concentrate, soy oil, coconut oil, calcium phosphate tribasic, potassium citrate, magnesium chloride, sodium citrate, ascorbic acid, calcium carbonate, mono and diglycerides, soy lecithin, carrageenan, choline chloride, m-inositol, taurine, niacinamide, L-carnitine, alpha tocopheryl acetate, zinc sulfate, potassium chloride, calcium pantothenate, ferrous sulfate, cupric sulfate, riboflavin, vitamin A palmitate, thiamine chloride hydrochloride, pyridoxine hydrochloride, biotin, folic acid, manganese sulfate, phylloquinone, vitamin D3, sodium selenite and cyanocobalamin.

Various PUFAs of this invention can be substituted and/or added to the infant formulae described above and to other infant formulae known to those in the art.

### II. NUTRITIONAL FORMULATIONS

### A. ENSURE^{®}

Usage: ENSURE is a low-residue liquid food designed primarily as an oral nutritional supplement to be used with or between meals or, in appropriate amounts, as a meal replacement. ENSURE is lactose- and gluten-free, and is suitable for use in modified diets, including low-cholesterol diets. Although it is primarily an oral supplement, it can be fed by tube.

### Patient Conditions:

- For patients on modified diets
- For elderly patients at nutrition risk
- For patients with involuntary weight loss
- For patients recovering from illness or surgery
- For patients who need a low-residue diet

Ingredients: -D Water, Sugar (Sucrose), Maltodextrin (Corn), Calcium and Sodium Caseinates, High-Oleic Safflower Oil, Soy Protein Isolate, Soy Oil, Canola Oil, Potassium Citrate, Calcium Phosphate Tribasic, Sodium Citrate, Magnesium Chloride, Magnesium Phosphate Dibasic, Artificial Flavor, Sodium Chloride, Soy Lecithin, Choline Chloride, Ascorbic Acid, Carrageenan, Zinc Sulfate, Ferrous Sulfate, Alpha-Tocopheryl Acetate, Gellan Gum, Niacinamide, Calcium Pantothenate, Manganese Sulfate, Cupric Sulfate, Vitamin A Palmitate, Thiamine Chloride Hydrochloride, Pyridoxine Hydrochloride, Riboflavin, Folic Acid, Sodium Molybdate, Chromium Chloride, Biotin, Potassium Iodide, Sodium Selenate.

### B. ENSURE^{®} BARS:

Usage: ENSURE BARS are complete, balanced nutrition for supplemental use between or with meals. They provide a delicious, nutrient-rich alternative to other snacks. ENSURE BARS contain <1 g lactose/bar, and Chocolate Fudge Brownie flavor is gluten-free. (Honey Graham Crunch flavor contains gluten.)

### Patient Conditions:

- For patients who need extra calories, protein, vitamins and minerals.
- Especially useful for people who do not take in enough calories and nutrients.
- For people who have the ability to chew and swallow
- Not to be used by anyone with a peanut allergy or any type of allergy to nuts.

Ingredients: Honey Graham Crunch -- High-Fructose Corn Syrup, Soy Protein Isolate, Brown Sugar, Honey, Maltodextrin (Corn), Crisp Rice (Milled Rice, Sugar [Sucrose], Salt [Sodium Chloride] and Malt), Oat Bran, Partially Hydrogenated Cottonseed and Soy Oils, Soy Polysaccharide, Glycerine, Whey Protein Concentrate, Polydextrose, Fructose, Calcium Caseinate, Cocoa Powder, Artificial Flavors, Canola Oil, High-Oleic Safflower Oil, Nonfat Dry Milk, Whey Powder, Soy Lecithin and Corn Oil. Manufactured in a facility that processes nuts.

Vitamins and Minerals: Calcium Phosphate Tribasic, Potassium Phosphate Dibasic, Magnesium Oxide, Salt (Sodium Chloride), Potassium Chloride, Ascorbic Acid, Ferric Orthophosphate, Alpha-Tocopheryl Acetate, Niacinamide, Zinc Oxide, Calcium Pantothenate, Copper Gluconate, Manganese Sulfate, Riboflavin, Beta Carotene, Pyridoxine Hydrochloride, Thiamine Mononitrate, Folic Acid, Biotin, Chromium Chloride, Potassium Iodide, Sodium Selenate, Sodium Molybdate, Phylloquinone, Vitamin D3 and Cyanocobalamin.

Protein: Honey Graham Crunch - The protein source is a blend of soy protein isolate and milk proteins.

| | |
|---|---|
| Soy protein isolate | 74% |
| Milk proteins | 26% |

Fat: Honey Graham Crunch - The fat source is a blend of partially hydrogenated cottonseed and soybean, canola, high oleic safflower, oils, and soy lecithin.

Partially hydrogenated cottonseed and soybean oil 76%

| | |
|---|---|
| Canola oil | 8% |
| High-oleic safflower oil | 8% |
| Corn oil | 4% |
| Soy lecithin | 4% |

Carbohydrate: Honey Graham Crunch - The carbohydrate source is a combination of high-fructose corn syrup, brown sugar, maltodextrin, honey, crisp rice, glycerine, soy polysaccharide, and oat bran.

| | |
|---|---|
| High-fructose corn syrup | 24% |
| Brown sugar | 21% |
| Maltodextrin | 12% |
| Honey | 11% |
| Crisp rice | 9% |
| Glycerine | 9% |
| Soy Polysaccharide | 7% |
| Oat bran | 7% |

### C. ENSURE^{®} HIGH PROTEIN:

Usage: ENSURE HIGH PROTEIN is a concentrated, high-protein liquid food designed for people who require additional calories, protein, vitamins, and minerals in their diets. It can be used as an oral nutritional supplement with or between meals or, in appropriate amounts, as a meal replacement. ENSURE HIGH PROTEIN is lactose- and gluten-free, and is suitable for use by people recovering from general surgery or hip fractures and by patients at risk for pressure ulcers.

### Patient Conditions:

- For patients who require additional calories, protein, vitamins, and minerals, such as patients recovering from general surgery or hip fractures, patients at risk for pressure ulcers, and patients on low-cholesterol diets

### Features:

- Low in saturated fat
- Contains 6 g of total fat and < 5 mg of cholesterol per serving
- Rich, creamy taste
- Excellent source of protein, calcium, and other essential vitamins and minerals
- For low-cholesterol diets
- Lactose-free, easily digested

### Ingredients:

Vanilla Supreme: -D Water, Sugar (Sucrose), Maltodextrin (Corn), Calcium and Sodium Caseinates, High-OIeic Safflower Oil, Soy Protein Isolate, Soy Oil, Canola Oil, Potassium Citrate, Calcium Phosphate Tribasic, Sodium Citrate, Magnesium Chloride, Magnesium Phosphate Dibasic, Artificial Flavor, Sodium Chloride, Soy Lecithin, Choline Chloride, Ascorbic Acid, Carrageenan, Zinc Sulfate, Ferrous Suffate, Alpha-Tocopheryl Acetate, Gellan Gum, Niacinamide, Calcium Pantothenate, Manganese Sulfate, Cupric Sulfate, Vitamin A Palmitate, Thiamine Chloride Hydrochloride, Pyridoxine Hydrochloride, Riboflavin, Folic Acid, Sodium Molybdate, Chromium Chloride, Biotin, Potassium Iodide, Sodium Selenate, Phylloquinone, Vitamin D3 and Cyanocobalamin.

### Protein:

The protein source is a blend of two high-biologic-value proteins: casein and soy.

| | |
|---|---|
| Sodium and calcium caseinates | 85% |
| Soy protein isolate | 15% |

### Fat:

The fat source is a blend of three oils: high-oleic safflower, canola, and soy.

| | |
|---|---|
| High-oleic safflower oil | 40% |
| Canola oil | 30% |
| Soy oil | 30% |

The level of fat in ENSURE HIGH PROTEIN meets American Heart Association (AHA) guidelines. The 6 grams of fat in ENSURE HIGH PROTEIN represent 24% of the total calories, with 2.6% of the fat being from saturated fatty acids and 7.9% from polyunsaturated fatty acids. These values are within the AHA guidelines of < 30% of total calories from fat, < 10% of the calories from saturated fatty acids, and < 10% of total calories from polyunsaturated fatty acids.

### Carbohydrate:

ENSURE HIGH PROTEIN contains a combination of maltodextrin and sucrose. The mild sweetness and flavor variety (vanilla supreme, chocolate royal, wild berry, and banana), plus VARI-FLAVORS^{®} Flavor Pacs in pecan, cherry, strawberry, lemon, and orange, help to prevent flavor fatigue and aid in patient compliance.

### Vanilla and other nonchocolate flavors:

| | |
|---|---|
| Sucrose | 60% |
| Maltodextrin | 40% |

### Chocolate:

| | |
|---|---|
| Sucrose | 70% |
| Maltodextrin | 30% |

### D. ENSURE^{®} LIGHT

Usage: ENSURE LIGHT is a low-fat liquid food designed for use as an oral nutritional supplement with or between meals. ENSURE LIGHT is lactose- and gluten-free, and is suitable for use in modified diets, including low-cholesterol diets.

### Patient Conditions:

- For normal-weight or overweight patients who need extra nutrition in a supplement that contains 50% less fat and 20% fewer calories than ENSURE.
- For healthy adults who don't eat right and need extra nutrition.

### Features:

- Low in fat and saturated fat
- Contains 3 g of total fat per serving and < 5 mg cholesterol
- Rich, creamy taste
- Excellent source of calcium and other essential vitamins and minerals
- For low-cholesterol diets
- Lactose-free, easily digested

### Ingredients:

French Vanilla: -D Water, Maltodextrin (Corn), Sugar (Sucrose), Calcium Caseinate, High-Oleic Safflower Oil, Canola Oil, Magnesium Chloride, Sodium Citrate, Potassium Citrate, Potassium Phosphate Dibasic, Magnesium Phosphate Dibasic, Natural and Artificial Flavor, Calcium Phosphate Tribasic, Cellulose Gel, Choline Chloride, Soy Lecithin, Carrageenan, Salt (Sodium Chloride), Ascorbic Acid, Cellulose Gum, Ferrous Sulfate, Alpha-Tocopheryl Acetate, Zinc Sulfate, Niacinamide, Manganese Sulfate, Calcium Pantothenate, Cupric Sulfate, Thiamine Chloride Hydrochloride, Vitamin A Palmitate, Pyridoxine Hydrochloride, Riboflavin, Chromium Chloride, Folic Acid, Sodium Molybdate, Biotin, Potassium Iodide, Sodium Selenate, Phylloquinone, Vitamin D3 and Cyanocobalamin.

### Protein:

The protein source is calcium caseinate.

| | |
|---|---|
| Calcium caseinate | 100% |

### Fat:

The fat source is a blend of two oils: high-oleic safflower and canola.

| | |
|---|---|
| High-oleic safflower oil | 70% |
| Canola oil | 30% |

The level of fat in ENSURE LIGHT meets American Heart Association (AHA) guidelines. The 3 grams of fat in ENSURE LIGHT represent 13.5% of the total calories, with 1.4% of the fat being from saturated fatty acids and 2.6% from polyunsaturated fatty acids. These values are within the AHA guidelines of < 30% of total calories from fat, < 10% of the, calories from saturated fatty acids, and < 10% of total calories from polyunsaturated fatty acids.

### Carbohydrate:

ENSURE LIGHT contains a combination of maltodextrin and sucrose. The chocolate flavor contains corn syrup as well. The mild sweetness and flavor variety (French vanilla, chocolate supreme, strawberry swirl), plus VARI-FLAVORS^{®} Flavor Pacs in pecan, cherry, strawberry, lemon, and orange, help to prevent flavor fatigue and aid in patient compliance.

### Vanilla and other nonchocolate flavors:

| | |
|---|---|
| Sucrose | 51% |
| Maltodextrin | 49% |

### Chocolate:

| | |
|---|---|
| Sucrose | 47.0% |
| Corn Syrup | 26.5% |
| Maltodextrin | 26.5% |

### Vitamins and Minerals:

An 8-fl-oz serving of ENSURE LIGHT provides at least 25% of the RDIs for 24 key vitamins and minerals.

### Caffeine:

Chocolate flavor contains 2.1 mg caffeine/8 fl oz.

### E. ENSURE PLUS^{®}

Usage: ENSURE PLUS is a high-calorie, low-residue liquid food for use when extra calories and nutrients, but a normal concentration of protein, are needed. It is designed primarily as an oral nutritional supplement to be used with or between meals or, in appropriate amounts, as a meal replacement. ENSURE PLUS is lactose- and gluten-free. Although it is primarily an oral nutritional supplement, it can be fed by tube.

Patient Conditions:
- For patients who require extra calories and nutrients, but a normal concentration of protein, in a limited volume
- For patients who need to gain or maintain healthy weight

### Features:

- Rich, creamy taste
- Good source of essential vitamins and minerals

### Ingredients:

Vanilla: -D Water, Corn Syrup, Maltodextrin (Corn), Corn Oil, Sodium and Calcium Caseinates, Sugar (Sucrose), Soy Protein Isolate, Magnesium Chloride, Potassium Citrate, Calcium Phosphate Tribasic, Soy Lecithin, Natural and Artificial Flavor, Sodium Citrate, Potassium Chloride, Choline Chloride, Ascorbic Acid, Carrageenan, Zinc Sulfate, Ferrous Sulfate, Alpha-Tocopheryl Acetate, Niacinamide, Calcium Pantothenate, Manganese Sulfate, Cupric Sulfate, Thiamine Chloride Hydrochloride, Pyridoxine Hydrochloride, Riboflavin, Vitamin A Palmitate, Folic Acid, Biotin, Chromium Chloride, Sodium Molybdate, Potassium Iodide, Sodium Selenite, Phylloquinone, Cyanocobalamin and Vitamin D3.

### Protein:

The protein source is a blend of two high-biologic-value proteins: casein and soy.

| | |
|---|---|
| Sodium and calcium caseinates | 84% |
| Soy protein isolate | 16% |

### Fat:

The fat source is corn oil.

| | |
|---|---|
| Corn oil | 100% |

### Carbohydrate:

ENSURE PLUS contains a combination of maltodextrin and sucrose. The mild sweetness and flavor variety (vanilla, chocolate, strawberry, coffee, buffer pecan, and eggnog), plus VARI-FLAVORS^{®} Flavor Pacs in pecan, cherry, strawberry, lemon, and orange, help to prevent flavor fatigue and aid in patient compliance.

### Vanilla, strawberry, butter pecan, and coffee flavors:

| | |
|---|---|
| Corn Syrup | 39% |
| Maltodextrin | 38% |
| Sucrose | 23% |

### Chocolate and eggnog flavors:

| | |
|---|---|
| Corn Syrup | 36% |
| Maltodextrin | 34% |
| Sucrose | 30% |

### Vitamins and Minerals:

An 8-fl-oz serving of ENSURE PLUS provides at least 15% of the RDIs for 25 key Vitamins and minerals.

### Caffeine:

Chocolate flavor contains 3.1 mg Caffeine/8 fl oz. Coffee flavor contains a trace amount of caffeine.

### F. ENSURE PLUS^{®} HN

Usage: ENSURE PLUS HN is a nutritionally complete high-calorie, high-nitrogen liquid food designed for people with higher calorie and protein needs or limited volume tolerance. It may be used for oral supplementation or for total nutritional support by tube. ENSURE PLUS HN is lactose- and gluten-free.

Patient Conditions:
- For patients with increased calorie and protein needs, such as following surgery or injury.
- For patients with limited volume tolerance and early satiety.

### Features:

- For supplemental or total nutrition
- For oral or tube feeding
- 1.5 CaVmL,
- High nitrogen
- Calorically dense

### Ingredients:

Vanilla: -D Water, Maltodextrin (Corn), Sodium and Calcium Caseinates, Corn Oil, Sugar (Sucrose), Soy Protein Isolate, Magnesium Chloride, Potassium Citrate, Calcium Phosphate Tribasic, Soy Lecithin, Natural and Artificial Flavor, Sodium Citrate, Choline Chloride, Ascorbic Acid, Taurine, L-Carnitine, Zinc Sulfate, Ferrous Sulfate, Alpha-Tocopheryl Acetate, Niacinamide, Carrageenan, Calcium Pantothenate, Manganese Sulfate, Cupric Sulfate, Thiamine Chloride Hydrochloride, Pyridoxine Hydrochloride, Riboflavin, Vitamin A Palmitate, Folic Acid, Biotin, Chromium Chloride, Sodium Molybdate, Potassium Iodide, Sodium Selenite, Phylloquinone, Cyanocobalamin and Vitamin D3.

### G. ENSURE^{®} POWDER:

Usage: ENSURE POWDER (reconstituted with water) is a low-residue liquid food designed primarily as an oral nutritional supplement to be used with or between meals. ENSURE POWDER is lactose- and gluten-free, and is suitable for use in modified diets, including low-cholesterol diets.

### Patient Conditions:

- For patients on modified diets
- For elderly patients at nutrition risk
- For patients recovering from illness/surgery
- For patients who need a low-residue diet

### Features:

- Convenient, easy to mix
- Low in saturated fat
- Contains 9 g of total fat and < 5 mg of cholesterol per serving
- High in vitamins and minerals
- For low-cholesterol diets
- Lactose-free, easily digested

Ingredients: -D Corn Syrup, Maltodextrin (Corn), Sugar (Sucrose), Corn Oil, Sodium and Calcium Caseinates, Soy Protein Isolate, Artificial Flavor, Potassium Citrate, Magnesium Chloride, Sodium Citrate, Calcium Phosphate Tribasic, Potassium Chloride, Soy Lecithin, Ascorbic Acid, Choline Chloride, Zinc Sulfate, Ferrous Sulfate, Alpha-Tocopheryl Acetate, Niacinamide, Calcium Pantothenate, Manganese Sulfate, Thiamine Chloride Hydrochloride, Cupric Sulfate, Pyridoxine Hydrochloride, Riboflavin, Vitamin A Palmitate, Folic Acid, Biotin, Sodium Molybdate, Chromium Chloride, Potassium Iodide, Sodium Selenate, Phylloquinone, Vitamin D3 and Cyanocobalamin.

### Protein:

The protein source is a blend of two high-biologic-value proteins: casein and soy.

| | |
|---|---|
| Sodium and calcium caseinates | 84% |
| Soy protein isolate | 16% |

### Fat:

The fat source is corn oil.

| | |
|---|---|
| Corn oil | 100% |

### Carbohydrate:

ENSURE POWDER contains a combination of corn syrup, maltodextrin, and sucrose. The mild sweetness of ENSURE POWDER, plus VARI-FLAVORS^{®} Flavor Pacs in pecan, cherry, strawberry, lemon, and orange, helps to prevent flavor fatigue and aid in patient compliance.

### Vanilla:

| | |
|---|---|
| Corn Syrup | 35% |
| Maltodextrin | 35% |
| Sucrose | 30% |

### H. ENSURE^{®} PUDDING

Usage: ENSURE PUDDING is a nutrient-dense supplement providing balanced nutrition in a nonliquid form to be used with or between meals. It is appropriate for consistency-modified diets (e.g., soft, pureed, or full liquid) or for people with swallowing impairments. ENSURE PUDDING is gluten-free.

### Patient Conditions:

- For patients on consistency-modified diets (e.g., soft, pureed, or full liquid)
- For patients with swallowing impairments

### Features:

- Rich and creamy, good taste
- Good source of essential vitamins and minerals
- Convenient-needs no refrigeration
- Gluten-free

Nutrient Profile per 5 oz: Calories 250, Protein 10.9%, Total Fat 34.9%, Carbohydrate 54.2%

### Ingredients:

Vanilla: -D Nonfat Milk, Water, Sugar (Sucrose), Partially Hydrogenated Soybean Oil, Modified Food Starch, Magnesium Sulfate, Sodium Stearoyl Lactylate, Sodium Phosphate Dibasic, Artificial Flavor, Ascorbic Acid, Zinc Sulfate, Ferrous Sulfate, Alpha-Tocopheryl Acetate, Choline Chloride, Niacinamide, Manganese Sulfate, Calcium Pantothenate, FD&C Yellow #5, Potassium Citrate, Cupric Sulfate, Vitamin A Palmitate, Thiamine Chloride Hydrochloride, Pyridoxine Hydrochloride, Riboflavin, FD&C Yellow #6, Folic Acid, Biotin, Phylloquinone, Vitamin D3 and Cyanocobalamin.

### Protein:

The protein source is nonfat milk.

| | |
|---|---|
| Nonfat milk | 100% |

### Fat:

The fat source is hydrogenated soybean oil.

| | |
|---|---|
| Hydrogenated soybean oil | 100% |

### Carbohydrate:

ENSURE PUDDING contains a combination of sucrose and modified food starch. The mild sweetness and flavor variety (vanilla, chocolate, butterscotch, and tapioca) help prevent flavor fatigue. The product contains 9.2 grams of lactose per serving.

### Vanilla and other nonchocolate flavors:

| | |
|---|---|
| Sucrose | 56% |
| Lactose | 27% |
| Modified food starch | 17% |

### Chocolate:

| | |
|---|---|
| Sucrose | 58% |
| Lactose | 26% |
| Modified food starch | 16% |

### I. ENSURE^{®} WITH FIBER:

Usage: ENSURE WITH FIBER is a fiber-containing, nutritionally complete liquid food designed for people who can benefit from increased dietary fiber and nutrients. ENSURE WITH FIBER is suitable for people who do not require a low-residue diet. It can be fed orally or by tube, and can be used as a nutritional supplement to a regular diet or, in appropriate amounts, as a meal replacement. ENSURE WITH FIBER is lactose- and gluten-free, and is suitable for use in modified diets, including low-cholesterol diets.

### Patient Conditions:

- For patients who can benefit from increased dietary fiber and nutrients

### Features:

- New advanced formula-low in saturated fat, higher in vitamins and minerals
- Contains 6 g of total fat and < 5 mg of cholesterol per serving
- Rich, creamy taste
- Good source of fiber
- Excellent source of essential vitamins and minerals
- For low-cholesterol diets
- Lactose- and gluten-free

### Ingredients:

Vanilla: -D Water; Maltodextrin (Corn), Sugar (Sucrose), Sodium and Calcium Caseinates, Oat Fiber, High-Oleic Safflower Oil, Canola Oil, Soy Protein Isolate, Corn Oil, Soy Fiber, Calcium Phosphate Tribasic, Magnesium Chloride, Potassium Citrate, Cellulose Gel, Soy Lecithin, Potassium Phosphate Dibasic, Sodium Citrate, Natural and Artificial Flavors, Choline Chloride, Magnesium Phosphate, Ascorbic Acid, Cellulose Gum, Potassium Chloride, Carrageenan, Ferrous Sulfate, Alpha-Tocopheryl Acetate, Zinc Sulfate, Niacinamide, Manganese Sulfate, Calcium Pantothenate, Cupric Sulfate, Vitamin A Palmitate, Thiamine Chloride Hydrochloride, Pyridoxine Hydrochloride, Riboflavin, Folic Acid, Chromium Chloride, Biotin, Sodium Molybdate, Potassium Iodide, Sodium Selenate, Phylloquinone, Vitamin

### D3 and Cyanocobalamin.

### Protein:

The protein source is a blend of two high-biologic-value proteins-casein and soy.

| | |
|---|---|
| Sodium and calcium caseinates | 80% |
| Soy protein isolate | 20% |

### Fat:

The fat source is a blend of three oils: high-oleic safflower, canola, and corn.

| | |
|---|---|
| High-oleic safflower oil | 40% |
| Canola oil | 40% |
| Corn oil | 20% |

The level of fat in ENSURE WITH FIBER meets American Heart Association (AHA) guidelines. The 6 grams of fat in ENSURE WITH FIBER represent 22% of the total calories, with 2.01 % of the fat being from saturated fatty acids and 6.7% from polyunsaturated fatty acids. These values are within the AHA guidelines of ≤ 30% of total calories from fat, < 10% of the calories from saturated fatty acids, and ≤ 10% of total calories from polyunsaturated fatty acids.

### Carbohydrate:

ENSURE WITH FIBER contains a combination of maltodextrin and sucrose. The mild sweetness and flavor variety (vanilla, chocolate, and butter pecan), plus VARI-FLAVORS^{®} Flavor Pacs in pecan, cherry, strawberry, lemon, and orange, help to prevent flavor fatigue and aid in patient compliance.

### Vanilla and other nonchocolate flavors:

| | |
|---|---|
| Maltodextrin | 66% |
| Sucrose | 25% |
| Oat Fiber | 7% |
| Soy Fiber | 2% |

### Chocolate:

| | |
|---|---|
| Maltodextrin | 55% |
| Sucrose | 36% |
| Oat Fiber | 7% |
| Soy Fiber | 2% |

### Fiber:

The fiber blend used in ENSURE WITH FIBER consists of oat fiber and soy polysaccharide. This blend results in approximately 4 grams of total dietary fiber per 8-fl. oz can. The ratio of insoluble to soluble fiber is 95:5.

The various nutritional supplements described above and known to others of skill in the art can be substituted and/or supplemented with the PUFAs produced in accordance with the present invention.

### J. Oxepa^{™} Nutritional Product

Oxepa is a low-carbohydrate, calorically dense, enteral nutritional product designed for the dietary management of patients with or at risk for ARDS. It has a unique combination of ingredients, including a patented oil blend containing eicosapentaenoic acid (EPA from fish oil), γ-linolenic acid (GLA from borage oil), and elevated antioxidant levels.

### Caloric Distribution:

Caloric density is high at 1.5 Cal/mL (355 Cal/8 fl oz), to minimize the volume required to meet energy needs. The distribution of Calories in Oxepa is shown in Table IV.

**Table IV. Caloric Distribution of Oxepa**

| | per 8 fl oz. | per liter | % of Cal |
|---|---|---|---|
| Calories | 355 | 1,500 | --- |
| Fat (g) | 22.2 | 93.7 | 55.2 |
| Carbohydrate (g) | 25 | 105.5 | 28.1 |
| Protein (g) | 14.8 | 62.5 | 16.7 |
| Water (g) | 186 | 785 | --- |

### Fat:

- Oxepa contains 22.2 g of fat per 8-fl oz serving (93.7 g/L).
- The fat source is an oil blend of 31.8% canola oil, 25% medium-chain triglycerides (MCTs), 20% borage oil, 20% fish oil, and 3.2 % soy lecithin. The typical fatty acid profile of Oxepa is shown in Table V.
- Oxepa provides a balanced amount of polyunsaturated, monounsaturated, and saturated fatty acids, as shown in Table VI.
- Medium-chain trigylcerides (MCTs) -- 25% of the fat blend -- aid gastric emptying because they are absorbed by the intestinal tract without emulsification by bile acids.

The various fatty acid components of Oxepa^{™} nutritional product can be substituted and/or supplemented with the PUFAs produced in accordance with this invention.

**Table V. Typical Fatty Acid Profile**

| | % Total Fatty Acids | g/8 fl oz* | 9/L* |
|---|---|---|---|
| Caproic (6:0) | 0.2 | 0.04 | 0.18 |
| Caprylic (8:0) | 14.69 | 3.1 | 13.07 |
| Capric (10:0) | 11.06 | 2.33 | 9.87 |
| Palmitic (16:0) | 5.59 | 1.18 | 4.98 |
| Palmitoleic | 1.82 | 0.38 | 1.62 |
| Stearic | 1.94 | 0.39 | 1.64 |
| Oleic | 24.44 | 5.16 | 21.75 |
| Linoleic | 16.28 | 3.44 | 14.49 |
| α-Linolenic | 3.47 | 0.73 | 3.09 |
| γ-Linolenic | 4.82 | 1.02 | 4.29 |
| Eicosapentaenoic | 5.11 | 1.08 | 4.55 |
| n-3-Docosapentaenoic | 0.55 | 0.12 | 0.49 |
| Docosahexaenoic | 2.27 | 0.48 | 2.02 |
| | | | |
| Others | 7.55 | 1.52 | 6.72 |

Fatty acids equal approximately 95% of total fat.

**Table VI. Fat Profile of Oxepa.**

| | |
|---|---|
| % of total calories from fat | 55.2 |
| Polyunsaturated fatty acids | 31.44 g/L |
| Monounsaturated fatty acids | 25.53 g/L |
| Saturated fatty acids | 32.38 g/L |
| n-6 to n-3 ratio | 1.75:1 |
| Cholesterol | 9.49 mg/8 fl oz |
| | 40.1 mg/L |

### Carbohydrate:

- The carbohydrate content is 25.0 g per 8-fl-oz serving (105.5 g/L).
- The carbohydrate sources are 45% maltodextrin (a complex carbohydrate) and 55% sucrose (a simple sugar), both of which are readily digested and absorbed.
- The high-fat and low-carbohydrate content of Oxepa is designed to minimize carbon dioxide (C02) production. High C02 levels can complicate weaning in ventilator-dependent patients. The low level of carbohydrate also may be useful for those patients who have developed stress-induced hyperglycemia.
- Oxepa is lactose-free.

Dietary carbohydrate, the amino acids from protein, and the glycerol moiety of fats can be converted to glucose within the body. Throughout this process, the carbohydrate requirements of glucose-dependent tissues (such as the central nervous system and red blood cells) are met. However, a diet free of carbohydrates can lead to ketosis, excessive catabolism of tissue protein, and loss of fluid and electrolytes. These effects can be prevented by daily ingestion of 50 to 100 g of digestible carbohydrate, if caloric intake is adequate. The carbohydrate level in Oxepa is also sufficient to minimize gluconeogenesis, if energy needs are being met.

### Protein:

- Oxepa contains 14.8 g of protein per 8-fl-oz serving (62.5 g/L).
- The total calorie/nitrogen ratio (150:1) meets the need of stressed patients.
- Oxepa provides enough protein to promote anabolism and the maintenance of lean body mass without precipitating respiratory problems. High protein intakes are a concern in patients with respiratory insufficiency. Although protein has little effect on C02 production, a high protein diet will increase ventilatory drive.
- The protein sources of Oxepa are 86.8% sodium caseinate and 13.2% calcium caseinate.
- The amino acid profile of the protein system in Oxepa meets or surpasses the standard for high quality protein set by the National Academy of Sciences.
* Oxepa is gluten-free.

### Default settings for the analysis programs

### GCG Programs

### FastA Search

### Default parameters:

| | | |
|---|---|---|
| range of interest | Begin=1 | END=last protein or nucleic acid |
| search set | all of SwissProt (protein) or acid) GenEMBL(nucleic | |
| word size | =(2) for protein | =(6) for nucleic acid |

Expected scores lists scores until E( ) value reaches 2.0

### TFastA search

### Default parameters:

| | | |
|---|---|---|
| range of interest | Begin=1 | END=last nucleic acid |
| search set | all of GenEMBL | |
| word size | wordsize=(2) | |

Expected scores lists scores until E() value reaches 2.0

### Pileup

### Default parameters:

| | |
|---|---|
| gap creation penalty | gap weight = 5 |
| gap extension penalty | gap length weight = 12 |
| plot figure | one page plot density =2.7 |

### Sequencher Program

### Default parameters:

| | |
|---|---|
| Automatic Assembly | Dirty data algorithm =slower contig assembly but more |
| | rigorous comparisons between the sequences |
| | minimum match =85% |
| | minimum overlap =20 |

### BLAST 2 (blastp, tblastn)

| | | | |
|---|---|---|---|
| Default parameters: | V=50 | Lambda=.329 | W=3 |
| | B=50 | K=0.140 | X=22 |
| | E=10 | H=0.427 | |
| blast n | | | |
| Default parameters: | V=100 | Lambda=1.37 | W=11 |
| | B=250 | K=0.171 | X1=22 |
| | E=10 | H=1.31 | X2=25 |

### BLAST 2 Command Line Arguments

| | | |
|---|---|---|
| -v Hits | number of best scores to show | |
| -b Alignments | number of best alignments to show | |
| -e Expectation value (E) | | [Real] default = 10.0 |
| -m Alignment view options: | | 0 = pairwise, |
| | | 1 = master-slave showing |
| | | identities, |
| | | 2 = master-slave, no |
| | | identities, |
| | | 3 = flat master-slave, show |
| | | identities, |
| | | 4 = flat master-slave, no |
| | | identities, |
| | | 5 = master-slave, no |
| | | identities and blunt ends, |
| | | 6 = flat master-slave, no |
| | | identities and blunt ends |
| | | [Integer] |
| | | default = 0 |
| -F Filter query seq. | (DUST with blastn, SEG with others) [T/F] default = T | |
| -G Cost to open a gap | (zero invokes default behavior) [Integer] default = 0 | |
| -E Cost to extend a gap | (zero invokes default behavior) [Integer] default = 0 | |
| -X X dropoff value for gapped alignment | (in bits) (zero invokes default behavior) [Integer]default = 0 | |
| -I Show GI's in deflines | [T/F] default = F | |
| -q Penalty for a nucleotide mismatch | (blastn only) [Integer] default = -3 | |
| -r Reward for a nucleotide match | (blastn only) [Integer] default = 1 | |
| -f Threshold for extending hits | default if zero [Integer] default = 0 | |
| -g Perfom gapped alignment | (not available with tblastx) [T/F] default = T | |
| -q Query Genetic code to use | [Integer] default = 1 | |
| -D DB Genetic code | (for tblast[nx] only) [Integer] default = 1 | |
| -J Believe the query defline | [T/F] default = F | |
| -M Matrix | [String] default = BLOSUM62 | |
| -W Word size | default if zero [Integer] default = 0 | |
| -z Effective length of the database | (use zero for the real size) [Integer] default = 0 | |
| -a Number of processors to use | [Integer] default = site configurable (SeqServer.conf) | |

Allowed and default values for gap open/gap extension cost (-G/-E) parameters:

| | | | | | | | | | | | | |
|---|---|---|---|---|---|---|---|---|---|---|---|---|
| BLOSUM62 | | | | | | | | | | | | |
| -G | 9 | 8 | 7 | 12 | 11 | 10 | | | | | | |
| -E | 2 | 2 | 2 | | 1 | 1 | 1 | | | | | |
| BLOSUM50 | | | | | | | | | | | | |
| -G | 12 | 11 | 10 | 9 | 15 | 14 | 13 | 12 | 18 | 17 | 16 | 15 |
| -E | 3 | 3 | 3 | 3 | 2 | 2 | 2 | 2 | 1 | 1 | 1 | 1 |
| PAM250 | | | | | | | | | | | | |
| -G | 13 | 12 | 11 | 10 | 15 | 14 | 13 | 12 | 19 | 18 | 17 | 16 |
| -E | 3 | 3 | 3 | 3 | 2 | 2 | 2 | 2 | 1 | 1 | 1 | 1 |
| BLOSUM90 | | | | | | | | | | | | |
| -G | 8 | 7 | 6 | 11 | 10 | 9 | | | | | | |
| -E | 2 | 2 | 2 | 1 | 1 | 1 | | | | | | |
| PAM30 | | | | | | | | | | | | |
| -G | 5 | 4 | 3 | 7 | 6 | 5 | 10 | 9 | 8 | | | |
| -E | 3 | 3 | 3 | 2 | 2 | 2 | 1 | 1 | 1 | | | |
| PAM70 | | | | | | | | | | | | |
| -G | 6 | 5 | 4 | 8 | 7 | 6 | 11 | 10 | 9 | | | |
| -E | 3 | 3 | 3 | 2 | 2 | 2 | 1 | 1 | 1 | | | |

## Claims

1. An isolated nucleotide sequence encoding a functionally active elongase having identity to at least about 50% of the nucleotide sequence shown in Figure 6, or its complement.

2. The isolated nucleotide sequence of claim 1 wherein said sequence is represented by the sequence shown in Figure 6.

3. A purified protein encoded by said nucleotide sequence of claims 1 or 2.

4. A method of producing an elongase enzyme comprising the steps of:
a) isolating a nucleotide sequence represented by Figure 6;
b) constructing a vector comprising: i) said isolated nucleotide sequence operably linked to ii) a promoter;
c) introducing said vector into a host cell under time and conditions sufficient for expression of said elongase enzyme.

5. A vector comprising: a) a nucleotide sequence as represented in Figure 6 operably linked to b) a promoter.

6. A host cell comprising said vector of claim 5.

7. A plant cell, plant or plant tissue comprising said vector of claim 5, wherein expression of said nucleotide sequence of said vector results in the production of at least one fatty acid selected from the group consisting of a monounsaturated fatty acid and a polyunsaturated fatty acid by said plant cell, plant or plant tissue.

8. A transgenic plant comprising said vector of claim 5, wherein expression of said nucleotide sequence of said vector results in the production of a polyunsaturated fatty acid in seeds of said transgenic plant.

9. A transgenic non-human mammal whose genome comprises a DNA sequence as defined in claim 1 operably linked to a promoter.

10. The transgenic non-human mammal of claim 9 wherein said DNA sequence is represented by Figure 6.

11. A method for producing a polyunsaturated fatty acid comprising the steps of:
a) isolating said nucleotide sequence represented by Figure 6;
b) constructing a vector comprising said isolated nucleotide sequence;
c) introducing said vector into a host cell under time and conditions sufficient for expression of an elongase enzyme encoded by said isolated nucleotide sequence; and
d) exposing said expressed elongase enzyme to a substrate polyunsaturated fatty acid in order to convert said substrate to a product polyunsaturated fatty acid.

12. The method according to claim 11 further comprising the step of exposing said product polyunsaturated fatty acid to at least one desaturase in order to convert said product polyunsaturated fatty acid to another polyunsaturated fatty acid.

13. The method according to claim 12 further comprising the step of exposing said another polyunsaturated fatty acid to one or more enzymes selected from the group consisting of at least one elongase and at least one additional desaturase in order to convert said another polyunsaturated fatty acid to a final polyunsaturated fatty acid.

14. An isolated nucleotide sequence encoding a functionally active elongase having identity to at least about 35% of the nucleotide sequence shown in Figure 22, or its complement.

15. The isolated nucleotide sequence of claim 14 wherein said sequence is represented by the sequence shown in Figure 22.

16. A purified protein encoded by said nucleotide sequence of claims 14 or 15.

17. A method of producing an elongase enzyme comprising the steps of:
a) isolating a nucleotide sequence represented by Figure 22;
b) constructing a vector comprising: i) said isolated nucleotide sequence operably linked to ii) a promoter;
c) introducing said vector into a host cell under time and conditions sufficient for expression of said elongase enzyme.

18. A vector comprising: a) a nucleotide sequence as represented in Figure 22 operably linked to b) a promoter.

19. A host cell comprising said vector of claim 18.

20. A plant cell, plant or plant tissue comprising said vector of claim 18, wherein expression of said nucleotide sequence of said vector results in the production of a polyunsaturated fatty acid by said plant cell, plant or plant tissue.

21. A transgenic plant comprising said vector of claim 18, wherein expression of said nucleotide sequence of said vector results in the production of a polyunsaturated fatty acid in seeds of said transgenic plant.

22. A transgenic non-human mammal whose genome comprises a DNA sequence as defined in claim 14, from M. alpina, encoding an elongase operably linked to a promoter.

23. The transgenic non-human mammal of claim 22 wherein said DNA sequence is represented by Figure 22.

24. A method for producing a polyunsaturated fatty acid comprising the steps of:
a) isolating said nucleotide sequence represented by Figure 22;
b) constructing a vector comprising said isolated nucleotide sequence;
c) introducing said vector into a host cell under time and conditions sufficient for expression of an elongase enzyme encoded by said isolated nucleotide sequence; and
d) exposing said expressed elongase enzyme to a substrate polyunsaturated fatty acid in order to convert said substrate to a product polyunsaturated fatty acid.

25. The method according to claim 24 further comprising the step of exposing said product polyunsaturated fatty acid to at least one desaturase in order to convert said product polyunsaturated fatty acid to another polyunsaturated fatty acid.

26. The method according to claim 25 further comprising the step of exposing said another polyunsaturated fatty acid to one or more enzymes selected from the group consisting of at least one elongase and at least one additional desaturase in order to convert said another polyunsaturated fatty acid to a final polyunsaturated fatty acid.

27. An isolated nucleotide sequence having identity to or complementary to the sequence shown in Figure 43 wherein said isolated nucleotide sequence encodes a polypeptide having elongase activity.

28. A purified protein encoded by said nucleotide sequence of claims 27.

29. A method of producing elongase enzyme comprising the steps of:
a) isolating a nucleotide sequence represented by Figure 43;
b) constructing a vector comprising: i) said isolated nucleotide sequence operably linked to ii) a promoter;
c) introducing said vector into a host cell under time and conditions sufficient for expression of said elongase enzyme.

30. A vector comprising: a) a nucleotide sequence as represented in Figure 43 operably linked to b) a promoter.

31. A host cell comprising said vector of claim 30.

32. A plant cell, plant or plant tissue comprising said vector of claim 30, wherein expression of said nucleotide sequence of said vector results in the production of at least one fatty acid selected from the group consisting of a monounsaturated fatty acid and a polyunsaturated fatty acid by said plant cell, plant or plant tissue.

33. A transgenic plant comprising said vector of claim 30, wherein expression of said nucleotide sequence of said vector results in the production of a polyunsaturated fatty acid in seeds of said transgenic plant.

34. A transgenic non-human mammal whose genome comprises a DNA sequence as defined in claim 27 encoding an elongase operably linked to a promoter.

35. The transgenic non-human mammal of claim 34 wherein said DNA sequence is represented by Figure 43.

36. A method for producing a polyunsaturated fatty acid comprising the steps of:
a) isolating said nucleotide sequence represented by Figure 43;
b) constructing a vector comprising said isolated nucleotide sequence;
c) introducing said vector into a host cell under time and conditions sufficient for expression of an elongase enzyme encoded by said isolated nucleotide sequence; and
d) exposing said expressed elongase enzyme to a substrate polyunsaturated fatty acid in order to convert said substrate to a product polyunsaturated fatty acid.

37. The method according to claim 36 further comprising the step of exposing said product polyunsaturated fatty acid to at least one desaturase in order to convert said product polyunsaturated fatty acid to another polyunsaturated fatty acid.

38. The method according to claim 37 further comprising the step of exposing said another polyunsaturated fatty acid to one or more enzymes selected from the group consisting of at least one elongase and at least one additional desaturase in order to convert said another polyunsaturated fatty acid to a final polyunsaturated fatty acid.

39. An isolated nucleotide sequence having identity to or complementary to the nucleotide sequence shown in Figure 46.

40. The isolated nucleotide sequence of claim 39 wherein said sequence is represented by the sequence shown in Figure 46.

41. A method of producing an elongase enzyme comprising the steps of:
a) isolating a nucleotide sequence represented by Figure 46;
b) constructing a vector comprising: i) said isolated nucleotide sequence operably linked to ii) a promoter;
c) introducing said vector into a host cell under time and conditions sufficient for expression of said elongase enzyme.

42. A vector comprising: a) a nucleotide sequence as represented in Figure 46 operably linked to b) a promoter.

43. A host cell comprising said vector of claim 42.

44. A plant cell, plant or plant tissue comprising said vector of claim 42, wherein expression of said nucleotide sequence of said vector results in the production of a polyunsaturated fatty acid by said plant cell, plant or plant tissue.

45. A transgenic plant comprising said vector of claim 42, wherein expression of said nucleotide sequence of said vector results in the production of a polyunsaturated fatty acid in seeds of said transgenic plant.

46. A transgenic non-human mammal whose genome comprises a DNA sequence as defined in claim 40, from C. elegans, encoding an elongase operably linked to a promoter.

47. A method for producing a polyunsaturated fatty acid comprising the steps of:
a) isolating said nucleotide sequence represented by Figure 46;
b) constructing a vector comprising said isolated nucleotide sequence;
c) introducing said vector into a host cell under time and conditions sufficient for expression of an elongase enzyme encoded by said isolated nucleotide sequence; and
d) exposing said expressed elongase enzyme to a substrate polyunsaturated fatty acid in order to convert said substrate to a product polyunsaturated fatty acid.

48. The method according to claim 47 further comprising the step of exposing said product polyunsaturated fatty acid to at least one desaturase in order to convert said product polyunsaturated fatty acid to another polyunsaturated fatty acid.

49. The method according to claim 48 further comprising the step of exposing said another polyunsaturated fatty acid to one or more enzymes selected from the group consisting of at least one elongase and at least one additional desaturase in order to convert said another polyunsaturated fatty acid to a final polyunsaturated fatty acid.

## Patentansprüche

1. Eine isolierte Nukleotidsequenz, die eine funktionell aktive Elongase codiert, die mindestens ungefähr 50% Identität hat mit der Nukleotidsequenz, die in Fig. 6 dargestellt ist, oder ihrem Komplement.

2. Die isolierte Nukleotidsequenz gemäß Anspruch 1, worin die Sequenz durch die in Fig. 6 gezeigte Sequenz dargestellt wird.

3. Ein gereinigtes Protein, das von der Nukleotidsequenz gemäß Anspruch 1 oder 2 codiert wird.

4. Ein Verfahren zur Herstellung eines Elongase-Enzyms, das folgende Schritte umfasst:
a) Isolierung einer in Fig. 6 dargestellten Nukleotidsequenz,
b) Konstruktion eines Vektors, der Folgendes umfasst: i) die isolierte Nukleotidsequenz, die operativ mit ii) einem Promotor verbunden ist,
c) Einführung des Vektors in eine Wirtszelle unter Zeit und Bedingungen, die zur Expression des Elongase-Enzyms ausreichend sind.

5. Ein Vektor, der Folgendes umfasst: a) eine Nukleotidsequenz wie in Fig. 6 dargestellt, operativ mit b) einem Promotor verbunden.

6. Eine Wirtszelle, die den Vektor gemäß Anspruch 5 umfasst.

7. Eine Pflanzenzelle, Pflanze oder Pflanzengewebe, die/das den Vektor gemäß Anspruch 5 umfasst, worin die Expression der Nukleotidsequenz des Vektors zur Produktion mindestens einer Fettsäure, gewählt aus der Gruppe bestehend aus einer einfach ungesättigten Fettsäure und einer mehrfach ungesättigten Fettsäure, durch die Pflanzenzelle, Pflanze oder das Pflanzengewebe, führt.

8. Eine transgene Pflanze, die den Vektor gemäß Anspruch 5 umfasst, worin die Expression der Nukleotidsequenz des Vektors zur Produktion einer mehrfach ungesättigten Fettsäure in Samen der transgenen Pflanze führt.

9. Ein transgenes nicht menschliches Säugetier, dessen Genom eine DNA-Sequenz wie in Anspruch 1 definiert, operativ mit einem Promotor verbunden, umfasst.

10. Das transgene nicht menschliche Säugetier gemäß Anspruch 9, worin die DNA-Sequenz durch Fig. 6 dargestellt wird.

11. Ein Verfahren zur Herstellung einer mehrfach ungesättigten Fettsäure, das folgende Schritte umfasst:
a) Isolation der in Fig. 6 dargestellten Nukleotidsequenz,
b) Konstruktion eines Vektors, der die isolierte Nukleotidsequenz umfasst,
c) Einführung des Vektors in eine Wirtszelle unter Zeit und Bedingungen, die zur Expression eines Elongase-Enzyms, das durch die isolierte Nukleotidsequenz codiert wird, ausreichen, und
d) Aussetzen des exprimierten Elongase-Enzyms gegenüber einer mehrfach ungesättigten Substrat-Fettsäure zur Umwandlung des Substrats in eine mehrfach ungesättigte Produkt-Fettsäure.

12. Das Verfahren gemäß Anspruch 11, das weiter den Schritt Aussetzen der mehrfach ungesättigten Produkt-Fettsäure gegenüber mindestens einer Desaturase zur Umwandlung der mehrfach ungesättigten Produkt-Fettsäure in eine andere mehrfach ungesättigte Fettsäure umfasst.

13. Das Verfahren gemäß Anspruch 12, das weiter den Schritt Aussetzen der anderen mehrfach ungesättigten Fettsäure gegenüber einem oder mehreren Enzymen, gewählt aus der Gruppe bestehend aus mindestens einer Elongase und mindestens einer zusätzlichen Desaturase, zur Umwandlung der anderen mehrfach ungesättigten Fettsäure in eine mehrfach ungesättigte End-Fettsäure umfasst.

14. Eine isolierte Nukleotidsequenz, die eine funktionell aktive Elongase codiert, die Identität hat mit mindestens ungefähr 35% der in Fig. 22 dargestellten Nukleotidsequenz, oder ihrem Komplement.

15. Die isolierte Nukleotidsequenz gemäß Anspruch 14, worin die Sequenz durch die in Fig. 22 gezeigte Sequenz dargestellt wird.

16. Ein gereinigtes Protein, codiert durch die Nukleotidsequenz gemäß Anspruch 14 oder 15.

17. Ein Verfahren zur Herstellung eines Elongase-Enzyms, das folgende Schritte umfasst:
a) Isolation einer Nukleotidsequenz, dargestellt durch Figur 22,
b) Konstruktion eines Vektors, der Folgendes umfasst: i) die isolierte Nukleotidsequenz, operativ mit ii) einem Promotor verbunden,
c) Einführen des Vektors in eine Wirtszelle unter Zeit und Bedingungen, die zur Expression des Elongase-Enzyms ausreichen.

18. Ein Vektor, der Folgendes umfasst: a) eine Nukleotidsequenz wie in Fig. 22 dargestellt, operativ mit b) einem Promotor verbunden.

19. Eine Wirtszelle, die den Vektor gemäß Anspruch 18 umfasst.

20. Eine Pflanzenzelle, Pflanze oder Pflanzengewebe, die/das den Vektor gemäß Anspruch 18 umfasst, worin die Expression der Nukleotidsequenz des Vektors zur Produktion einer mehrfach ungesättigten Fettsäure durch die Pflanzenzelle, Pflanze oder das Pflanzengewebe führt.

21. Eine transgene Pflanze, die den Vektor gemäß Anspruch 18 umfasst, worin die Expression der Nukleotidsequenz des Vektors zur Produktion einer mehrfach ungesättigten Fettsäure in Samen der transgenen Pflanze führt.

22. Ein transgenes nicht menschliches Säugetier, dessen Genom eine DNA-Sequenz wie in Anspruch 14 umfasst, von M. alpina, welche eine Elongase codiert, die operativ mit einem Promotor verbunden ist.

23. Das transgene nicht menschliche Säugetier gemäß Anspruch 22, worin die DNA-Sequenz in Fig. 22 dargestellt wird.

24. Ein Verfahren zur Herstellung einer mehrfach ungesättigten Fettsäure, das folgende Schritte umfasst:
a) Isolieren der Nukleotidsequenz, die in Fig. 22 dargestellt ist,
b) Konstruktion eines Vektors, der die isolierte Nukleotidsequenz umfasst,
c) Einführen des Vektors in eine Wirtszelle unter Zeit und Bedingungen, die zur Expression eines Elongase-Enzyms, das durch die isolierte Nukleotidsequenz codiert wird, ausreichen, und
d) Aussetzen des exprimierten Elongase-Enzyms gegenüber einer mehrfach ungesättigten Substrat-Fettsäure, um das Substrat in eine mehrfach ungesättigte Produkt-Fettsäure umzuwandeln.

25. Das Verfahren gemäß Anspruch 24, das weiter den Schritt Aussetzen der mehrfach ungesättigten Produkt-Fettsäure gegenüber einer Desaturase, um die mehrfach ungesättigte Produkt-Fettsäure in eine andere mehrfach ungesättigte Fettsäure umzuwandeln, umfasst.

26. Das Verfahren gemäß Anspruch 25, das weiter den Schritt Aussetzen der anderen mehrfach ungesättigten Fettsäure gegenüber einem oder mehreren Enzymen, gewählt aus der Gruppe bestehend aus mindestens einer Elongase und mindestens einer zusätzlichen Desaturase, um die andere mehrfach ungesättigte Fettsäure in eine mehrfach ungesättigte End-Fettsäure umzuwandeln, umfasst.

27. Eine isolierte Nukleotidsequenz, die identisch mit oder komplementär zu der in Fig. 43 dargestellten Sequenz ist, worin die isolierte Nukleotidsequenz ein Polypeptid mit Elongase-Aktivität codiert.

28. Ein gereinigtes Protein, codiert durch die Nukleotidsequenz in Anspruch 27.

29. Ein Verfahren zur Herstellung von Elongase-Enzym, das folgende Schritte umfasst:
a) Isolation einer Nukleotidsequenz, die in Fig. 43 dargestellt wird,
b) Konstruktion eines Vektors, der Folgendes umfasst: i) die isolierte Nukleotidsequenz, operativ mit ii) einem Promotor verbunden,
c) Einführen des Vektors in eine Wirtszelle unter Zeit und Bedingungen, die zur Expression des Elongase-Enzyms ausreichen.

30. Ein Vektor, der Folgendes umfasst: a) eine Nukleotidsequenz wie in Fig. 43 dargestellt, operativ mit b) einem Promotor verbunden.

31. Eine Wirtszelle, die den Vektor gemäß Anspruch 30 umfasst.

32. Eine Pflanzenzelle, Pflanze oder ein Pflanzengewebe, die/das den Vektor gemäß Anspruch 30 umfasst, worin die Expression der Nukleotidsequenz des Vektors zur Produktion mindestens einer Fettsäure, gewählt aus der Gruppe bestehend aus einer einfach ungesättigten Fettsäure und einer mehrfach ungesättigten Fettsäure, durch die Pflanzenzelle oder Pflanze oder das Pflanzengewebe führt.

33. Eine transgene Pflanze, die den Vektor gemäß Anspruch 30 umfasst, worin die Expression der Nukleotidsequenz des Vektors zur Produktion einer mehrfach ungesättigten Fettsäure in Samen der transgenen Pflanze führt.

34. Ein transgenes nicht menschliches Säugetier, dessen Genom eine DNA-Sequenz wie in Anspruch 27 definiert umfasst, welche eine Elongase codiert, die operativ mit einem Promotor verbunden ist.

35. Das transgene nicht menschliche Säugetier gemäß Anspruch 34, worin die DNA-Sequenz in Fig. 43 dargestellt wird.

36. Ein Verfahren zur Herstellung einer mehrfach ungesättigten Fettsäure, das folgende Schritte umfasst:
a) Isolation der in Fig. 43 dargestellten Nukleotidsequenz,
b) Konstruktion eines Vektors, der die isolierte Nukleotidsequenz umfasst,
c) Einführen des Vektors in eine Wirtszelle unter Zeit und Bedingungen, die zur Expression eines Elongase-Enzyms, das durch die isolierte Nukleotidsequenz codiert wird, ausreichen, und
d) Aussetzen des exprimierten Elongase-Enzyms gegenüber einer mehrfach ungesättigten Substrat-Fettsäure, um das Substrat in eine mehrfach ungesättigte Produkt-Fettsäure umzuwandeln.

37. Das Verfahren gemäß Anspruch 36, das weiter den Schritt Aussetzen der mehrfach ungesättigten Produkt-Fettsäure gegenüber mindestens einer Desaturase, um die mehrfach ungesättigte Produkt-Fettsäure in eine andere mehrfach ungesättigte Fettsäure umzuwandeln, umfasst.

38. Das Verfahren gemäß Anspruch 37, das weiter den Schritt Aussetzen der anderen mehrfach ungesättigten Fettsäure gegenüber einem oder mehreren Enzymen, die gewählt sind aus der Gruppe bestehend aus mindestens einer Elongase und mindestens einer zusätzlichen Desaturase, um die andere mehrfach ungesättigte Fettsäure in eine mehrfach ungesättigte End-Fettsäure umzuwandeln, umfasst.

39. Eine isolierte Nukleotidsequenz, die identisch mit oder komplementär zu der in Fig. 46 dargestellten Nukleotidsequenz ist.

40. Die isolierte Nukleotidsequenz gemäß Anspruch 39, worin die Sequenz durch die in Fig. 46 gezeigte Sequenz dargestellt wird.

41. Ein Verfahren zur Herstellung eines Elongase-Enzyms, das folgende Schritte umfasst:
a) Isolation einer Nukleotidsequenz, die durch Fig. 46 dargestellt wird,
b) Konstruktion eines Vektors, der Folgendes umfasst: i)die isolierte Nukleotidsequenz, operativ mit ii) einem Promotor verbunden,
c) Einführen des Vektors in eine Wirtszelle unter Zeit und Bedingungen, die zur Expression des Elongase-Enzyms ausreichend sind.

42. Ein Vektor, der Folgendes umfasst: a) eine Nukleotidsequenz wie in Fig. 46 dargestellt, operativ mit b) einem Promotor verbunden.

43. Eine Wirtszelle, die den Vektor gemäß Anspruch 42 umfasst.

44. Eine Pflanzenzelle, Pflanze oder Pflanzengewebe, die/das den Vektor gemäß Anspruch 42 umfasst, worin die Expression der Nukleotidsequenz des Vektors zur Produktion einer mehrfach ungesättigten Fettsäure durch die Pflanzenzelle, Pflanze oder das Pflanzengewebe führt.

45. Eine transgene Pflanze, die den Vektor gemäß Anspruch 42 umfasst, worin die Expression der Nukleotidsequenz des Vektors zur Produktion einer mehrfach ungesättigten Fettsäure in Samen der transgenen Pflanze führt.

46. Ein transgenes nicht menschliches Säugetier, dessen Genom eine DNA-Sequenz wie in Anspruch 40 definiert, von C. elegans, umfasst, die eine Elongase codiert, welche operativ mit einem Promotor verbunden ist.

47. Ein Verfahren zur Herstellung einer mehrfach ungesättigten Fettsäure, das folgende Schritte umfasst:
a) Isolation der Nukleotidsequenz, die in Fig. 46 dargestellt wird,
b) Konstruktion eines Vektors, der die isolierte Nukleotidsequenz umfasst,
c) Einführung des Vektors in eine Wirtszelle unter Zeit und Bedingungen, die zur Expression eines Elongase-Enzyms, das durch die isolierte Nukleotidsequenz codiert wird, ausreichen, und
d) Aussetzen des exprimierten Elongase-Enzyms gegenüber einer mehrfach ungesättigten Substrat-Fettsäure, um das Substrat in eine mehrfach ungesättigte Produkt-Fettsäure umzuwandeln.

48. Das Verfahren gemäß Anspruch 47, das weiter den Schritt Aussetzen der mehrfach ungesättigten Produkt-Fettsäure gegenüber mindestens einer Desaturase zur Umwandlung der mehrfach ungesättigten Produkt-Fettsäure in eine andere mehrfach ungesättigte Fettsäure umfasst.

49. Das Verfahren gemäß Anspruch 48, das weiter den Schritt Aussetzen der anderen mehrfach ungesättigten Fettsäure gegenüber einem oder mehreren Enzymen, gewählt aus der Gruppe bestehend aus mindestens einer Elongase und mindestens einer zusätzlichen Desaturase, zur Umwandlung der anderen mehrfach ungesättigten Fettsäure in eine mehrfach ungesättigte End-Fettsäure, umfasst.

## Revendications

1. Séquence de nucléotides isolée codant pour une élongase fonctionnellement active présentant une identité par rapport à au moins 50 % environ de la séquence de nucléotides présentée à la figure 6, ou sa complémentaire.

2. Séquence de nucléotides isolée de la revendication 1, ladite séquence étant représentée par la séquence présentée à la figure 6.

3. Protéine purifiée codée par ladite séquence de nucléotides des revendications 1 ou 2.

4. Procédé de production d'une enzyme élongase comprenant les étapes consistant à :
a) isoler une séquence de nucléotides représentée par la figure 6 ;
b) construire un vecteur contenant : i) ladite séquence de nucléotides isolée fonctionnellement reliée à ii) un promoteur ;
c) introduire ledit vecteur dans une cellule hôte pour un espace de temps et dans des conditions, suffisants pour l'expression de ladite enzyme élongase.

5. Vecteur contenant : a) une séquence de nucléotides telle que représentée à la figure 6 fonctionnellement reliée à b) un promoteur.

6. Cellule hôte contenant ledit vecteur de la revendication 5.

7. Cellule végétale, plante ou tissu végétal contenant ledit vecteur de la revendication 5, dans lesquels l'expression de ladite séquence de nucléotides dudit vecteur donne lieu à la production d'au moins un acide gras choisi dans le groupe formé par un acide gras mono-insaturé et un acide gras poly-insaturé par ladite cellule végétale, ladite plante ou ledit tissu végétal.

8. Plante transgénique contenant ledit vecteur de la revendication 5, dans laquelle l'expression de ladite séquence de nucléotides dudit vecteur donne lieu à la production d'un acide gras poly-insaturé dans les graines de ladite plante transgénique.

9. Mammifère transgénique non humain dont le génome comporte une séquence d'ADN telle que définie dans la revendication 1 fonctionnellement reliée à un promoteur.

10. Mammifère transgénique non humain de la revendication 9 dans lequel ladite séquence d'ADN est représentée par la figure 6.

11. Procédé de production d'un acide gras poly-insaturé comprenant les étapes consistant à :
a) isoler ladite séquence de nucléotides représentée par la figure 6 ;
b) construire un vecteur contenant ladite séquence de nucléotides isolée ;
c) introduire ledit vecteur dans une cellule hôte pour un espace de temps et dans des conditions suffisants pour l'expression d'une enzyme élongase codée par ladite séquence de nucléotides isolée ; et
d) exposer ladite enzyme élongase exprimée à un acide gras poly-insaturé substrat afin de transformer ledit substrat en un acide gras poly-insaturé produit.

12. Procédé selon la revendication 11 comprenant en outre l'étape consistant à exposer ledit acide gras poly-insaturé produit à au moins une désaturase afin de transformer ledit acide gras poly-insaturé produit en un autre acide gras poly-insaturé.

13. Procédé selon la revendication 12 comprenant en outre l'étape consistant à exposer ledit autre acide gras poly-insaturé à une ou plusieurs enzymes choisies dans le groupe formé par au moins une élongase et au moins une désaturase supplémentaire afin de transformer ledit autre acide gras poly-insaturé en un acide gras poly-insaturé final.

14. Séquence de nucléotides isolée codant pour une élongase fonctionnellement active présentant une identité par rapport à au moins 35 % environ de la séquence de nucléotides présentée à la figure 22, ou sa complémentaire.

15. Séquence de nucléotides isolée de la revendication 14, ladite séquence étant représentée par la séquence présentée à la figure 22.

16. Protéine purifiée codée par ladite séquence de nucléotides des revendications 14 ou 15.

17. Procédé de production d'une enzyme élongase comprenant les étapes consistant à :
a) isoler une séquence de nucléotides représentée par la figure 22 ;
b) construire un vecteur contenant : i) ladite séquence de nucléotides isolée fonctionnellement reliée à ii) un promoteur ;
c) introduire ledit vecteur dans une cellule hôte pour un espace de temps et dans des conditions suffisants pour l'expression de ladite enzyme élongase.

18. Vecteur contenant : a) une séquence de nucléotides telle que représentée à la figure 22 fonctionnellement reliée à b) un promoteur.

19. Cellule hôte contenant ledit vecteur de la revendication 18.

20. Cellule végétale, plante ou tissu végétal contenant ledit vecteur de la revendication 18, dans lesquels l'expression de ladite séquence de nucléotides dudit vecteur donne lieu à la production d'un acide gras poly-insaturé par ladite cellule végétale, ladite plante ou ledit tissu végétal.

21. Plante transgénique contenant ledit vecteur de la revendication 18, dans laquelle l'expression de ladite séquence de nucléotides dudit vecteur donne lieu à la production d'un acide gras poly-insaturé dans les graines de ladite plante transgénique.

22. Mammifère transgénique non humain dont le génome comporte une séquence d'ADN telle que définie dans la revendication 14, issue de M. alpina, codant pour une élongase fonctionnellement reliée à un promoteur.

23. Mammifère transgénique non humain de la revendication 22 dans lequel ladite séquence d'ADN est représentée par la figure 22.

24. Procédé de production d'un acide gras poly-insaturé comprenant les étapes consistant à :
a) isoler ladite séquence de nucléotides représentée par la figure 22 ;
b) construire un vecteur contenant ladite séquence de nucléotides isolée ;
c) introduire ledit vecteur dans une cellule hôte pour un espace de temps et dans des conditions suffisants pour l'expression d'une enzyme élongase codée par ladite séquence de nucléotides isolée; et
d) exposer ladite enzyme élongase exprimée à un acide gras poly-insaturé substrat afin de transformer ledit substrat en un acide gras poly-insaturé produit.

25. Procédé selon la revendication 24 comprenant en outre l'étape consistant à exposer ledit acide gras poly-insaturé produit à au moins une désaturase afin de transformer ledit acide gras poly-insaturé produit en un autre acide gras poly-insaturé.

26. Procédé selon la revendication 25 comprenant en outre l'étape consistant à exposer ledit autre acide gras poly-insaturé à une ou plusieurs enzymes choisies dans le groupe formé par au moins une élongase et au moins une désaturase supplémentaire afin de transformer ledit autre acide gras poly-insaturé en un acide gras poly-insaturé final.

27. Séquence de nucléotides isolée présentant une identité, ou une complémentarité, par rapport à la séquence présentée à la figure 43 où ladite séquence de nucléotides isolée code pour un polypeptide doué d'activité élongase.

28. Protéine purifiée codée par ladite séquence de nucléotides de la revendication 27.

29. Procédé de production d'une enzyme élongase comprenant les étapes consistant à :
a) isoler une séquence de nucléotides représentée par la figure 43 ;
b) construire un vecteur contenant : i) ladite séquence de nucléotides isolée fonctionnellement reliée à ii) un promoteur ;
c) introduire ledit vecteur dans une cellule hôte pour un espace de temps et dans des conditions, suffisants pour l'expression de ladite enzyme élongase.

30. Vecteur contenant : a) une séquence de nucléotides telle que représentée à la figure 43 fonctionnellement reliée à b) un promoteur.

31. Cellule hôte contenant ledit vecteur de la revendication 30.

32. Cellule végétale, plante ou tissu végétal contenant ledit vecteur de la revendication 30, dans lesquels l'expression de ladite séquence de nucléotides dudit vecteur donne lieu à la production d'au moins un acide gras choisi dans le groupe formé par un acide gras mono-insaturé et un acide gras poly-insaturé par ladite cellule végétale, ladite plante ou ledit tissu végétal.

33. Plante transgénique contenant ledit vecteur de la revendication 30, dans laquelle l'expression de ladite séquence de nucléotides dudit vecteur donne lieu à la production d'un acide gras poly-insaturé dans les graines de ladite plante transgénique.

34. Mammifère transgénique non humain dont le génome comporte une séquence d'ADN telle que définie dans la revendication 27 codant pour une élongase fonctionnellement reliée à un promoteur.

35. Mammifère transgénique non humain de la revendication 34 dans lequel ladite séquence d'ADN est représentée par la figure 43.

36. Procédé de production d'un acide gras poly-insaturé comprenant les étapes consistant à :
a) isoler ladite séquence de nucléotides représentée par la figure 43 ;
b) construire un vecteur contenant ladite séquence de nucléotides isolée ;
c) introduire ledit vecteur dans une cellule hôte pour un espace de temps et dans des conditions suffisants pour l'expression d'une enzyme élongase codée par ladite séquence de nucléotides isolée ; et
d) exposer ladite enzyme élongase exprimée à un acide gras poly-insaturé substrat afin de transformer ledit substrat en un acide gras poly-insaturé produit.

37. Procédé selon la revendication 36 comprenant en outre l'étape consistant à exposer ledit acide gras poly-insaturé produit à au moins une désaturase afin de transformer ledit acide gras poly-insaturé produit en un autre acide gras poly-insaturé.

38. Procédé selon la revendication 37 comprenant en outre l'étape consistant à exposer ledit autre acide gras poly-insaturé à une ou plusieurs enzymes choisies dans le groupe formé par au moins une élongase et au moins une désaturase supplémentaire afin de transformer ledit autre acide gras poly-insaturé en un acide gras poly-insaturé final.

39. Séquence de nucléotides isolée présentant une identité ou une complémentarité par rapport à la séquence de nucléotides présentée à la figure 46.

40. Séquence de nucléotides isolée de la revendication 39, ladite séquence étant représentée par la séquence présentée à la figure 46.

41. Procédé de production d'une enzyme élongase comprenant les étapes consistant à :
a) isoler une séquence de nucléotides représentée par la figure 46 ;
b) construire un vecteur contenant : i) ladite séquence de nucléotides isolée fonctionnellement reliée à ii) un promoteur ;
c) introduire ledit vecteur dans une cellule hôte pour un espace de temps et dans des conditions suffisants pour l'expression de ladite enzyme élongase.

42. Vecteur contenant : a) une séquence de nucléotides telle que représentée à la figure 46 fonctionnellement reliée à b) un promoteur.

43. Cellule hôte contenant ledit vecteur de la revendication 42.

44. Cellule végétale, plante ou tissu végétal contenant ledit vecteur de la revendication 42, dans lesquels l'expression de ladite séquence de nucléotides dudit vecteur donne lieu à la production d'un acide gras poly-insaturé par ladite cellule végétale, ladite plante ou ledit tissu végétal.

45. Plante transgénique contenant ledit vecteur de la revendication 42, dans laquelle l'expression de ladite séquence de nucléotides dudit vecteur donne lieu à la production d'un acide gras poly-insaturé dans les graines de ladite plante transgénique.

46. Mammifère transgénique non humain dont le génome comporte une séquence d'ADN telle que définie dans la revendication 40, issue de C. elegans, codant pour une élongase fonctionnellement reliée à un promoteur.

47. Procédé de production d'un acide gras poly-insaturé comprenant les étapes consistant à :
a) isoler ladite séquence de nucléotides représentée par la figure 46 ;
b) construire un vecteur contenant ladite séquence de nucléotides isolée ;
c) introduire ledit vecteur dans une cellule hôte pour un espace de temps et dans des conditions suffisants pour l'expression d'une enzyme élongase codée par ladite séquence de nucléotides isolée ; et
d) exposer ladite enzyme élongase exprimée à un acide gras poly-insaturé substrat afin de transformer ledit substrat en un acide gras poly-insaturé produit.

48. Procédé selon la revendication 47 comprenant en outre l'étape consistant à exposer ledit acide gras poly-insaturé produit à au moins une désaturase afin de transformer ledit acide gras poly-insaturé produit en un autre acide gras poly-insaturé.

49. Procédé selon la revendication 48 comprenant en outre l'étape consistant à exposer ledit autre acide gras poly-insaturé à une ou plusieurs enzymes choisies dans le groupe formé par au moins une élongase et au moins une désaturase supplémentaire afin de transformer ledit autre acide gras poly-insaturé en un acide gras poly-insaturé final.


### SEQUENCE LISTING

<110> MUKERJI, Pradip
   DAS, Tapas
   HUANG, Yung-Sheng
   PARKER-BARNES, Jennifer M.
   LEONARD, Amanda Eun-Yeong
   THURMOND, Jennifer
<120> ELONGASE GENES AND USES THEREOF
<130> 6407.US.P1
<140> US 09/145,828
   <141> 1998-09-02
<160> 78
<170> FastSEQ for Windows Version 3.0
<210> 1
   <211> 954
   <212> DNA
   <213> Mortierella alpina
<400> 1
   atggccgccg caatcttgga caaggtcaac ttcggcattg atcagccctt cggaatcaag      60
   ctcgacacct actttgctca ggcctatgaa ctcgtcaccg gaaagtccat cgactccttc     120
   gtcttccagg agggcgtcac gcctctctcg acccagagag aggtcgccat gtggactatc     180
   acttacttcg tcgtcatctt tggtggtcgc cagatcatga agagccagga cgccttcaag     240
   ctcaagcccc tcttcatcct ccacaacttc ctcctgacga tcgcgtccgg atcgctgttg     300
   ctcctgttca tcgagaacct ggtccccatc ctcgccagaa acggactttt ctacgccatc     360
   tgcgacgacg gtgcctggac ccagcgcctc gagctcctct actacctcaa ctacctggtc     420
   aagtactggg agttggccga caccgtcttt ttggtcctca agaagaagcc tcttgagttc     480
   ctgcactact tccaccactc gatgaccatg gttctctgct ttgtccagct tggaggatac     540
   acttcagtgt cctgggtccc tattaccctc aacttgactg tccacgtctt catgtactac     600
   tactacatgc gctccgctgc cggtgttcgc atctggtgga agcagtactt gaccactctc     660
   cagatcgtcc agttcgttct tgacctcgga ttcatctact tctgcgccta cacctacttc     720
   gccttcacct acttcccctg ggctcccaac gtcggcaagt gcgccggtac cgagggtgct     780
   gctctctttg gctgcggact cctctccagc tatctcttgc tctttatcaa cttctaccgc     840
   attacctaca atgccaaggc caaggcagcc aaggagcgtg gaagcaactt tacccccaag     900
   actgtcaagt ccggcggatc gcccaagaag ccctccaaga gcaagcacat ctaa           954
<210> 2
   <211> 955
   <212> DNA
   <213> Mortierella alpina
<400> 2
   atggagtcga ttgcgccatt cctcccatca aagatgccgc aagatctgtt tatggacctt      60
   gccaccgcta tcggtgtccg ggccgcgccc tatgtcgatc ctctcgaggc cgcgctggtg     120
   gcccaggccg agaagtacat ccccacgatt gtccatcaca cgcgtgggtt cctggtcgcg     180
   gtggagtcgc ctttggcccg tgagctgccg ttgatgaacc cgttccacgt gctgttgatc     240
   gtgctcgctt atttggtcac ggtctttgtg ggcatgcaga tcatgaagaa ctttgagcgg     300
   ttcgaggtca agacgttttc gctcctgcac aacttttgtc tggtctcgat cagcgcctac     360
   atgtgcggtg ggatcctgta aggcttatca ggccaactat ggactgtttg agaacgctgc     420
   tgatcatacc ttcaagggtc ttcctatggc caagatgatc tggctcttct acttctccaa     480
   gatcatggag tttgtcgaca ccatgatcat ggtcctcaag aagaacaacc gccagatctc     540
   cttcttgcac gtttaccacc acagctccat cttcaccatc tggtggttgg tcacctttgt     600
   tgcacccaac ggtgaagcct acttctctgc tgcgttgaac tcgttcatcc atgtgatcat     660
   gtacggctac tacttcttgt cggccttggg cttcaagcag gtgtcgttca tcaagttcta     720
   catcacgcgc tcgcagatga cacagttctg catgatgtcg gtccagtctt cctgggacat     780
   gtacgccatg aaggtccttg gccgccccgg ataccccttc ttcatcacgg ctctgctttg     840
   gttctacatg tggaccatgc tcggtctctt ctacaacttt tacagaaaga acgccaagtt     900
   ggccaagcag gccaaggccg acgctgccaa ggagaaggca aggaagttgc agtaa          955
<210> 3
   <211> 914
   <212> DNA
   <213> Homo sapiens
<400> 3
   atggaacatt ttgatgcatc acttagtacc tatttcaagg cattgctagg ccctcgagat    60
   actagagtaa aaggatggtt tcttctggac aattatatac ccacatttat ctgctctgtc   120
   atatatttac taattgtatg gctgggacca aaatacatga ggaataaaca gccattctct   180
   tgccggggga ttttagtggt gtataacctt ggactcacac tgctgtctct gtatatgttc   240
   tgtgagttag taacaggagt atgggaaggc aaatacaact tcttctgtca gggcacacgc   300
   accgcaggag aatcagatat gaagattatc cgtgtcctct ggtggtacta cttctccaaa   360
   ctcatagaat ttatggacac tttcttcttc atcctgcgca agaacaacca ccagatcacg   420
   gtcctgcacg tctaccacca tgcctcgatg ctgaacatct ggtggtttgt gatgaactgg   480
   gtcccctgcg gccactctta ttttggtgcc acacttaata gcttcatcca cgtcctcatg   540
   tactcttact atggtttgtc gtcagtccct tccatgcgtc catacctctg gtggaagaag   600
   tacatcactc aggggcagct gcttcagttt gtgctgacaa tcatccagac cagctgcggg   660
   gtcatctggc cgtgcacatt ccctcttggt tggttgtatt tccagattgg atacatgatt   720
   tccctgattg ctctcttcac aaacttctac attcagacct acaacaagaa aggggcctcc   780
   cgaaggaaag accacctgaa ggaccaccag aatgggtcca tggctgctgt gaatggacac   840
   accaacagct tttcacccct ggaaaacaat gtgaagccaa ggaagctgcg gaaggattga   900
   agtcaaagaa ttga                                                     914
<210> 4
   <211> 867
   <212> DNA
   <213> Caenorhabditis elegans
<400> 4
   atggctcagc atccgctcgt tcaacggctt ctcgatgtca aattcgacac gaaacgattt    60
   gtggctattg ctactcatgg gccaaagaat ttccctgacg cagaaggtcg caagttcttt   120
   gctgatcact ttgatgttac tattcaggct tcaatcctgt acatggtcgt tgtgttcgga   180
   acaaaatggt tcatgcgtaa tcgtcaacca ttccaattga ctattccact caacatctgg   240
   aatttcatcc tcgccgcatt ttccatcgca ggagctgtca aaatgacccc agagttcttt   300
   ggaaccattg ccaacaaagg aattgtcgca tcctactgca aagtgtttga tttcacgaaa   360
   ggagagaatg gatactgggt gtggctcttc atggcttcca aacttttcga acttgttgac   420
   accatcttct tggttctccg taaacgtcca ctcatgttcc ttcactggta tcaccatatt   480
   ctcaccatga tctacgcctg gtactctcat ccattgaccc caggattcaa cagatacgga   540
   atttatctta actttgtcgt ccacgccttc atgtactctt actacttcct tcgctcgatg   600
   aagattcgcg tgccaggatt catcgcccaa gctatcacat ctcttcaaat cgttcaattc   660
   atcatctctt gcgccgttct tgctcatctt ggttatctca tgcacttcac caatgccaac   720
   tgtgatttcg agccatcagt attcaagctc gcagttttca tggacacaac atacttggct   780
   cttttcgtca acttcttcct ccaatcatat gttctccgcg gaggaaaaga caagtacaag   840
   gcagtgccaa agaagaagaa caactaa                                       867
<210> 5
   <211> 33
   <212> DNA
   <213> Mortierella alpina
<400> 5
   gaattcaggc atggccgccg caatcttgga caa 33
<210> 6
   <211> 49
   <212> DNA
   <213> Mortierella alpina
<400> 6
   gaattcaggc atctcatgga tccgccatgg ccgccgcaat cttggacaa 49
<210> 7
   <211> 317
   <212> PRT
   <213> Mortierella alpina
<400> 7
   Met Ala Ala Ala Ile Leu Asp Lys Val Asn Phe Gly Ile Asp Gln Pro
    1               5                  10                  15
   Phe Gly Ile Lys Leu Asp Thr Tyr Phe Ala Gln Ala Tyr Glu Leu Val
               20                  25                  30
   Thr Gly Lys Ser Ile Asp Ser Phe Val Phe Gln Glu Gly Val Thr Pro
           35                  40                  45
   Leu Ser Thr Gln Arg Glu Val Ala Met Trp Thr Ile Thr Tyr Phe Val
       50                  55                  60
   Val Ile Phe Gly Gly Arg Gln Ile Met Lys Ser Gln Asp Ala Phe Lys
   65                  70                  75                  80
   Leu Lys Pro Leu Phe Ile Leu His Asn Phe Leu Leu Thr Ile Ala Ser
                   85                  90                  95
   Gly Ser Leu Leu Leu Leu Phe Ile Glu Asn Leu Val Pro Ile Leu Ala
               100                 105                 110
   Arg Asn Gly Leu Phe Tyr Ala Ile Cys Asp Asp Gly Ala Trp Thr Gln
           115                 120                 125
   Arg Leu Glu Leu Leu Tyr Tyr Leu Asn Tyr Leu Val Lys Tyr Trp Glu
       130                 135                 140
   Leu Ala Asp Thr Val Phe Leu Val Leu Lys Lys Lys Pro Leu Glu Phe
   145                 150                 155                 160
   Leu His Tyr Phe His His Ser Met Thr Met Val Leu Cys Phe Val Gln
                   165                 170                 175
   Leu Gly Gly Tyr Thr Ser Val Ser Trp Val Pro Ile Thr Leu Asn Leu
               180                 185                 190
   Thr Val His Val Phe Met Tyr Tyr Tyr Tyr Met Arg Ser Ala Ala Gly
           195                 200                 205
   Val Arg Ile Trp Trp Lys Gln Tyr Leu Thr Thr Leu Gln Ile Val Gln
       210                 215                 220
   Phe Val Leu Asp Leu Gly Phe Ile Tyr Phe Cys Ala Tyr Thr Tyr Phe
   225                 230                 235                 240
   Ala Phe Thr Tyr Phe Pro Trp Ala Pro Asn Val Gly Lys Cys Ala Gly
                   245                 250                 255
   Thr Glu Gly Ala Ala Leu Phe Gly Cys Gly Leu Leu Ser Ser Tyr Leu
               260                 265                 270
   Leu Leu Phe Ile Asn Phe Tyr Arg Ile Thr Tyr Asn Ala Lys Ala Lys
           275                 280                 285
   Ala Ala Lys Glu Arg Gly Ser Asn Phe Thr Pro Lys Thr Val Lys Ser
       290                 295                 300
   Gly Gly Ser Pro Lys Lys Pro Ser Lys Ser Lys His Ile
   305                 310                 315
<210> 8
   <211> 347
   <212> PRT
   <213> Saccharomyces cerevisiae
<400> 8
   Met Asn Ser Leu Val Thr Gln Tyr Ala Ala Pro Leu Phe Glu Arg Tyr
   1               5                   10                  15
   Pro Gln Leu His Asp Tyr Leu Pro Thr Leu Glu Arg Pro Phe Phe Asn
               20                  25                  30
   Ile Ser Leu Trp Glu His Phe Asp Asp Val Val Thr Arg Val Thr Asn
           35                  40                  45
   Gly Arg Phe Val Pro Ser Glu Phe Gln Phe Ile Ala Gly Glu Leu Pro
       50                  55                  60
   Leu Ser Thr Leu Pro Pro Val Leu Tyr Ala Ile Thr Ala Tyr Tyr Val
   65                  70                  75                  80
   Ile Ile Phe Gly Gly Arg Phe Leu Leu Ser Lys Ser Lys Pro Phe Lys
                   85                  90                  95
   Leu Asn Gly Leu Phe Gln Leu His Asn Leu Val Leu Thr Ser Leu Ser
               100                 105                 110
   Leu Thr Leu Leu Leu Leu Met Val Glu Gln Leu Val Pro Ile Ile Val
           115                 120                 125
   Gln His Gly Leu Tyr Phe Ala Ile Cys Asn Ile Gly Ala Trp Thr Gln
       130                 135                 140
   Pro Leu Val Thr Leu Tyr Tyr Met Asn Tyr Ile Val Lys Phe Ile Glu
   145                 150                 155                 160
   Phe Ile Asp Thr Phe Phe Leu Val Leu Lys His Lys Lys Leu Thr Phe
                   165                 170                 175
   Leu His Thr Tyr His His Gly Ala Thr Ala Leu Leu Cys Tyr Thr Gln
               180                 185                 190
   Leu Met Gly Thr Thr Ser Ile Ser Trp Val Pro Ile Ser Leu Asn Leu
           195                 200                 205
   Gly Val His Val Val Met Tyr Trp Tyr Tyr Phe Leu Ala Ala Arg Gly
       210                 215                 220
   Ile Arg Val Trp Trp Lys Glu Trp Val Thr Arg Phe Gln Ile Ile Gln
   225                 230                 235                 240
   Phe Val Leu Asp Ile Gly Phe Ile Tyr Phe Ala Val Tyr Gln Lys Ala
                   245                 250                 255
   Val His Leu Tyr Phe Pro Ile Leu Pro His Cys Gly Asp Cys Val Gly
               260                 265                 270
   Ser Thr Thr Ala Thr Phe Ala Gly Cys Ala Ile Ile Ser Ser Tyr Leu
           275                 280                 285
   Val Leu Phe Ile Ser Phe Tyr Ile Asn Val Tyr Lys Arg Lys Gly Thr
       290                 295                 300
   Lys Thr Ser Arg Val Val Lys Arg Ala His Gly Gly Val Ala Ala Lys
   305                 310                 315                 320
   Val Asn Glu Tyr Val Asn Val Asp Leu Lys Asn Val Pro Thr Pro Ser
                   325                 330                 335
   Pro Ser Pro Lys Pro Gln His Arg Arg Lys Arg
               340                 345
<210> 9
   <211> 345
   <212> PRT
   <213> Saccharomyces cerevisiae
<400> 9
<210> 10
   <211> 35
   <212> DNA
   <213> Mortierella alpina
<400> 10
   catctcatgg atccgccatg gccgccgcaa tcttg 35
<210> 11
   <211> 587
   <212> DNA
   <213> Saccharomyces cerevisiae
<400> 11
   tctcgaccca gagagaggtc gccatgtgga ctatcactta cttcgtcgtc atctttggtg      60
   gtcgccagat catgaagagc caggacgcct tcaagctcaa gcccctcttc atcctccaca     120
   acttcctcct gacgatcgcg tccggatcgc tgttgctcct gttcatcgag aacctggtcc     180
   ccatcctcgc cagaaacgga cttttctacg ccatctgcga cgacggtgcc tggacccagc     240
   gcctcgagct cctctactac ctcaactacc tggtcaagta ctgggagttg gccgacaccg     300
   tctttttggt cctcaagaag aagcctcttg agttcctgca ctacttccac cactcgatga     360
   ccatggttct ctgctttgtc cagcttggag gatacacttc agtgtcctgg gtccctatta     420
   ccctcaactt gactgtccac gtcttcatgt actactacta catgcgctcc gctgccggtg     480
   ttcgcatctg gtggaagcag tacttgacca ctctccagat cgtccagttc gttcttgacc     540
   tcggattcat ctacttctgc gcctacacct acttcgcctt cacctac                   587
<210> 12
   <211> 590
   <212> DNA
   <213> Saccharomyces cerevisiae
<400> 12
   cattaagcac tttgccccct gtgctatacg ccatcactgc ctattacgtt attatttttg       60
   gtggcaggtt tttgttaagt aagtcgaaac catttaaatt aaatggcctt ttccaattgc     120
   ataatttggt tttaacttca ctttcattga cgcttttatt gcttatggtt gaacaattag     180
   tgccaattat tgttcagcac gggttatact tcgctatctg taatattggt gcttggactc     240
   aaccgctcgt tacattatat tacatgaatt acattgtcaa gtttattgaa tttatagaca     300
   cctttttctt ggtgctaaaa cataaaaaat tgacattttt gcatacttat caccatggcg     360
   ctactgcctt attatgttac acccaattga tgggcaccac atctatttct tgggtcccta      420
   tttcattgaa ccttggtgtt cacgtggtta tgtattggta ctatttcttg gctgccagag     480
   gcatcagggt ctggtggaag gaatgggtta ccagatttca aattatccaa tttgttttgg     540
   atatcggttt catatatttt gctgtctacc aaaaagcagt tcacttgtat                590
<210> 13
   <211> 278
   <212> PRT
   <213> Caenorhabditis elegans
<400> 13
   Arg Thr Phe Lys Met Met Asp Gln Ile Leu Gly Thr Asn Phe Thr Tyr
   1 5 10 15
   Glu Gly Ala Lys Glu Val Ala Arg Gly Leu Glu Gly Phe Ser Ala Lys
   20 25 30
   Leu Ala Val Gly Tyr Ile Ala Thr Ile Phe Gly Leu Lys Tyr Tyr Met
   35 40 45
   Lys Asp Arg Lys Ala Phe Asp Leu Ser Thr Pro Leu Asn Ile Trp Asn
   50 55 60
   Gly Ile Leu Ser Thr Phe Ser Leu Leu Gly Phe Leu Phe Thr Phe Pro
   65 70 75 80
   Thr Leu Leu Ser Val Ile Arg Lys Asp Gly Phe Ser His Thr Tyr Ser
   85 90 95
   His Val Ser Glu Leu Tyr Thr Asp Ser Thr Ser Gly Tyr Trp Ile Phe
   100 105 110
   Leu Trp Val Ile Ser Lys Ile Pro Glu Leu Leu Asp Thr Val Phe Ile
   115 120 125
   Val Leu Arg Lys Arg Pro Leu Ile Phe Met His Trp Tyr His His Ala
   130 135 140
   Leu Thr Gly Tyr Tyr Ala Leu Val Cys Tyr His Glu Asp Ala Val His
   145 150 155 160
   Met Val Trp Val Val Trp Met Asn Tyr Ile Ile His Ala Phe Met Tyr
   165 170 175
   Gly Tyr Tyr Leu Leu Lys Ser Leu Lys Val Pro Ile Pro Pro Ser Val
   180 185 190
   Ala Gln Ala Ile Thr Thr Ser Gln Met Val Gln Phe Ala Val Ala Ile
   195 200 205
   Phe Ala Gln Val His Val Ser Tyr Lys His Tyr Val Glu Gly Val Glu
   210 215 220
   Gly Leu Ala Tyr Ser Phe Arg Gly Thr Ala Ile Gly Phe Phe Met Leu
   225 230 235 240
   Thr Thr Tyr Phe Tyr Leu Trp Ile Gln Phe Tyr Lys Glu His Tyr Leu
   245 250 255
   Lys Asn Gly Gly Lys Lys Tyr Asn Leu Ala Lys Asp Gln Ala Lys Thr
   260 265 270
   Gln Thr Lys Lys Ala Asn
   275
<210> 14
   <211> 293
   <212> PRT
   <213> Artificial Sequence
<220>
   <223> Position 293 is artificial or unknown
<400> 14
   Ala Gln Ala Tyr Glu Leu Val Thr Gly Lys Ser Ile Asp Ser Phe Val
    1               5                  10                  15
   Phe Gln Glu Gly Val Thr Pro Leu Ser Thr Gln Arg Glu Val Ala Met
               20                  25                  30
   Trp Thr Ile Thr Tyr Phe Val Val Ile Phe Gly Gly Arg Gln Ile Met
           35                  40                  45
   Lys Ser Gln Asp Ala Phe Lys Leu Lys Pro Leu Phe Ile Leu His Asn
       50                  55                  60
   Phe Leu Leu Thr Ile Ala Ser Gly Ser Leu Leu Leu Leu Phe Ile Glu
   65                  70                  75                  80
   Asn Leu Val Pro Ile Leu Ala Arg Asn Gly Leu Phe Tyr Ala Ile Cys
                   85                  90                  95
   Asp Asp Gly Ala Trp Thr Gln Arg Leu Glu Leu Leu Tyr Tyr Leu Asn
               100                 105                 110
   Tyr Leu Val Lys Tyr Trp Glu Leu Ala Asp Thr Val Phe Leu Val Leu
           115                 120                 125
   Lys Lys Lys Pro Leu Glu Phe Leu His Tyr Phe His His Ser Met Thr
       130                 135                 140
   Met Val Leu Cys Phe Val Gln Leu Gly Gly Tyr Thr Ser Val Ser Trp
   145                 150                 155                 160
   Val Pro Ile Thr Leu Asn Leu Thr Val His Val Phe Met Tyr Tyr Tyr
                   165                 170                 175
   Tyr Met Arg Ser Ala Ala Gly Val Arg Ile Trp Trp Lys Gln Tyr Leu
               180                 185                 190
   Thr Thr Leu Gln Ile Val Gln Phe Val Leu Asp Leu Gly Phe Ile Tyr
           195                 200                 205
   Phe Cys Ala Tyr Thr Tyr Phe Ala Phe Thr Tyr Phe Pro Trp Ala Pro
       210                 215                 220
   Asn Val Gly Lys Cys Ala Gly Thr Glu Gly Ala Ala Leu Phe Gly Cys
   225                 230                 235                 240
   Gly Leu Leu Ser Ser Tyr Leu Leu Leu Phe Ile Asn Phe Tyr Arg Ile
                   245                 250                 255
   Thr Tyr Asn Ala Lys Ala Lys Ala Ala Lys Glu Arg Gly Ser Asn Phe
               260                 265                 270
   Thr Pro Lys Thr Val Lys Ser Gly Gly Ser Pro Lys Lys Pro Ser Lys
           275                 280                 285
   Ser Lys His Ile Xaa
       290
<210> 15
   <211> 238
   <212> PRT
   <213> Caenorhabditis elegans
<400> 15
   Ser Leu Leu Thr Asn Gln Asp Glu Val Phe Pro His Ile Arg Ala Arg
    1               5                  10                  15
   Arg Phe Ile Gln Glu His Phe Gly Leu Phe Val Gln Met Ala Ile Ala
               20                  25                  30
   Tyr Val Ile Leu Val Phe Ser Ile Lys Arg Phe Met Arg Asp Arg Glu
           35                  40                  45
   Pro Phe Gln Leu Thr Thr Ala Leu Arg Leu Trp Asn Phe Phe Leu Ser
       50                  55                  60
   Val Phe Ser Ile Tyr Gly Ser Trp Thr Met Phe Pro Phe Met Val Gln
   65                  70                  75                  80
   Gln Ile Arg Leu Tyr Gly Leu Tyr Gly Cys Gly Cys Glu Ala Leu Ser
                   85                  90                  95
   Asn Leu Pro Ser Gln Ala Glu Tyr Trp Leu Phe Leu Thr Ile Leu Ser
               100                 105                 110
   Lys Ala Val Glu Phe Val Asp Thr Phe Phe Leu Val Leu Arg Lys Lys
           115                 120                 125
   Pro Leu Ile Phe Leu His Trp Tyr His His Met Ala Thr Phe Val Phe
       130                 135                 140
   Phe Cys Ser Asn Tyr Pro Thr Pro Ser Ser Gln Ser Arg Val Gly Val
   145                 150                 155                 160
   Ile Val Asn Leu Phe Val His Ala Phe Met Tyr Pro Tyr Tyr Phe Thr
                   165                 170                 175
   Arg Ser Met Asn Ile Lys Val Pro Ala Lys Ile Ser Met Ala Val Thr
               180                 185                 190
   Val Leu Gln Leu Thr Gln Phe Met Cys Phe Ile Tyr Gly Cys Thr Leu
           195                 200                 205
   Met Tyr Tyr Ser Leu Ala Thr Asn Gln Ala Arg Tyr Pro Ser Asn Thr
       210                 215                 220
   Pro Ala Thr Leu Gln Cys Leu Ser Tyr Thr Leu His Leu Leu
   225                 230                 235
<210> 16
   <211> 293
   <212> PRT
   <213> Artificial Sequence
<220>
   <223> Position 293 is Artificial or unknown
<400> 16
   Glu Leu Val Thr Gly Lys Ser Ile Asp Ser Phe Val Phe Gln Glu Gly
   1               5                   10                  15
   Val Thr Pro Leu Ser Thr Gln Arg Glu Val Ala Met Trp Thr Ile Thr
               20                  25                  30
   Tyr Phe Val Val Ile Phe Gly Gly Arg Gln Ile Met Lys Ser Gln Asp
           35                  40                  45
   Ala Phe Lys Leu Lys Pro Leu Phe Ile Leu His Asn Phe Leu Leu Thr
       50                  55                  60
   Ile Ala Ser Gly Ser Leu Leu Leu Leu Phe Ile Glu Asn Leu Val Pro
   65                  70                  75                  80
   Ile Leu Ala Arg Asn Gly Leu Phe Tyr Ala Ile Cys Asp Asp Gly Ala
                   85                  90                  95
   Trp Thr Gln Arg Leu Glu Leu Leu Tyr Tyr Leu Asn Tyr Leu Val Lys
               100                 105                 110
   Tyr Trp Glu Leu Ala Asp Thr Val Phe Leu Val Leu Lys Lys Lys Pro
           115                 120                 125
   Leu Glu Phe Leu His Tyr Phe His His Ser Met Thr Met Val Leu Cys
       130                 135                 140
   Phe Val Gln Leu Gly Gly Tyr Thr Ser Val Ser Trp Val Pro Ile Thr
   145                 150                 155                 160
   Leu Asn Leu Thr Val His Val Phe Met Tyr Tyr Tyr Tyr Met Arg Ser
                   165                 170                 175
   Ala Met Ala Glu Leu Ala Gly Val Arg Ile Trp Trp Lys Gln Tyr Leu
               180                 185                 190
   Thr Thr Leu Gln Ile Val Gln Phe Val Leu Asp Leu Gly Phe Ile Tyr
           195                 200                 205
   Phe Cys Ala Tyr Thr Tyr Phe Ala Phe Thr Tyr Phe Pro Trp Ala Pro
       210                 215                 220
   Asn Val Gly Lys Cys Ala Gly Thr Glu Gly Ala Ala Leu Phe Gly Cys
   225                 230                 235                 240
   Gly Leu Leu Ser Ser Tyr Leu Leu Leu Phe Ile Asn Phe Tyr Arg Ile
                   245                 250                 255
   Thr Tyr Asn Ala Lys Ala Lys Ala Ala Lys Glu Arg Gly Ser Asn Phe
               260                 265                 270
   Thr Pro Lys Thr Val Lys Ser Gly Gly Ser Pro Lys Lys Pro Ser Lys
           275                 280                 285
   Ser Lys His Ile Xaa
       290
<210> 17
   <211> 101
   <212> PRT
   <213> Caenorhabditis elegans
<400> 17
   Met Leu Tyr Ser Ile Thr Arg Arg Cys Tyr Thr Phe Phe Val Thr Ser
    1               5                  10                  15
   Leu His Phe Tyr Gln Leu Tyr Val Thr Glu Cys Leu Glu Asn Val Ile
               20                  25                  30
   Phe Asn Val Leu Val Asn Gly Gln Ser Ile Asn Ser Arg Trp Lys Asp
           35                  40                  45
   Ala Glu Lys Thr Ile Thr Ser Phe Pro Phe His Phe Pro Gln Thr Phe
       50                  55                  60
   Phe Gln Gln Pro His Ile Leu Thr Leu His Phe Leu Phe Phe Val Phe
   65                  70                  75                  80
   Val Ser Val Thr Leu Val Thr Val Phe Lys Lys Pro Lys Cys Glu Phe
                   85                  90                  95
   Pro His Ser Leu Ala
               100
<210> 18
   <211> 115
   <212> PRT
   <213> Caenorhabditis elegans
<400> 18
   Met Ala Ala Ala Ile Leu Asp Lys Val Asn Phe Gly Ile Asp Gln Pro
    1               5                  10                  15
   Phe Gly Ile Lys Leu Asp Thr Tyr Phe Ala Gln Ala Tyr Glu Leu Val
               20                  25                  30
   Thr Gly Lys Ser Ile Asp Ser Phe Val Phe Gln Glu Gly Val Thr Pro
           35                  40                  45
   Leu Ser Thr Gln Arg Glu Val Ala Met Trp Thr Ile Thr Tyr Phe Val
       50                  55                  60
   Val Ile Phe Gly Gly Arg Gln Ile Met Lys Ser Gln Asp Ala Phe Lys
   65                  70                  75                  80
   Leu Lys Pro Leu Phe Ile Leu His Asn Phe Leu Leu Thr Ile Ala Ser
                   85                  90                  95
   Gly Ser Leu Leu Leu Leu Phe Ile Glu Asn Leu Val Pro Ile Leu Ala
               100                 105                 110
   Arg Asn Gly
           115
<210> 19
   <211> 272
   <212> PRT
   <213> Artificial Sequence
<220>
   <223> Position number 272 is artificial or unknown
<400> 19
   Met Asp Thr Ser Met Asn Phe Ser Arg Gly Leu Lys Met Asp Leu Met
    1              5                   10                  15
   Gln Pro Tyr Asp Phe Glu Thr Phe Gln Asp Leu Arg Pro Phe Leu Glu
               20                  25                  30
   Glu Tyr Trp Val Ser Ser Phe Leu Ile Val Val Val Tyr Leu Leu Leu
           35                  40                  45
   Ile Val Val Gly Gln Thr Tyr Met Arg Thr Arg Lys Ser Phe Ser Leu
       50                  55                  60
   Gln Arg Pro Leu Ile Leu Trp Ser Phe Phe Leu Ala Ile Phe Ser Ile
   65                  70                  75                  80
   Leu Gly Thr Leu Arg Met Trp Lys Phe Met Ala Thr Val Met Phe Thr
                   85                  90                  95
   Val Gly Leu Lys Gln Thr Val Cys Phe Ala Ile Tyr Thr Asp Asp Ala
               100                 105                 110
   Val Val Arg Phe Trp Ser Phe Leu Phe Leu Leu Ser Lys Val Val Glu
           115                 120                 125
   Leu Gly Asp Thr Ala Phe Ile Ile Leu Arg Lys Arg Pro Leu Ile Phe
       130                 135                 140
   Val His Trp Tyr His His Ser Thr Val Leu Leu Phe Thr Ser Phe Gly
   145                 150                 155                 160
   Tyr Lys Asn Lys Val Pro Ser Gly Gly Trp Phe Met Thr Met Asn Phe
                   165                 170                 175
   Gly Val His Ser Val Met Tyr Thr Tyr Tyr Thr Met Lys Ala Ala Lys
               180                 185                 190
   Leu Lys His Pro Asn Leu Leu Pro Met Val Ile Thr Ser Leu Gln Ile
           195                 200                 205
   Leu Gln Met Val Leu Gly Thr Ile Phe Gly Ile Leu Asn Tyr Ile Trp
       210                 215                 220
   Arg Gln Glu Lys Gly Cys His Thr Thr Thr Glu His Phe Phe Trp Ser
   225                 230                 235                 240
   Phe Met Leu Tyr Gly Thr Tyr Phe Ile Leu Phe Ala His Phe Phe His
                   245                 250                 255
   Arg Ala Tyr Leu Arg Pro Lys Gly Lys Val Ala Ser Lys Ser Gln Xaa
               260                 265                 270
<210> 20
   <211> 318
   <212> PRT
   <213> Artificial Sequence
<220>
   <223> Position Number 318 is artificial or unknown
<400> 20
   Met Ala Ala Ala Ile Leu Asp Lys Val Asn Phe Gly Ile Asp Gln Pro
    1               5                  10                  15
   Phe Gly Ile Lys Leu Asp Thr Tyr Phe Ala Gln Ala Tyr Glu Leu Val
               20                  25                  30
   Thr Gly Lys Ser Ile Asp Ser Phe Val Phe Gln Glu Gly Val Thr Pro
           35                  40                  45
   Leu Ser Thr Gln Arg Glu Val Ala Met Trp Thr Ile Thr Tyr Phe Val
       50                  55                  60
   Val Ile Phe Gly Gly Arg Gln Ile Met Lys Ser Gln Asp Ala Phe Lys
   65                  70                  75                  80
   Leu Lys Pro Leu Phe Ile Leu His Asn Phe Leu Leu Thr Ile Ala Ser
                   85                  90                  95
   Gly Ser Leu Leu Leu Leu Phe Ile Glu Asn Leu Val Pro Ile Leu Ala
               100                 105                 110
   Arg Asn Gly Leu Phe Tyr Ala Ile Cys Asp Asp Gly Ala Trp Thr Gln
           115                 120                 125
   Arg Leu Glu Leu Leu Tyr Tyr Leu Asn Tyr Leu Val Lys Tyr Trp Glu
       130                 135                 140
   Leu Ala Asp Thr Val Phe Leu Val Leu Lys Lys Lys Pro Leu Glu Phe
   145                 150                 155                 160
   Leu His Tyr Phe His His Ser Met Thr Met Val Leu Cys Phe Val Gln
                   165                 170                 175
   Leu Gly Gly Tyr Thr Ser Val Ser Trp Val Pro Ile Thr Leu Asn Leu
               180                 185                 190
   Thr Val His Val Phe Met Tyr Tyr Tyr Tyr Met Arg Ser Ala Ala Gly
           195                 200                 205
   Val Arg Ile Trp Trp Lys Gln Tyr Leu Thr Thr Leu Gln Ile Val Gln
       210                 215                 220
   Phe Val Leu Asp Leu Gly Phe Ile Tyr Phe Cys Ala Tyr Thr Tyr Phe
   225                 230                 235                 240
   Ala Phe Thr Tyr Phe Pro Trp Ala Pro Asn Val Gly Lys Cys Ala Gly
                   245                 250                 255
   Thr Glu Gly Ala Ala Leu Phe Gly Cys Gly Leu Leu Ser Ser Tyr Leu
               260                 265                 270
   Leu Leu Phe Ile Asn Phe Tyr Arg Ile Thr Tyr Asn Ala Lys Ala Lys
           275                 280                 285
   Ala Ala Lys Glu Arg Gly Ser Asn Phe Thr Pro Lys Thr Val Lys Ser
       290                 295                 300
   Gly Gly Ser Pro Lys Lys Pro Ser Lys Ser Lys His Ile Xaa
   305                 310                 315
<210> 21
   <211> 178
   <212> PRT
   <213> Homo sapiens
<400> 21
   Asn Leu Val Pro Ile Leu Ala Arg Asn Gly Leu Phe Tyr Ala Ile Cys
    1               5                  10                  15
   Asp Asp Gly Ala Trp Thr Gln Arg Leu Glu Leu Leu Tyr Tyr Leu Asn
               20                  25                  30
   Tyr Leu Val Lys Tyr Trp Glu Leu Ala Asp Thr Val Phe Leu Val Leu
           35                  40                  45
   Lys Lys Lys Pro Leu Glu Phe Leu His Tyr Phe His His Ser Met Thr
       50                  55                  60
   Met Val Leu Cys Phe Val Gln Leu Gly Gly Tyr Thr Ser Val Ser Trp
   65                  70                  75                  80
   Val Pro Ile Thr Leu Asn Leu Thr Val His Val Phe Met Tyr Tyr Tyr
                   85                  90                  95
   Tyr Met Arg Ser Ala Ala Gly Val Arg Ile Trp Trp Lys Gln Tyr Leu
               100                 105                 110
   Thr Thr Leu Gln Ile Val Gln Phe Val Leu Asp Leu Gly Phe Ile Tyr
           115                 120                 125
   Phe Cys Ala Tyr Thr Tyr Phe Ala Phe Thr Tyr Phe Pro Trp Ala Pro
       130                 135                 140
   Asn Val Gly Lys Cys Ala Gly Thr Glu Gly Ala Ala Leu Phe Gly Cys
   145                 150                 155                 160
   Gly Leu Leu Ser Ser Tyr Leu Leu Leu Phe Ile Asn Phe Tyr Arg Ile
                   165                 170                 175
   Thr Tyr
<210> 22
   <211> 147
   <212> PRT
   <213> Homo sapiens
<400> 22
   Ser Leu Leu Val Val Lys Asp Leu Thr Tyr Leu Leu Pro Leu Cys Leu
    1               5                  10                  15
   Pro Gly Asp Thr Ile Phe Ile Ile Leu Arg Lys Gln Lys Leu Ile Phe
               20                  25                  30
   Leu His Trp Tyr His His Ile Thr Val Leu Leu Tyr Ser Trp Tyr Ser
           35                  40                  45
   Tyr Lys Asp Met Val Ala Gly Gly Gly Trp Phe Met Thr Met Asn Tyr
       50                  55                  60
   Gly Val His Ala Val Met Tyr Ser Tyr Tyr Ala Leu Arg Ala Ala Gly
   65                  70                  75                  80
   Phe Arg Val Ser Arg Lys Phe Ala Met Phe Ile Thr Leu Ser Gln Ile
                   85                  90                  95
   Thr Gln Met Leu Met Gly Cys Val Val Asn Tyr Leu Val Phe Cys Trp
               100                 105                 110
   Met Gln His Asp Gln Cys His Ser His Phe Gln Asn Ile Phe Trp Ser
           115                 120                 125
   Ser Leu Met Tyr Leu Ser Tyr Leu Val Leu Phe Cys His Phe Phe Phe
       130                 135                 140
   Glu Ala Tyr
   145
<210> 23
   <211> 280
   <212> PRT
   <213> Artificial Sequence
<220>
   <223> Position 280 is artificial or unknown
<400> 23
   Ser Phe Val Phe Gln Glu Gly Val Thr Pro Leu Ser Thr Gln Arg Glu
    1               5                  10                  15
   Val Ala Met Trp Thr Ile Thr Tyr Phe Val Val Ile Phe Gly Gly Arg
               20                  25                  30
   Gln Ile Met Lys Ser Gln Asp Ala Phe Lys Leu Lys Pro Leu Phe Ile
           35                  40                  45
   Leu His Asn Phe Leu Leu Thr Ile Ala Ser Gly Ser Leu Leu Leu Leu
       50                  55                  60
   Phe Ile Glu Asn Leu Val Pro Ile Leu Ala Arg Asn Gly Leu Phe Tyr
   65                  70                  75                  80
   Ala Ile Cys Asp Asp Gly Ala Trp Thr Gln Arg Leu Glu Leu Leu Tyr
                   85                  90                  95
   Tyr Leu Asn Tyr Leu Val Lys Tyr Trp Glu Leu Ala Asp Thr Val Phe
               100                 105                 110
   Leu Val Leu Lys Lys Lys Pro Leu Glu Phe Leu His Tyr Phe His His
           115                 120                 125
   Ser Met Thr Met Val Leu Cys Phe Val Gln Leu Gly Gly Tyr Thr Ser
       130                 135                 140
   Val Ser Trp Val Pro Ile Thr Leu Asn Leu Thr Val His Val Phe Met
   145                 150                 155                 160
   Tyr Tyr Tyr Tyr Met Arg Ser Ala Ala Gly Val Arg Ile Trp Trp Lys
                   165                 170                 175
   Gln Tyr Leu Thr Thr Leu Gln Ile Val Gln Phe Val Leu Asp Leu Gly
               180                 185                 190
   Phe Ile Tyr Phe Cys Ala Tyr Thr Tyr Phe Ala Phe Thr Tyr Phe Pro
           195                 200                 205
   Trp Ala Pro Asn Val Gly Lys Cys Ala Gly Thr Glu Gly Ala Ala Leu
       210                 215                 220
   Phe Gly Cys Gly Leu Leu Ser Ser Tyr Leu Leu Leu Phe Ile Asn Phe
   225                 230                 235                 240
   Tyr Arg Ile Thr Tyr Asn Ala Lys Ala Lys Ala Ala Lys Glu Arg Gly
                   245                 250                 255
   Ser Asn Phe Thr Pro Lys Thr Val Lys Ser Gly Gly Ser Pro Lys Lys
               260                 265                 270
   Pro Ser Lys Ser Lys His Ile Xaa
           275                 280
<210> 24
   <211> 283
   <212> PRT
   <213> Artificial Sequence
<220>
   <223> Position 282 is artificial or unknown
<400> 24
   Pro Arg Tyr Lys Ser Gln Arg Met Val Pro Pro Gly Gln Leu His Pro
   1 5 10 15
   Tyr Val Cys Leu Phe Cys Tyr Leu Leu Thr His Cys Met Ala Gly Thr
   20 25 30
   Lys Ile His Glu Glu Pro Ala Ala Val Leu Leu Pro Ser Ile Leu Gln
   35 40 45
   Leu Tyr Asn Leu Gly Leu Thr Leu Leu Ser Leu Tyr Met Phe Tyr Glu
   50 55 60
   Leu Val Thr Gly Val Trp Glu Gly Lys Tyr Asn Phe Phe Cys Gln Gly
   65 70 75 80
   Thr Arg Ser Ala Gly Glu Ser Asp Met Lys Ile Ile Arg Val Leu Trp
   85 90 95
   Trp Tyr Tyr Phe Ser Lys Leu Ile Glu Phe Met Asp Thr Phe Phe Phe
   100 105 110
   Ile Leu Arg Lys Asn Asn His Gln Ile Thr Val Leu His Val Tyr His
   115 120 125
   His Ala Thr Met Leu Asn Ile Trp Trp Phe Val Met Asn Trp Val Pro
   130 135 140
   Cys Gly His Ser Tyr Phe Gly Ala Thr Leu Asn Ser Phe Ile His Val
   145 150 155 160
   Leu Met Tyr Ser Tyr Tyr Gly Leu Ser Ser Ile Pro Ser Met Arg Pro
   165 170 175
   Tyr Leu Trp Trp Lys Lys Tyr Ile Thr Gln Gly Gln Leu Val Gln Phe
   180 185 190
   Val Leu Thr Ile Ile Gln Thr Thr Cys Gly Val Phe Trp Pro Cys Ser
   195 200 205
   Phe Pro Leu Gly Trp Leu Phe Phe Gln Ile Gly Tyr Met Ile Ser Leu
   210 215 220
   Ile Ala Leu Phe Thr Asn Phe Tyr Ile Gln Thr Tyr Asn Lys Lys Gly
   225 230 235 240
   Ala Ser Arg Arg Lys Glu His Leu Lys Gly His Gln Asn Gly Ser Val
   245 250 255
   Ala Ala Val Asn Gly His Thr Asn Ser Phe Pro Ser Leu Glu Asn Ser
   260 265 270
   Val Lys Pro Arg Lys Gln Arg Lys Asp Xaa Gln
   275 280
<210> 25
   <211> 446
   <212> PRT
   <213> Mortierella alpina
<400> 25
   Met Gly Thr Asp Gln Gly Lys Thr Phe Thr Trp Glu Glu Leu Ala Ala
   1 5 10 15
   His Asn Thr Lys Asp Asp Leu Leu Leu Ala Ile Arg Gly Arg Val Tyr
                   20                  25                  30
   Asp Val Thr Lys Phe Leu Ser Arg His Pro Gly Gly Val Asp Thr Leu
               35                  40                  45
   Leu Leu Gly Ala Gly Arg Asp Val Thr Pro Val Phe Glu Met Tyr His
           50                  55                  60
   Ala Phe Gly Ala Ala Asp Ala Ile Met Lys Lys Tyr Tyr Val Gly Thr
   65                  70                  75                  80
   Leu Val Ser Asn Glu Leu Pro Ile Phe Pro Glu Pro Thr Val Phe His
                   85                  90                  95
   Lys Thr Ile Lys Thr Arg Val Glu Gly Tyr Phe Thr Asp Arg Asn Ile
               100                 105                 110
   Asp Pro Lys Asn Arg Pro Glu Ile Trp Gly Arg Tyr Ala Leu Ile Phe
           115                 120                 125
   Gly Ser Leu Ile Ala Ser Tyr Tyr Ala Gln Leu Phe Val Pro Phe Val
       130                 135                 140
   Val Glu Arg Thr Trp Leu Gln Val Val Phe Ala Ile Ile Met Gly Phe
   145                 150                 155                 160
   Ala Cys Ala Gln Val Gly Leu Asn Pro Leu His Asp Ala Ser His Phe
                   165                 170                 175
   Ser Val Thr His Asn Pro Thr Val Trp Lys Ile Leu Gly Ala Thr His
               180                 185                 190
   Asp Phe Phe Asn Gly Ala Ser Tyr Leu Val Trp Met Tyr Gln His Met
           195                 200                 205
   Leu Gly His His Pro Tyr Thr Asn Ile Ala Gly Ala Asp Pro Asp Val
       210                 215                 220
   Ser Thr Ser Glu Pro Asp Val Arg Arg Ile Lys Pro Asn Gln Lys Trp
   225                 230                 235                 240
   Phe Val Asn His Ile Asn Gln His Met Phe Val Pro Phe Leu Tyr Gly
                   245                 250                 255
   Leu Leu Ala Phe Lys Val Arg Ile Gln Asp Ile Asn Ile Leu Tyr Phe
               260                 265                 270
   Val Lys Thr Asn Asp Ala Ile Arg Val Asn Pro Ile Ser Thr Trp His
           275                 280                 285
   Thr Val Met Phe Trp Gly Gly Lys Ala Phe Phe Val Trp Tyr Arg Leu
       290                 295                 300
   Ile Val Pro Leu Gln Tyr Leu Pro Leu Gly Lys Val Leu Leu Leu Phe
   305                 310                 315                 320
   Thr Val Ala Asp Met Val Ser Ser Tyr Trp Leu Ala Leu Thr Phe Gln
                   325                 330                 335
   Ala Asn His Val Val Glu Glu Val Gln Trp Pro Leu Pro Asp Glu Asn
               340                 345                 350
   Gly Ile Ile Gln Lys Asp Trp Ala Ala Met Gln Val Glu Thr Thr Gln
           355                 360                 365
   Asp Tyr Ala His Asp Ser His Leu Trp Thr Ser Ile Thr Gly Ser Leu
       370                 375                 380
   Asn Tyr Gln Ala Val His His Leu Phe Pro Asn Val Ser Gln His His
   385                 390                 395                 400
   Tyr Pro Asp Ile Leu Ala Ile Ile Lys Asn Thr Cys Ser Glu Tyr Lys
                   405                 410                 415
   Val Pro Tyr Leu Val Lys Asp Thr Phe Trp Gln Ala Phe Ala Ser His
               420                 425                 430
   Leu Glu His Leu Arg Val Leu Gly Leu Arg Pro Lys Glu Glu
           435                 440                 445
<210> 26
   <211> 318
   <212> PRT
   <213> Mortierella alpina
<400> 26
   Met Glu Ser Ile Ala Pro Phe Leu Pro Ser Lys Met Pro Gln Asp Leu
   1               5                   10                  15
   Phe Met Asp Leu Ala Thr Ala Ile Gly Val Arg Ala Ala Pro Tyr Val
               20                  25                  30
   Asp Pro Leu Glu Ala Ala Leu Val Ala Gln Ala Glu Lys Tyr Ile Pro
           35                  40                  45
   Thr Ile Val His His Thr Arg Gly Phe Leu Val Ala Val Glu Ser Pro
       50                  55                  60
   Leu Ala Arg Glu Leu Pro Leu Met Asn Pro Phe His Val Leu Leu Ile
   65                  70                  75                  80
   Val Leu Ala Tyr Leu Val Thr Val Phe Val Gly Met Gln Ile Met Lys
                   85                  90                  95
   Asn Phe Glu Arg Phe Glu Val Lys Thr Phe Ser Leu Leu His Asn Phe
               100                 105                 110
   Cys Leu Val Ser Ile Ser Ala Tyr Met Cys Gly Gly Ile Leu Tyr Glu
           115                 120                 125
   Ala Tyr Gln Ala Asn Tyr Gly Leu Phe Glu Asn Ala Ala Asp His Thr
       130                 135                 140
   Phe Lys Gly Leu Pro Met Ala Lys Met Ile Trp Leu Phe Tyr Phe Ser
   145                 150                 155                 160
   Lys Ile Met Glu Phe Val Asp Thr Met Ile Met Val Leu Lys Lys Asn
                   165                 170                 175
   Asn Arg Gln Ile Ser Phe Leu His Val Tyr His His Ser Ser Ile Phe
               180                 185                 190
   Thr Ile Trp Trp Leu Val Thr Phe Val Ala Pro Asn Gly Glu Ala Tyr
           195                 200                 205
   Phe Ser Ala Ala Leu Asn Ser Phe Ile His Val Ile Met Tyr Gly Tyr
       210                 215                 220
   Tyr Phe Leu Ser Ala Leu Gly Phe Lys Gln Val Ser Phe Ile Lys Phe
   225                 230                 235                 240
   Tyr Ile Thr Arg Ser Gln Met Thr Gln Phe Cys Met Met Ser Val Gln
                   245                 250                 255
   Ser Ser Trp Asp Met Tyr Ala Met Lys Val Leu Gly Arg Pro Gly Tyr
               260                 265                 270
   Pro Phe Phe Ile Thr Ala Leu Leu Trp Phe Tyr Met Trp Thr Met Leu
           275                 280                 285
   Gly Leu Phe Tyr Asn Phe Tyr Arg Lys Asn Ala Lys Leu Ala Lys Gln
       290                 295                 300
   Ala Lys Ala Asp Ala Ala Lys Glu Lys Ala Arg Lys Leu Gln
   305                 310                 315
<210> 27
   <211> 279
   <212> PRT
   <213> Mortierella alpina
<400> 27
   Val Ala Gln Ala Glu Lys Tyr Ile Pro Thr Ile Val His His Thr Arg
    1               5                  10                  15
   Gly Phe Leu Val Ala Val Glu Ser Pro Leu Ala Arg Glu Leu Pro Leu
               20                  25                  30
   Met Asn Pro Phe His Val Leu Leu Ile Val Leu Ala Tyr Leu Val Thr
           35                  40                  45
   Val Phe Val Gly Met Gln Ile Met Lys Asn Phe Glu Arg Phe Glu Val
       50                  55                  60
   Lys Thr Phe Ser Leu Leu His Asn Phe Cys Leu Val Ser Ile Ser Ala
   65                  70                  75                  80
   Tyr Met Cys Gly Gly Ile Leu Tyr Glu Ala Tyr Gln Ala Asn Tyr Gly
                   85                  90                  95
   Leu Phe Glu Asn Ala Ala Asp His Thr Phe Lys Gly Leu Pro Met Ala
               100                 105                 110
   Lys Met Ile Trp Leu Phe Tyr Phe Ser Lys Ile Met Glu Phe Val Asp
           115                 120                 125
   Thr Met Ile Met Val Leu Lys Lys Asn Asn Arg Gln Ile Ser Phe Leu
       130                 135                 140
   His Val Tyr His His Ser Ser Ile Phe Thr Ile Trp Trp Leu Val Thr
   145                 150                 155                 160
   Phe Val Ala Pro Asn Gly Glu Ala Tyr Phe Ser Ala Ala Leu Asn Ser
                   165                 170                 175
   Phe Ile His Val Ile Met Tyr Gly Tyr Tyr Phe Leu Ser Ala Leu Gly
               180                 185                 190
   Phe Lys Gln Val Ser Phe Ile Lys Phe Tyr Ile Thr Arg Ser Gln Met
           195                 200                 205
   Thr Gln Phe Cys Met Met Ser Val Gln Ser Ser Trp Asp Met Tyr Ala
       210                 215                 220
   Met Lys Val Leu Gly Arg Pro Gly Tyr Pro Phe Phe Ile Thr Ala Leu
   225                 230                 235                 240
   Leu Trp Phe Tyr Met Trp Thr Met Leu Gly Leu Phe Tyr Asn Phe Tyr
                   245                 250                 255
   Arg Lys Asn Ala Lys Leu Ala Lys Gln Ala Lys Ala Asp Ala Ala Lys
               260                 265                 270
   Glu Lys Ala Arg Lys Leu Gln
           275
<210> 28
   <211> 301
   <212> PRT
   <213> Artificial Sequence
<220>
   <223> Position number 301 is artificial or unknown
<400> 28
   Gly Ile Lys Leu Asp Thr Tyr Phe Ala Gln Ala Tyr Glu Leu Val Thr
    1               5                  10                  15
   Gly Lys Ser Ile Asp Ser Phe Val Phe Gln Glu Gly Val Thr Pro Leu
               20                  25                  30
   Ser Thr Gln Arg Glu Val Ala Met Trp Thr Ile Thr Tyr Phe Val Val
           35                  40                  45
   Ile Phe Gly Gly Arg Gln Ile Met Lys Ser Gln Asp Ala Phe Lys Leu
       50                  55                  60
   Lys Pro Leu Phe Ile Leu His Asn Phe Leu Leu Thr Ile Ala Ser Gly
   65                  70                  75                  80
   Ser Leu Leu Leu Leu Phe Ile Glu Asn Leu Val Pro Ile Leu Ala Arg
                   85                  90                  95
   Asn Gly Leu Phe Tyr Ala Ile Cys Asp Asp Gly Ala Trp Thr Gln Arg
               100                 105                 110
   Leu Glu Leu Leu Tyr Tyr Leu Asn Tyr Leu Val Lys Tyr Trp Glu Leu
           115                 120                 125
   Ala Asp Thr Val Phe Leu Val Leu Lys Lys Lys Pro Leu Glu Phe Leu
       130                 135                 140
   His Tyr Phe His His Ser Met Thr Met Val Leu Cys Phe Val Gln Leu
   145                 150                 155                 160
   Gly Gly Tyr Thr Ser Val Ser Trp Val Pro Ile Thr Leu Asn Leu Thr
                   165                 170                 175
   Val His Val Phe Met Tyr Tyr Tyr Tyr Met Arg Ser Ala Ala Gly Val
               180                 185                 190
   Arg Ile Trp Trp Lys Gln Tyr Leu Thr Thr Leu Gln Ile Val Gln Phe
           195                 200                 205
   Val Leu Asp Leu Gly Phe Ile Tyr Phe Cys Ala Tyr Thr Tyr Phe Ala
       210                 215                 220
   Phe Thr Tyr Phe Pro Trp Ala Pro Asn Val Gly Lys Cys Ala Gly Thr
   225                 230                 235                 240
   Glu Gly Ala Ala Leu Phe Gly Cys Gly Leu Leu Ser Ser Tyr Leu Leu
                   245                 250                 255
   Leu Phe Ile Asn Phe Tyr Arg Ile Thr Tyr Asn Ala Lys Ala Lys Ala
               260                 265                 270
   Ala Lys Glu Arg Gly Ser Asn Phe Thr Pro Lys Thr Val Lys Ser Gly
           275                 280                 285
   Gly Ser Pro Lys Lys Pro Ser Lys Ser Lys His Ile Xaa
       290                 295                 300
<210> 29
   <211> 289
   <212> PRT
   <213> Mortierella alpina
<400> 29
   Tyr Glu Leu Val Thr Gly Lys Ser Ile Asp Ser Phe Val Phe Gln Glu
    1               5                  10                  15
   Gly Val Thr Pro Leu Ser Thr Gln Arg Glu Val Ala Met Trp Thr Ile
               20                  25                  30
   Thr Tyr Phe Val Val Ile Phe Gly Gly Arg Gln Ile Met Lys Ser Gln
           35                  40                  45
   Asp Ala Phe Lys Leu Lys Pro Leu Phe Ile Leu His Asn Phe Leu Leu
       50                  55                  60
   Thr Ile Ala Ser Gly Ser Leu Leu Leu Leu Phe Ile Glu Asn Leu Val
   65                  70                  75                  80
   Pro Ile Leu Ala Arg Asn Gly Leu Phe Tyr Ala Ile Cys Asp Asp Gly
                   85                  90                  95
   Ala Trp Thr Gln Arg Leu Glu Leu Leu Tyr Tyr Leu Asn Tyr Leu Val
               100                 105                 110
   Lys Tyr Trp Glu Leu Ala Asp Thr Val Phe Leu Val Leu Lys Lys Lys
           115                 120                 125
   Pro Leu Glu Phe Leu His Tyr Phe His His Ser Met Thr Met Val Leu
       130                 135                 140
   Cys Phe Val Gln Leu Gly Gly Tyr Thr Ser Val Ser Trp Val Pro Ile
   145                 150                 155                 160
   Thr Leu Asn Leu Thr Val His Val Phe Met Tyr Tyr Tyr Tyr Met Arg
                   165                 170                 175
   Ser Ala Ala Gly Val Arg Ile Trp Trp Lys Gln Tyr Leu Thr Thr Leu
               180                 185                 190
   Gln Ile Val Gln Phe Val Leu Asp Leu Gly Phe Ile Tyr Phe Cys Ala
           195                 200                 205
   Tyr Thr Tyr Phe Ala Phe Thr Tyr Phe Pro Trp Ala Pro Asn Val Gly
       210                 215                 220
   Lys Cys Ala Gly Thr Glu Gly Ala Ala Leu Phe Gly Cys Gly Leu Leu
   225                 230                 235                 240
   Ser Ser Tyr Leu Leu Leu Phe Ile Asn Phe Tyr Arg Ile Thr Tyr Asn
                   245                 250                 255
   Ala Lys Ala Lys Ala Ala Lys Glu Arg Gly Ser Asn Phe Thr Pro Lys
               260                 265                 270
   Thr Val Lys Ser Gly Gly Ser Pro Lys Lys Pro Ser Lys Ser Lys His
           275                 280                 285
   Ile
<210> 30
   <211> 292
   <212> PRT
   <213> Artificial Sequence
<220>
   <223> Poisition 292 is artificial or unknown
<400> 30
   Ser Thr Tyr Phe Lys Ala Leu Leu Gly Pro Arg Asp Thr Arg Val Lys
    1               5                  10                  15
   Gly Trp Phe Leu Leu Asp Asn Tyr Ile Pro Thr Phe Ile Cys Ser Val
               20                  25                  30
   Ile Tyr Leu Leu Ile Val Trp Leu Gly Pro Lys Tyr Met Arg Asn Lys
           35                  40                  45
   Gln Pro Phe Ser Cys Arg Gly Ile Leu Val Val Tyr Asn Leu Gly Leu
       50                  55                  60
   Thr Leu Leu Ser Leu Tyr Met Phe Cys Glu Leu Val Thr Gly Val Trp
   65                  70                  75                  80
   Glu Gly Lys Tyr Asn Phe Phe Cys Gln Gly Thr Arg Thr Ala Gly Glu
                   85                  90                  95
   Ser Asp Met Lys Ile Ile Arg Val Leu Trp Trp Tyr Tyr Phe Ser Lys
               100                 105                 110
   Leu Ile Glu Phe Met Asp Thr Phe Phe Phe Ile Leu Arg Lys Asn Asn
           115                 120                 125
   His Gln Ile Thr Val Leu His Val Tyr His His Ala Ser Met Leu Asn
       130                 135                 140
   Ile Trp Trp Phe Val Met Asn Trp Val Pro Cys Gly His Ser Tyr Phe
   145                 150                 155                 160
   Gly Ala Thr Leu Asn Ser Phe Ile His Val Leu Met Tyr Ser Tyr Tyr
                   165                 170                 175
   Gly Leu Ser Ser Val Pro Ser Met Arg Pro Tyr Leu Trp Trp Lys Lys
               180                 185                 190
   Tyr Ile Thr Gln Gly Gln Leu Leu Gln Phe Val Leu Thr Ile Ile Gln
           195                 200                 205
   Thr Ser Cys Gly Val Ile Trp Pro Cys Thr Phe Pro Leu Gly Trp Leu
       210                 215                 220
   Tyr Phe Gln Ile Gly Tyr Met Ile Ser Leu Ile Ala Leu Phe Thr Asn
   225                 230                 235                 240
   Phe Tyr Ile Gln Thr Tyr Asn Lys Lys Gly Ala Ser Arg Arg Lys Asp
                   245                 250                 255
   His Leu Lys Asp His Gln Asn Gly Ser Met Ala Ala Val Asn Gly His
               260                 265                 270
   Thr Asn Ser Phe Ser Pro Leu Glu Asn Asn Val Lys Pro Arg Lys Leu
           275                 280                 285
   Arg Lys Asp Xaa
       290
<210> 31
   <211> 291
   <212> PRT
   <213> Mortierella alpina
<400> 31
   Gln Ala Tyr Glu Leu Val Thr Gly Lys Ser Ile Asp Ser Phe Val Phe
    1               5                  10                  15
   Gln Glu Gly Val Thr Pro Leu Ser Thr Gln Arg Glu Val Ala Met Trp
               20                  25                  30
   Thr Ile Thr Tyr Phe Val Val Ile Phe Gly Gly Arg Gln Ile Met Lys
           35                  40                  45
   Ser Gln Asp Ala Phe Lys Leu Lys Pro Leu Phe Ile Leu His Asn Phe
       50                  55                  60
   Leu Leu Thr Ile Ala Ser Gly Ser Leu Leu Leu Leu Phe Ile Glu Asn
   65                  70                  75                  80
   Leu Val Pro Ile Leu Ala Arg Asn Gly Leu Phe Tyr Ala Ile Cys Asp
                   85                  90                  95
   Asp Gly Ala Trp Thr Gln Arg Leu Glu Leu Leu Tyr Tyr Leu Asn Tyr
               100                 105                 110
   Leu Val Lys Tyr Trp Glu Leu Ala Asp Thr Val Phe Leu Val Leu Lys
           115                 120                 125
   Lys Lys Pro Leu Glu Phe Leu His Tyr Phe His His Ser Met Thr Met
       130                 135                 140
   Val Leu Cys Phe Val Gln Leu Gly Gly Tyr Thr Ser Val Ser Trp Val
   145                 150                 155                 160
   Pro Ile Thr Leu Asn Leu Thr Val His Val Phe Met Tyr Tyr Tyr Tyr
                   165                 170                 175
   Met Arg Ser Ala Ala Gly Val Arg Ile Trp Trp Lys Gln Tyr Leu Thr
               180                 185                 190
   Thr Leu Gln Ile Val Gln Phe Val Leu Asp Leu Gly Phe Ile Tyr Phe
           195                 200                 205
   Cys Ala Tyr Thr Tyr Phe Ala Phe Thr Tyr Phe Pro Trp Ala Pro Asn
       210                 215                 220
   Val Gly Lys Cys Ala Gly Thr Glu Gly Ala Ala Leu Phe Gly Cys Gly
   225                 230                 235                 240
   Leu Leu Ser Ser Tyr Leu Leu Leu Phe Ile Asn Phe Tyr Arg Ile Thr
                   245                 250                 255
   Tyr Asn Ala Lys Ala Lys Ala Ala Lys Glu Arg Gly Ser Asn Phe Thr
               260                 265                 270
   Pro Lys Thr Val Lys Ser Gly Gly Ser Pro Lys Lys Pro Ser Lys Ser
           275                 280                 285
   Lys His Ile
       290
<210> 32
   <211> 276
   <212> PRT
   <213> Artificial Sequence
<220>
   <223> Poisiton 276 is artificial or unknown
<400> 32
   Val Asn Leu Tyr Gln Glu Val Met Lys His Ala Asp Pro Arg Ile Gln
    1               5                  10                  15
   Gly Tyr Pro Leu Met Gly Ser Pro Leu Leu Met Thr Ser Ile Leu Leu
               20                  25                  30
   Thr Tyr Val Tyr Phe Val Leu Ser Leu Gly Pro Arg Ile Met Ala Asn
           35                  40                  45
   Arg Lys Pro Phe Gln Leu Arg Gly Phe Met Ile Val Tyr Asn Phe Ser
       50                  55                  60
   Leu Val Ala Leu Ser Leu Tyr Ile Val Tyr Glu Phe Leu Met Ser Gly
   65                  70                  75                  80
   Trp Leu Ser Thr Tyr Thr Trp Arg Cys Asp Pro Val Asp Tyr Ser Asn
                   85                  90                  95
   Ser Pro Glu Ala Leu Arg Met Val Arg Val Ala Trp Leu Phe Leu Phe
               100                 105                 110
   Ser Lys Phe Ile Glu Leu Met Asp Thr Val Ile Phe Ile Leu Arg Lys
           115                 120                 125
   Lys Asp Gly Gln Val Thr Phe Leu His Val Phe His His Ser Val Leu
       130                 135                 140
   Pro Trp Ser Trp Trp Trp Gly Val Lys Ile Ala Pro Gly Gly Met Gly
   145                 150                 155                 160
   Ser Phe His Ala Met Ile Asn Ser Ser Val His Val Ile Met Tyr Leu
                   165                 170                 175
   Tyr Tyr Gly Leu Ser Ala Phe Gly Pro Val Ala Gln Pro Tyr Leu Trp
               180                 185                 190
   Trp Lys Lys His Met Thr Ala Ile Gln Leu Ile Gln Phe Val Leu Val
           195                 200                 205
   Ser Leu His Ile Ser Gln Tyr Tyr Phe Met Ser Ser Cys Asn Tyr Gln
       210                 215                 220
   Tyr Pro Val Ile Ile His Leu Ile Trp Met Tyr Gly Thr Ile Phe Phe
   225                 230                 235                 240
   Met Leu Phe Ser Asn Phe Trp Tyr His Ser Tyr Thr Lys Gly Lys Arg
                   245                 250                 255
   Leu Pro Arg Ala Leu Gln Gln Asn Gly Ala Pro Gly Ile Ala Lys Val
               260                 265                 270
   Lys Ala Asn Xaa
           275
<210> 33
   <211> 219
   <212> PRT
   <213> Mortierella alpina
<400> 33
   Leu Leu Leu Leu Phe Ile Glu Asn Leu Val Pro Ile Leu Ala Arg Asn
    1               5                  10                  15
   Gly Leu Phe Tyr Ala Ile Cys Asp Asp Gly Ala Trp Thr Gln Arg Leu
               20                  25                  30
   Glu Leu Leu Tyr Tyr Leu Asn Tyr Leu Val Lys Tyr Trp Glu Leu Ala
           35                  40                  45
   Asp Thr Val Phe Leu Val Leu Lys Lys Lys Pro Leu Glu Phe Leu His
       50                  55                  60
   Tyr Phe His His Ser Met Thr Met Val Leu Cys Phe Val Gln Leu Gly
   65                  70                  75                  80
   Gly Tyr Thr Ser Val Ser Trp Val Pro Ile Thr Leu Asn Leu Thr Val
                   85                  90                  95
   His Val Phe Met Tyr Tyr Tyr Tyr Met Arg Ser Ala Ala Gly Val Arg
               100                 105                 110
   Ile Trp Trp Lys Gln Tyr Leu Thr Thr Leu Gln Ile Val Gln Phe Val
           115                 120                 125
   Leu Asp Leu Gly Phe Ile Tyr Phe Cys Ala Tyr Thr Tyr Phe Ala Phe
       130                 135                 140
   Thr Tyr Phe Pro Trp Ala Pro Asn Val Gly Lys Cys Ala Gly Thr Glu
   145                 150                 155                 160
   Gly Ala Ala Leu Phe Gly Cys Gly Leu Leu Ser Ser Tyr Leu Leu Leu
                   165                 170                 175
   Phe Ile Asn Phe Tyr Arg Ile Thr Tyr Asn Ala Lys Ala Lys Ala Ala
               180                 185                 190
   Lys Glu Arg Gly Ser Asn Phe Thr Pro Lys Thr Val Lys Ser Gly Gly
           195                 200                 205
   Ser Pro Lys Lys Pro Ser Lys Ser Lys His Ile
       210                 215
<210> 34
   <211> 202
   <212> PRT
   <213> Mortierella alpina
<400> 34
   Ile Val Tyr Glu Phe Leu Met Ser Gly Trp Leu Ser Thr Tyr Thr Trp
    1               5                  10                  15
   Arg Cys Asp Pro Ile Asp Phe Ser Asn Ser Pro Glu Ala Leu Arg Met
               20                  25                  30
   Val Arg Val Ala Trp Leu Phe Met Leu Ser Lys Val Ile Glu Leu Met
           35                  40                  45
   Asp Thr Val Ile Phe Ile Leu Arg Lys Lys Asp Gly Gln Val Thr Phe
       50                  55                  60
   Leu His Val Phe His His Ser Val Leu Pro Trp Ser Trp Trp Trp Gly
   65                  70                  75                  80
   Ile Lys Ile Ala Pro Gly Gly Met Gly Ser Phe His Ala Met Ile Asn
   85 90 95
   Ser Ser Val His Val Val Met Tyr Leu Tyr Tyr Gly Leu Ser Ala Leu
   100 105 110
   Gly Pro Val Ala Gln Pro Tyr Leu Trp Trp Lys Lys His Met Thr Ala
   115 120 125
   Ile Gln Leu Ile Gln Phe Val Leu Val Ser Leu His Ile Ser Gln Tyr
   130 135 140
   Tyr Phe Met Pro Ser Cys Asn Tyr Gln Tyr Pro Val Ile Ile His Leu
   145 150 155 160
   Ile Trp Met Thr Ile Phe Phe Ile Leu Phe Ser Asn Phe Trp Tyr His
   165 170 175
   Ser Tyr Thr Lys Gly Lys Arg Leu Pro Arg Ala Val Gln Gln Asn Gly
   180 185 190
   Ala Pro Ala Thr Thr Lys Val Lys Ala Asn
   195 200
<210> 35
   <211> 174
   <212> PRT
   <213> Mortierella alpina
<400> 35
   Tyr Glu Leu Val Thr Gly Lys Ser Ile Asp Ser Phe Val Phe Gln Glu
   1 5 10 15
   Gly Val Thr Pro Leu Ser Thr Gln Arg Glu Val Ala Met Trp Thr Ile
   20 25 30
   Thr Tyr Phe Val Val Ile Phe Gly Gly Arg Gln Ile Met Lys Ser Gln
   35 40 45
   Asp Ala Phe Lys Leu Lys Pro Leu Phe Ile Leu His Asn Phe Leu Leu
   50 55 60
   Thr Ile Ala Ser Gly Ser Leu Leu Leu Leu Phe Ile Glu Asn Leu Val
   65 70 75 80
   Pro Ile Leu Ala Arg Asn Gly Leu Phe Tyr Ala Ile Cys Asp Asp Gly
   85 90 95
   Ala Trp Thr Gln Arg Leu Glu Leu Leu Tyr Tyr Leu Asn Tyr Leu Val
   100 105 110
   Lys Tyr Trp Glu Leu Ala Asp Thr Val Phe Leu Val Leu Lys Lys Lys
   115 120 125
   Pro Leu Glu Phe Leu His Tyr Phe His His Ser Met Thr Met Val Leu
   130 135 140
   Cys Phe Val Gln Leu Gly Gly Tyr Thr Ser Val Ser Trp Val Pro Ile
   145 150 155 160
   Thr Leu Asn Leu Thr Val His Val Phe Met Tyr Tyr Tyr Tyr
   165 170
<210> 36
   <211> 145
   <212> PRT
   <213> Mortierella alpina
<400> 36
   Asn Ala Phe Leu Asp Asn Met Phe Gly Pro Arg Asp Ser Arg Val Arg
   1 5 10 15
   Gly Trp Phe Leu Leu Asp Ser Tyr Leu Pro Thr Phe Ile Leu Thr Ile
   20 25 30
   Thr Tyr Leu Leu Ser Ile Trp Leu Gly Asn Lys Tyr Met Lys Asn Arg
   35 40 45
   Pro Ala Leu Ser Leu Arg Gly Ile Leu Thr Leu Tyr Asn Leu Ala Ile
   50 55 60
   Thr Leu Leu Ser Ala Tyr Met Leu Val Glu Leu Ile Leu Ser Ser Trp
   65 70 75 80
   Glu Gly Gly Tyr Asn Leu Gln Cys Gln Asn Leu Asp Ser Ala Gly Glu
   85 90 95
   Gly Asp Val Arg Val Ala Lys Val Leu Val Trp Tyr Tyr Phe Ser Lys
   100 105 110
   Leu Val Glu Phe Leu Asp Thr Ile Phe Phe Val Leu Arg Lys Lys Ala
   115 120 125
   Asn Gln Ile Thr Phe Leu His Val Tyr His His Ala Ser Met Phe Asn
   130 135 140
   Ile
   145
<210> 37
   <211> 278
   <212> PRT
   <213> Caenorhabditis elegans
<400> 37
   Ala Gln Ala Glu Lys Tyr Ile Pro Thr Ile Val His His Thr Arg Gly
    1               5                  10                  15
   Phe Leu Val Ala Val Glu Ser Pro Leu Ala Arg Glu Leu Pro Leu Met
               20                  25                  30
   Asn Pro Phe His Val Leu Leu Ile Val Leu Ala Tyr Leu Val Thr Val
           35                  40                  45
   Phe Val Gly Met Gln Ile Met Lys Asn Phe Glu Arg Phe Glu Val Lys
       50                  55                  60
   Thr Phe Ser Leu Leu His Asn Phe Cys Leu Val Ser Ile Ser Ala Tyr
   65                  70                  75                  80
   Met Cys Gly Gly Ile Leu Tyr Glu Ala Tyr Gln Ala Asn Tyr Gly Leu
                   85                  90                  95
   Phe Glu Asn Ala Ala Asp His Thr Phe Lys Gly Leu Pro Met Ala Lys
               100                 105                 110
   Met Ile Trp Leu Phe Tyr Phe Ser Lys Ile Met Glu Phe Val Asp Thr
           115                 120                 125
   Met Ile Met Val Leu Lys Lys Asn Asn Arg Gln Ile Ser Phe Leu His
       130                 135                 140
   Val Tyr His His Ser Ser Ile Phe Thr Ile Trp Trp Leu Val Thr Phe
   145                 150                 155                 160
   Val Ala Pro Asn Gly Glu Ala Tyr Phe Ser Ala Ala Leu Asn Ser Phe
                   165                 170                 175
   Ile His Val Ile Met Tyr Gly Tyr Tyr Phe Leu Ser Ala Leu Gly Phe
               180                 185                 190
   Lys Gln Val Ser Phe Ile Lys Phe Tyr Ile Thr Arg Ser Gln Met Thr
           195                 200                 205
   Gln Phe Cys Met Met Ser Val Gln Ser Ser Trp Asp Met Tyr Ala Met
       210                 215                 220
   Lys Val Leu Gly Arg Pro Gly Tyr Pro Phe Phe Ile Thr Ala Leu Leu
   225                 230                 235                 240
   Trp Phe Tyr Met Trp Thr Met Leu Gly Leu Phe Tyr Asn Phe Tyr Arg
                   245                 250                 255
   Lys Asn Ala Lys Leu Ala Lys Gln Ala Lys Ala Asp Ala Ala Lys Glu
               260                 265                 270
   Lys Ala Arg Lys Leu Gln
           275
<210> 38
   <211> 293
   <212> PRT
   <213> Caenorhabditis elegans
<400> 38
   Met Glu His Phe Asp Ala Ser Leu Ser Thr Tyr Phe Lys Ala Leu Leu
    1               5                  10                  15
   Gly Pro Arg Asp Thr Arg Val Lys Gly Trp Phe Leu Leu Asp Asn Tyr
               20                  25                  30
   Ile Pro Thr Phe Ile Cys Ser Val Ile Tyr Leu Leu Ile Val Trp Leu
           35                  40                  45
   Gly Pro Lys Tyr Met Arg Asn Lys Gln Pro Phe Ser Cys Arg Gly Ile
       50                  55                  60
   Leu Val Val Tyr Asn Leu Gly Leu Thr Leu Leu Ser Leu Tyr Met Phe
   65                  70                  75                  80
   Cys Glu Leu Val Thr Gly Val Trp Glu Gly Lys Tyr Asn Phe Phe Cys
                   85                  90                  95
   Gln Gly Thr Arg Thr Ala Gly Glu Ser Asp Met Lys Ile Ile Arg Val
               100                 105                 110
   Leu Trp Trp Tyr Tyr Phe Ser Lys Leu Ile Glu Phe Met Asp Thr Phe
           115                 120                 125
   Phe Phe Ile Leu Arg Lys Asn Asn His Gln Ile Thr Val Leu His Val
       130                 135                 140
   Tyr His His Ala Ser Met Leu Asn Ile Trp Trp Phe Val Met Asn Trp
   145                 150                 155                 160
   Val Pro Cys Gly His Ser Tyr Phe Gly Ala Thr Leu Asn Ser Phe Ile
                   165                 170                 175
   His Val Leu Met Tyr Ser Tyr Tyr Gly Leu Ser Ser Val Pro Ser Met
               180                 185                 190
   Arg Pro Tyr Leu Trp Trp Lys Lys Tyr Ile Thr Gln Gly Gln Leu Leu
           195                 200                 205
   Gln Phe Val Leu Thr Ile Ile Gln Thr Ser Cys Gly Val Ile Trp Pro
       210                 215                 220
   Cys Thr Phe Pro Leu Gly Trp Leu Tyr Phe Gln Ile Gly Tyr Met Ile
   225                 230                 235                 240
   Ser Leu Ile Ala Leu Phe Thr Asn Phe Tyr Ile Gln Thr Tyr Asn Lys
                   245                 250                 255
   Lys Gly Ala Ser Arg Arg Lys Asp His Leu Lys Asp His Gln Asn Gly
               260                 265                 270
   Ser Met Ala Ala Val Asn Gly His Thr Asn Ser Phe Ser Pro Leu Glu
           275                 280                 285
   Asn Asn Val Lys Pro
       290
<210> 39
   <211> 174
   <212> PRT
   <213> Caenorhabditis elegans
<400> 39
   Tyr Glu Leu Val Thr Gly Lys Ser Ile Asp Ser Phe Val Phe Gln Glu
   1 5 10 15
   Gly Val Thr Pro Leu Ser Thr Gln Arg Glu Val Ala Met Trp Thr Ile
   20 25 30
   Thr Tyr Phe Val Val Ile Phe Gly Gly Arg Gln Ile Met Lys Ser Gln
   35 40 45
   Asp Ala Phe Lys Leu Lys Pro Leu Phe Ile Leu His Asn Phe Leu Leu
   50 55 60
   Thr Ile Ala Ser Gly Ser Leu Leu Leu Leu Phe Ile Glu Asn Leu Val
   65 70 75 80
   Pro Ile Leu Ala Arg Asn Gly Leu Phe Tyr Ala Ile Cys Asp Asp Gly
   85 90 95
   Ala Trp Thr Gln Arg Leu Glu Leu Leu Tyr Tyr Leu Asn Tyr Leu Val
   100 105 110
   Lys Tyr Trp Glu Leu Ala Asp Thr Val Phe Leu Val Leu Lys Lys Lys
   115 120 125
   Pro Leu Glu Phe Leu His Tyr Phe His His Ser Met Thr Met Val Leu
   130 135 140
   Cys Phe Val Gln Leu Gly Gly Tyr Thr Ser Val Ser Trp Val Pro Ile
   145 150 155 160
   Thr Leu Asn Leu Thr Val His Val Phe Met Tyr Tyr Tyr Tyr
   165 170
<210> 40
   <211> 141
   <212> PRT
   <213> Artificial Sequence
<220>
   <223> Poisition Number 141 is artificial or unknown
<400> 40
   Asp Thr Ile Phe Ile Ile Leu Arg Lys Gln Lys Leu Ile Phe Leu His
   1 5 10 15
   Trp Tyr His His Ile Thr Val Leu Leu Tyr Ser Trp Tyr Ser Tyr Lys
   20 25 30
   Asp Met Val Ala Gly Gly Gly Trp Phe Met Thr Met Asn Tyr Gly Val
   35 40 45
   His Ala Val Met Tyr Ser Tyr Tyr Ala Leu Arg Ala Ala Gly Phe Arg
   50 55 60
   Val Ser Arg Lys Phe Ala Met Phe Ile Thr Leu Ser Gln Ile Thr Gln
   65 70 75 80
   Met Leu Met Gly Cys Val Val Asn Tyr Leu Val Phe Cys Trp Met Gln
   85 90 95
   His Asp Gln Cys His Ser His Phe Gln Asn Ile Phe Trp Ser Ser Leu
   100 105 110
   Met Tyr Leu Ser Tyr Leu Val Leu Phe Cys His Phe Phe Phe Glu Ala
   115 120 125
   Tyr Ile Gly Lys Met Arg Lys Thr Thr Lys Ala Glu Xaa
   130 135 140
<210> 41
   <211> 238
   <212> PRT
   <213> Caenorhabditis elegans
<400> 41
   Leu Ile Val Leu Ala Tyr Leu Val Thr Val Phe Val Gly Met Gln Ile
    1               5                  10                  15
   Met Lys Asn Phe Glu Arg Phe Glu Val Lys Thr Phe Ser Leu Leu His
               20                  25                  30
   Asn Phe Cys Leu Val Ser Ile Ser Ala Tyr Met Cys Gly Gly Ile Leu
           35                  40                  45
   Tyr Glu Ala Tyr Gln Ala Asn Tyr Gly Leu Phe Glu Asn Ala Ala Asp
       50                  55                  60
   His Thr Phe Lys Gly Leu Pro Met Ala Lys Met Ile Trp Leu Phe Tyr
   65                  70                  75                  80
   Phe Ser Lys Ile Met Glu Phe Val Asp Thr Met Ile Met Val Leu Lys
                   85                  90                  95
   Lys Asn Asn Arg Gln Ile Ser Phe Leu His Val Tyr His His Ser Ser
               100                 105                 110
   Ile Phe Thr Ile Trp Trp Leu Val Thr Phe Val Ala Pro Asn Gly Glu
           115                 120                 125
   Ala Tyr Phe Ser Ala Ala Leu Asn Ser Phe Ile His Val Ile Met Tyr
       130                 135                 140
   Gly Tyr Tyr Phe Leu Ser Ala Leu Gly Phe Lys Gln Val Ser Phe Ile
   145                 150                 155                 160
   Lys Phe Tyr Ile Thr Arg Ser Gln Met Thr Gln Phe Cys Met Met Ser
                   165                 170                 175
   Val Gln Ser Ser Trp Asp Met Tyr Ala Met Lys Val Leu Gly Arg Pro
               180                 185                 190
   Gly Tyr Pro Phe Phe Ile Thr Ala Leu Leu Trp Phe Tyr Met Trp Thr
           195                 200                 205
   Met Leu Gly Leu Phe Tyr Asn Phe Tyr Arg Lys Asn Ala Lys Leu Ala
       210                 215                 220
   Lys Gln Ala Lys Ala Asp Ala Ala Lys Glu Lys Ala Arg Lys
   225                 230                 235
<210> 42
   <211> 144
   <212> PRT
   <213> Caenorhabditis elegans
<400> 42
   Leu Tyr Asn Leu Gly Ile Thr Leu Leu Ser Ala Tyr Met Leu Ala Glu
    1               5                  10                  15
   Leu Ile Leu Ser Thr Trp Glu Gly Gly Tyr Asn Leu Gln Cys Gln Asp
               20                  25                  30
   Leu Thr Ser Ala Gly Glu Ala Asp Ile Arg Val Ala Lys Val Leu Trp
           35                  40                  45
   Trp Tyr Tyr Phe Ser Lys Ser Val Glu Phe Leu Asp Thr Ile Phe Phe
       50                  55                  60
   Val Leu Arg Lys Lys Thr Ser Gln Ile Thr Phe Leu His Val Tyr His
   65                  70                  75                  80
   His Ala Ser Met Phe Asn Ile Trp Trp Cys Val Leu Asn Trp Ile Pro
                   85                  90                  95
   Cys Gly Gln Ser Phe Phe Gly Pro Thr Leu Asn Ser Phe Ile His Ile
               100                 105                 110
   Leu Met Tyr Ser Tyr Tyr Gly Leu Ser Val Phe Pro Ser Met His Lys
           115                 120                 125
   Tyr Leu Trp Trp Lys Lys Tyr Leu Thr Gln Ala Gln Leu Val Gln Phe
       130                 135                 140
<210> 43
   <211> 241
   <212> PRT
   <213> Caenorhabditis elegans
<400> 43
   Leu Leu Ile Val Leu Ala Tyr Leu Val Thr Val Phe Val Gly Met Gln
    1               5                  10                  15
   Ile Met Lys Asn Phe Glu Arg Phe Glu Val Lys Thr Phe Ser Leu Leu
               20                  25                  30
   His Asn Phe Cys Leu Val Ser Ile Ser Ala Tyr Met Cys Gly Gly Ile
           35                  40                  45
   Leu Tyr Glu Ala Tyr Gln Ala Asn Tyr Gly Leu Phe Glu Asn Ala Ala
       50                  55                  60
   Asp His Thr Phe Lys Gly Leu Pro Met Ala Lys Met Ile Trp Leu Phe
   65                  70                  75                  80
   Tyr Phe Ser Lys Ile Met Glu Phe Val Asp Thr Met Ile Met Val Leu
                   85                  90                  95
   Lys Lys Asn Asn Arg Gln Ile Ser Phe Leu His Val Tyr His His Ser
               100                 105                 110
   Ser Ile Phe Thr Ile Trp Trp Leu Val Thr Phe Val Ala Pro Asn Gly
           115                 120                 125
   Glu Ala Tyr Phe Ser Ala Ala Leu Asn Ser Phe Ile His Val Ile Met
       130                 135                 140
   Tyr Gly Tyr Tyr Phe Leu Ser Ala Leu Gly Phe Lys Gln Val Ser Phe
   145                 150                 155                 160
   Ile Lys Phe Tyr Ile Thr Arg Ser Gln Met Thr Gln Phe Cys Met Met
                   165                 170                 175
   Ser Val Gln Ser Ser Trp Asp Met Tyr Ala Met Lys Val Leu Gly Arg
               180                 185                 190
   Pro Gly Tyr Pro Phe Phe Ile Thr Ala Leu Leu Trp Phe Tyr Met Trp
           195                 200                 205
   Thr Met Leu Gly Leu Phe Tyr Asn Phe Tyr Arg Lys Asn Ala Lys Leu
       210                 215                 220
   Ala Lys Gln Ala Lys Ala Asp Ala Ala Lys Glu Lys Ala Arg Lys Leu
   225                 230                 235                 240
   Gln
<210> 44
   <211> 217
   <212> PRT
   <213> Caenorhabditis elegans
<400> 44
   Ile Val Tyr Asn Phe Ser Leu Val Ile Leu Ser Leu Tyr Ile Val Tyr
    1               5                  10                  15
   Glu Phe Leu Met Ser Gly Trp Leu Ser Thr Tyr Thr Trp Arg Cys Asp
               20                  25                  30
   Pro Ile Asp Phe Ser Asn Ser Pro Glu Ala Leu Arg Met Val Arg Val
           35                  40                  45
   Ala Trp Leu Phe Met Leu Ser Lys Val Ile Glu Leu Met Asp Thr Val
       50                  55                  60
   Ile Phe Ile Leu Arg Lys Lys Asp Gly Gln Val Thr Phe Leu His Val
   65                  70                  75                  80
   Phe His His Ser Val Leu Pro Trp Ser Trp Trp Trp Gly Ile Lys Ile
                   85                  90                  95
   Ala Pro Gly Gly Met Gly Ser Phe His Ala Met Ile Asn Ser Ser Val
               100                 105                 110
   His Val Val Met Tyr Leu Tyr Tyr Gly Leu Ser Ala Leu Gly Pro Val
           115                 120                 125
   Ala Gln Pro Tyr Leu Trp Trp Lys Lys His Met Thr Ala Ile Gln Leu
       130                 135                 140
   Ile Gln Phe Val Leu Val Ser Leu His Ile Ser Gln Tyr Tyr Phe Met
   145                 150                 155                 160
   Pro Ser Cys Asn Tyr Gln Tyr Pro Val Ile Ile His Leu Ile Trp Met
                   165                 170                 175
   Tyr Gly Thr Ile Phe Phe Ile Leu Phe Ser Asn Phe Trp Tyr His Ser
               180                 185                 190
   Tyr Thr Lys Gly Lys Arg Leu Pro Arg Ala Val Gln Gln Asn Gly Ala
           195                 200                 205
   Pro Ala Thr Thr Lys Val Lys Ala Asn
       210                 215
<210> 45
   <211> 178
   <212> PRT
   <213> Caenorhabditis elegans
<400> 45
   Pro Thr Ile Val His His Thr Arg Gly Phe Leu Val Ala Val Glu Ser
    1               5                  10                  15
   Pro Leu Ala Arg Glu Leu Pro Leu Met Asn Pro Phe His Val Leu Leu
               20                  25                  30
   Ile Val Leu Ala Tyr Leu Val Thr Val Phe Val Gly Met Gln Ile Met
           35                  40                  45
   Lys Asn Phe Glu Arg Phe Glu Val Lys Thr Phe Ser Leu Leu His Asn
       50                  55                  60
   Phe Cys Leu Val Ser Ile Ser Ala Tyr Met Cys Gly Gly Ile Leu Tyr
   65                  70                  75                  80
   Glu Ala Tyr Gln Ala Asn Tyr Gly Leu Phe Glu Asn Ala Ala Asp His
                   85                  90                  95
   Thr Phe Lys Gly Leu Pro Met Ala Lys Met Ile Trp Leu Phe Tyr Phe
               100                 105                 110
   Ser Lys Ile Met Glu Phe Val Asp Thr Met Ile Met Val Leu Lys Lys
           115                 120                 125
   Asn Asn Arg Gln Ile Ser Phe Leu His Val Tyr His His Ser Ser Ile
       130                 135                 140
   Phe Thr Ile Trp Trp Leu Val Thr Phe Val Ala Pro Asn Gly Glu Ala
   145                 150                 155                 160
   Tyr Phe Ser Ala Ala Leu Asn Ser Phe Ile His Val Ile Met Tyr Gly
                   165                 170                 175
   Tyr Tyr
<210> 46
   <211> 148
   <212> PRT
   <213> Caenorhabditis elegans
<400> 46
   Asn Glu Val Asn Ala Phe Leu Asp Asn Met Phe Gly Pro Arg Asp Ser
    1               5                  10                  15
   Arg Val Arg Gly Trp Phe Leu Leu Asp Ser Tyr Leu Pro Thr Phe Ile
               20                  25                  30
   Leu Thr Ile Thr Tyr Leu Leu Ser Ile Trp Leu Gly Asn Lys Tyr Met
           35                  40                  45
   Lys Asn Arg Pro Ala Leu Ser Leu Arg Gly Ile Leu Thr Leu Tyr Asn
       50                  55                  60
   Leu Ala Ile Thr Leu Leu Ser Ala Tyr Met Leu Val Glu Leu Ile Leu
   65                  70                  75                  80
   Ser Ser Trp Glu Gly Gly Tyr Asn Leu Gln Cys Gln Asn Leu Asp Ser
                   85                  90                  95
   Ala Gly Glu Gly Asp Val Arg Val Ala Lys Val Leu Val Trp Tyr Tyr
               100                 105                 110
   Phe Ser Lys Leu Val Glu Phe Leu Asp Thr Ile Phe Phe Val Leu Arg
           115                 120                 125
   Lys Lys Ala Asn Gln Ile Thr Phe Leu His Val Tyr His His Ala Ser
       130                 135                 140
   Met Phe Asn Ile
   145
<210> 47
   <211> 302
   <212> PRT
   <213> Caenorhabditis elegans
<400> 47
   Phe Met Asp Leu Ala Thr Ala Ile Gly Val Arg Ala Ala Pro Tyr Val
    1               5                  10                  15
   Asp Pro Leu Glu Ala Ala Leu Val Ala Gln Ala Glu Lys Tyr Ile Pro
               20                  25                  30
   Thr Ile Val His His Thr Arg Gly Phe Leu Val Ala Val Glu Ser Pro
           35                  40                  45
   Leu Ala Arg Glu Leu Pro Leu Met Asn Pro Phe His Val Leu Leu Ile
       50                  55                  60
   Val Leu Ala Tyr Leu Val Thr Val Phe Val Gly Met Gln Ile Met Lys
   65                  70                  75                  80
   Asn Phe Glu Arg Phe Glu Val Lys Thr Phe Ser Leu Leu His Asn Phe
                   85                  90                  95
   Cys Leu Val Ser Ile Ser Ala Tyr Met Cys Gly Gly Ile Leu Tyr Glu
               100                 105                 110
   Ala Tyr Gln Ala Asn Tyr Gly Leu Phe Glu Asn Ala Ala Asp His Thr
           115                 120                 125
   Phe Lys Gly Leu Pro Met Ala Lys Met Ile Trp Leu Phe Tyr Phe Ser
       130                 135                 140
   Lys Ile Met Glu Phe Val Asp Thr Met Ile Met Val Leu Lys Lys Asn
   145                 150                 155                 160
   Asn Arg Gln Ile Ser Phe Leu His Val Tyr His His Ser Ser Ile Phe
                   165                 170                 175
   Thr Ile Trp Trp Leu Val Thr Phe Val Ala Pro Asn Gly Glu Ala Tyr
               180                 185                 190
   Phe Ser Ala Ala Leu Asn Ser Phe Ile His Val Ile Met Tyr Gly Tyr
           195                 200                 205
   Tyr Phe Leu Ser Ala Leu Gly Phe Lys Gln Val Ser Phe Ile Lys Phe
       210                 215                 220
   Tyr Ile Thr Arg Ser Gln Met Thr Gln Phe Cys Met Met Ser Val Gln
   225                 230                 235                 240
   Ser Ser Trp Asp Met Tyr Ala Met Lys Val Leu Gly Arg Pro Gly Tyr
                   245                 250                 255
   Pro Phe Phe Ile Thr Ala Leu Leu Trp Phe Tyr Met Trp Thr Met Leu
               260                 265                 270
   Gly Leu Phe Tyr Asn Phe Tyr Arg Lys Asn Ala Lys Leu Ala Lys Gln
           275                 280                 285
   Ala Lys Ala Asp Ala Ala Lys Glu Lys Ala Arg Lys Leu Gln
       290                 295                 300
<210> 48
   <211> 271
   <212> PRT
   <213> Caenorhabditis elegans
<400> 48
   Met Asp Thr Ser Met Asn Phe Ser Arg Gly Leu Lys Met Asp Leu Met
   1 5 10 15
   Gln Pro Tyr Asp Phe Glu Thr Phe Gln Asp Leu Arg Pro Phe Leu Glu
   20 25 30
   Glu Tyr Trp Val Ser Ser Phe Leu Ile Val Val Val Tyr Leu Leu Leu
   35 40 45
   Ile Val Val Gly Gln Thr Tyr Met Arg Thr Arg Lys Ser Phe Ser Leu
   50 55 60
   Gln Arg Pro Leu Ile Leu Trp Ser Phe Phe Leu Ala Ile Phe Ser Ile
   65 70 75 80
   Leu Gly Thr Leu Arg Met Trp Lys Phe Met Ala Thr Val Met Phe Thr
   85 90 95
   Val Gly Leu Lys Gln Thr Val Cys Phe Ala Ile Tyr Thr Asp Asp Ala
   100 105 110
   Val Val Arg Phe Trp Ser Phe Leu Phe Leu Leu Ser Lys Val Val Glu
   115 120 125
   Leu Gly Asp Thr Ala Phe Ile Ile Leu Arg Lys Arg Pro Leu Ile Phe
   130 135 140
   Val His Trp Tyr His His Ser Thr Val Leu Leu Phe Thr Ser Phe Gly
   145 150 155 160
   Tyr Lys Asn Lys Val Pro Ser Gly Gly Trp Phe Met Thr Met Asn Phe
   165 170 175
   Gly Val His Ser Val Met Tyr Thr Tyr Tyr Thr Met Lys Ala Ala Lys
   180 185 190
   Leu Lys His Pro Asn Leu Leu Pro Met Val Ile Thr Ser Leu Gln Ile
   195 200 205
   Leu Gln Met Val Leu Gly Thr Ile Phe Gly Ile Leu Asn Tyr Ile Trp
   210 215 220
   Arg Gln Glu Lys Gly Cys His Thr Thr Thr Glu His Phe Phe Trp Ser
   225 230 235 240
   Phe Met Leu Tyr Gly Thr Tyr Phe Ile Leu Phe Ala His Phe Phe His
   245 250 255
   Arg Ala Tyr Leu Arg Pro Lys Gly Lys Val Ala Ser Lys Ser Gln
   260 265 270
<210> 49
   <211> 265
   <212> PRT
   <213> Caenorhabditis elegans
<400> 49
   Thr Arg Gly Phe Leu Val Ala Val Glu Ser Pro Leu Ala Arg Glu Leu
    1               5                  10                  15
   Pro Leu Met Asn Pro Phe His Val Leu Leu Ile Val Leu Ala Tyr Leu
               20                  25                  30
   Val Thr Val Phe Val Gly Met Gln Ile Met Lys Asn Phe Glu Arg Phe
           35                  40                  45
   Glu Val Lys Thr Phe Ser Leu Leu His Asn Phe Cys Leu Val Ser Ile
       50                  55                  60
   Ser Ala Tyr Met Cys Gly Gly Ile Leu Tyr Glu Ala Tyr Gln Ala Asn
   65                  70                  75                  80
   Tyr Gly Leu Phe Glu Asn Ala Ala Asp His Thr Phe Lys Gly Leu Pro
                   85                  90                  95
   Met Ala Lys Met Ile Trp Leu Phe Tyr Phe Ser Lys Ile Met Glu Phe
               100                 105                 110
   Val Asp Thr Met Ile Met Val Leu Lys Lys Asn Asn Arg Gln Ile Ser
           115                 120                 125
   Phe Leu His Val Tyr His His Ser Ser Ile Phe Thr Ile Trp Trp Leu
       130                 135                 140
   Val Thr Phe Val Ala Pro Asn Gly Glu Ala Tyr Phe Ser Ala Ala Leu
   145                 150                 155                 160
   Asn Ser Phe Ile His Val Ile Met Tyr Gly Tyr Tyr Phe Leu Ser Ala
                   165                 170                 175
   Leu Gly Phe Lys Gln Val Ser Phe Ile Lys Phe Tyr Ile Thr Arg Ser
               180                 185                 190
   Gln Met Thr Gln Phe Cys Met Met Ser Val Gln Ser Ser Trp Asp Met
           195                 200                 205
   Tyr Ala Met Lys Val Leu Gly Arg Pro Gly Tyr Pro Phe Phe Ile Thr
       210                 215                 220
   Ala Leu Leu Trp Phe Tyr Met Trp Thr Met Leu Gly Leu Phe Tyr Asn
   225                 230                 235                 240
   Phe Tyr Arg Lys Asn Ala Lys Leu Ala Lys Gln Ala Lys Ala Asp Ala
                   245                 250                 255
   Ala Lys Glu Lys Ala Arg Lys Leu Gln
               260                 265
<210> 50
   <211> 265
   <212> PRT
   <213> Caenorhabditis elegans
<400> 50
   Ala Thr His Gly Pro Lys Asn Phe Pro Asp Ala Glu Gly Arg Lys Phe
    1               5                  10                  15
   Phe Ala Asp His Phe Asp Val Thr Ile Gln Ala Ser Ile Leu Tyr Met
               20                  25                  30
   Val Val Val Phe Gly Thr Lys Trp Phe Met Arg Asn Arg Gln Pro Phe
           35                  40                  45
   Gln Leu Thr Ile Pro Leu Asn Ile Trp Asn Phe Ile Leu Ala Ala Phe
       50                  55                  60
   Ser Ile Ala Gly Ala Val Lys Met Thr Pro Glu Phe Phe Gly Thr Ile
   65                  70                  75                  80
   Ala Asn Lys Gly Ile Val Ala Ser Tyr Cys Lys Val Phe Asp Phe Thr
                   85                  90                  95
   Lys Gly Glu Asn Gly Tyr Trp Val Trp Leu Phe Met Ala Ser Lys Leu
               100                 105                 110
   Phe Glu Leu Val Asp Thr Ile Phe Leu Val Leu Arg Lys Arg Pro Leu
           115                 120                 125
   Met Phe Leu His Trp Tyr His His Ile Leu Thr Met Ile Tyr Ala Trp
       130                 135                 140
   Tyr Ser His Pro Leu Thr Pro Gly Phe Asn Arg Tyr Gly Ile Tyr Leu
   145                 150                 155                 160
   Asn Phe Val Val His Ala Phe Met Tyr Ser Tyr Tyr Phe Leu Arg Ser
                   165                 170                 175
   Met Lys Ile Arg Val Pro Gly Phe Ile Ala Gln Ala Ile Thr Ser Leu
               180                 185                 190
   Gln Ile Val Gln Phe Ile Ile Ser Cys Ala Val Leu Ala His Leu Gly
           195                 200                 205
   Tyr Leu Met His Phe Thr Asn Ala Asn Cys Asp Phe Glu Pro Ser Val
       210                 215                 220
   Phe Lys Leu Ala Val Phe Met Asp Thr Thr Tyr Leu Ala Leu Phe Val
   225                 230                 235                 240
   Asn Phe Phe Leu Gln Ser Tyr Val Leu Arg Gly Gly Lys Asp Lys Tyr
                   245                 250                 255
   Lys Ala Val Pro Lys Lys Lys Asn Asn
               260                 265
<210> 51
   <211> 317
   <212> PRT
   <213> Caenorhabditis elegans
<400> 51
   Met Ala Ala Ala Ile Leu Asp Lys Val Asn Phe Gly Ile Asp Gln Pro
    1               5                  10                  15
   Phe Gly Ile Lys Leu Asp Thr Tyr Phe Ala Gln Ala Tyr Glu Leu Val
               20                  25                  30
   Thr Gly Lys Ser Ile Asp Ser Phe Val Phe Gln Glu Gly Val Thr Pro
           35                  40                  45
   Leu Ser Thr Gln Arg Glu Val Ala Met Trp Thr Ile Thr Tyr Phe Val
       50                  55                  60
   Val Ile Phe Gly Gly Arg Gln Ile Met Lys Ser Gln Asp Ala Phe Lys
   65                  70                  75                  80
   Leu Lys Pro Leu Phe Ile Leu His Asn Phe Leu Leu Thr Ile Ala Ser
                   85                  90                  95
   Gly Ser Leu Leu Leu Leu Phe Ile Glu Asn Leu Val Pro Ile Leu Ala
               100                 105                 110
   Arg Asn Gly Leu Phe Tyr Ala Ile Cys Asp Asp Gly Ala Trp Thr Gln
           115                 120                 125
   Arg Leu Glu Leu Leu Tyr Tyr Leu Asn Tyr Leu Val Lys Tyr Trp Glu
       130                 135                 140
   Leu Ala Asp Thr Val Phe Leu Val Leu Lys Lys Lys Pro Leu Glu Phe
   145                 150                 155                 160
   Leu His Tyr Phe His His Ser Met Thr Met Val Leu Cys Phe Val Gln
                   165                 170                 175
   Leu Gly Gly Tyr Thr Ser Val Ser Trp Val Pro Ile Thr Leu Asn Leu
               180                 185                 190
   Thr Val His Val Phe Met Tyr Tyr Tyr Tyr Met Arg Ser Ala Ala Gly
           195                 200                 205
   Val Arg Ile Trp Trp Lys Gln Tyr Leu Thr Thr Leu Gln Ile Val Gln
       210                 215                 220
   Phe Val Leu Asp Leu Gly Phe Ile Tyr Phe Cys Ala Tyr Thr Tyr Phe
   225                 230                 235                 240
   Ala Phe Thr Tyr Phe Pro Trp Ala Pro Asn Val Gly Lys Cys Ala Gly
                   245                 250                 255
   Thr Glu Gly Ala Ala Leu Phe Gly Cys Gly Leu Leu Ser Ser Tyr Leu
               260                 265                 270
   Leu Leu Phe Ile Asn Phe Tyr Arg Ile Thr Tyr Asn Ala Lys Ala Lys
           275                 280                 285
   Ala Ala Lys Glu Arg Gly Ser Asn Phe Thr Pro Lys Thr Val Lys Ser
       290                 295                 300
   Gly Gly Ser Pro Lys Lys Pro Ser Lys Ser Lys His Ile
   305                 310                 315
<210> 52
   <211> 288
   <212> PRT
   <213> Caenorhabditis elegans
<400> 52
   Met Ala Gln His Pro Leu Val Gln Arg Leu Leu Asp Val Lys Phe Asp
    1               5                  10                  15
   Thr Lys Arg Phe Val Ala Ile Ala Thr His Gly Pro Lys Asn Phe Pro
               20                  25                  30
   Asp Ala Glu Gly Arg Lys Phe Phe Ala Asp His Phe Asp Val Thr Ile
           35                  40                  45
   Gln Ala Ser Ile Leu Tyr Met Val Val Val Phe Gly Thr Lys Trp Phe
       50                  55                  60
   Met Arg Asn Arg Gln Pro Phe Gln Leu Thr Ile Pro Leu Asn Ile Trp
   65                  70                  75                  80
   Asn Phe Ile Leu Ala Ala Phe Ser Ile Ala Gly Ala Val Lys Met Thr
                   85                  90                  95
   Pro Glu Phe Phe Gly Thr Ile Ala Asn Lys Gly Ile Val Ala Ser Tyr
               100                 105                 110
   Cys Lys Val Phe Asp Phe Thr Lys Gly Glu Asn Gly Tyr Trp Val Trp
           115                 120                 125
   Leu Phe Met Ala Ser Lys Leu Phe Glu Leu Val Asp Thr Ile Phe Leu
       130                 135                 140
   Val Leu Arg Lys Arg Pro Leu Met Phe Leu His Trp Tyr His His Ile
   145                 150                 155                 160
   Leu Thr Met Ile Tyr Ala Trp Tyr Ser His Pro Leu Thr Pro Gly Phe
                   165                 170                 175
   Asn Arg Tyr Gly Ile Tyr Leu Asn Phe Val Val His Ala Phe Met Tyr
               180                 185                 190
   Ser Tyr Tyr Phe Leu Arg Ser Met Lys Ile Arg Val Pro Gly Phe Ile
           195                 200                 205
   Ala Gln Ala Ile Thr Ser Leu Gln Ile Val Gln Phe Ile Ile Ser Cys
       210                 215                 220
   Ala Val Leu Ala His Leu Gly Tyr Leu Met His Phe Thr Asn Ala Asn
   225                 230                 235                 240
   Cys Asp Phe Glu Pro Ser Val Phe Lys Leu Ala Val Phe Met Asp Thr
                   245                 250                 255
   Thr Tyr Leu Ala Leu Phe Val Asn Phe Phe Leu Gln Ser Tyr Val Leu
               260                 265                 270
   Arg Gly Gly Lys Asp Lys Tyr Lys Ala Val Pro Lys Lys Lys Asn Asn
           275                 280                 285
<210> 53
   <211> 282
   <212> PRT
   <213> Drosophila melanogaster
<400> 53
   Ala Ala Leu Val Ala Gln Ala Glu Lys Tyr Ile Pro Thr Ile Val His
    1              5                  10                  15
   His Thr Arg Gly Phe Leu Val Ala Val Glu Ser Pro Leu Ala Arg Glu
               20                  25                  30
   Leu Pro Leu Met Asn Pro Phe His Val Leu Leu Ile Val Leu Ala Tyr
           35                  40                  45
   Leu Val Thr Val Phe Val Gly Met Gln Ile Met Lys Asn Phe Glu Arg
       50                  55                  60
   Phe Glu Val Lys Thr Phe Ser Leu Leu His Asn Phe Cys Leu Val Ser
   65                  70                  75                  80
   Ile Ser Ala Tyr Met Cys Gly Gly Ile Leu Tyr Glu Ala Tyr Gln Ala
                   85                  90                  95
   Asn Tyr Gly Leu Phe Glu Asn Ala Ala Asp His Thr Phe Lys Gly Leu
               100                 105                 110
   Pro Met Ala Lys Met Ile Trp Leu Phe Tyr Phe Ser Lys Ile Met Glu
           115                 120                 125
   Phe Val Asp Thr Met Ile Met Val Leu Lys Lys Asn Asn Arg Gln Ile
       130                 135                 140
   Ser Phe Leu His Val Tyr His His Ser Ser Ile Phe Thr Ile Trp Trp
   145                 150                 155                 160
   Leu Val Thr Phe Val Ala Pro Asn Gly Glu Ala Tyr Phe Ser Ala Ala
                   165                 170                 175
   Leu Asn Ser Phe Ile His Val Ile Met Tyr Gly Tyr Tyr Phe Leu Ser
               180                 185                 190
   Ala Leu Gly Phe Lys Gln Val Ser Phe Ile Lys Phe Tyr Ile Thr Arg
           195                 200                 205
   Ser Gln Met Thr Gln Phe Cys Met Met Ser Val Gln Ser Ser Trp Asp
       210                 215                 220
   Met Tyr Ala Met Lys Val Leu Gly Arg Pro Gly Tyr Pro Phe Phe Ile
   225                 230                 235                 240
   Thr Ala Leu Leu Trp Phe Tyr Met Trp Thr Met Leu Gly Leu Phe Tyr
                   245                 250                 255
   Asn Phe Tyr Arg Lys Asn Ala Lys Leu Ala Lys Gln Ala Lys Ala Asp
               260                 265                 270
   Ala Ala Lys Glu Lys Ala Arg Lys Leu Gln
           275                 280
<210> 54
   <211> 278
   <212> PRT
   <213> Drosophila melanogaster
<400> 54
   Pro Thr Lys Met Ile Asn Met Asp Ile Ser Val Thr Pro Asn Tyr Ser
    1              5                  10                  15
   Tyr Ile Phe Asp Phe Glu Asn Asp Phe Ile His Gln Arg Thr Arg Lys
               20                  25                  30
   Trp Met Leu Glu Asn Trp Thr Trp Val Phe Tyr Tyr Cys Gly Ile Tyr
           35                  40                  45
   Met Leu Val Ile Phe Gly Gly Gln His Phe Met Gln Asn Arg Pro Arg
       50                  55                  60
   Phe Gln Leu Arg Gly Pro Leu Ile Ile Trp Asn Thr Leu Leu Ala Met
   65                  70                  75                  80
   Phe Ser Ile Met Gly Ala Ala Arg Thr Ala Pro Glu Leu Ile His Val
                   85                  90                  95
   Leu Arg His Tyr Gly Leu Phe His Ser Val Cys Val Pro Ser Tyr Ile
               100                 105                 110
   Glu Gln Asp Arg Val Cys Gly Phe Trp Thr Trp Leu Phe Val Leu Ser
           115                 120                 125
   Lys Leu Pro Glu Leu Gly Asp Thr Ile Phe Ile Val Leu Arg Lys Gln
       130                 135                 140
   Pro Leu Ile Phe Leu His Trp Tyr His His Ile Thr Val Leu Ile Tyr
   145                 150                 155                 160
   Ser Trp Phe Ser Tyr Thr Glu Tyr Thr Ser Ser Ala Arg Trp Phe Ile
                   165                 170                 175
   Val Met Asn Tyr Cys Val His Ser Val Met Tyr Ser Tyr Tyr Ala Leu
               180                 185                 190
   Lys Ala Ala Arg Phe Asn Pro Pro Arg Phe Ile Ser Met Ile Ile Thr
           195                 200                 205
   Ser Leu Gln Leu Ala Gln Met Ile Ile Gly Cys Ala Ile Asn Val Trp
       210                 215                 220
   Ala Asn Gly Phe Leu Lys Thr His Gly Thr Xaa Ser Cys His Ile Ser
   225                 230                 235                 240
   Gln Arg Asn Ile Asn Leu Ser Ile Ala Met Tyr Ser Ser Tyr Phe Val
                   245                 250                 255
   Leu Phe Ala Arg Phe Phe Tyr Lys Ala Tyr Leu Ala Pro Gly Gly His
               260                 265                 270
   Lys Ser Arg Arg Met Ala
           275
<210> 55
   <211> 286
   <212> PRT
   <213> Drosophila melanogaster
<400> 55
   Val Thr Gly Lys Ser Ile Asp Ser Phe Val Phe Gln Glu Gly Val Thr
   1 5 10 15
   Pro Leu Ser Thr Gln Arg Glu Val Ala Met Trp Thr Ile Thr Tyr Phe
   20 25 30
   Val Val Ile Phe Gly Gly Arg Gln Ile Met Lys Ser Gln Asp Ala Phe
   35 40 45
   Lys Leu Lys Pro Leu Phe Ile Leu His Asn Phe Leu Leu Thr Ile Ala
   50 55 60
   Ser Gly Ser Leu Leu Leu Leu Phe Ile Glu Asn Leu Val Pro Ile Leu
   65 70 75 80
   Ala Arg Asn Gly Leu Phe Tyr Ala Ile Cys Asp Asp Gly Ala Trp Thr
   85 90 95
   Gln Arg Leu Glu Leu Leu Tyr Tyr Leu Asn Tyr Leu Val Lys Tyr Trp
   100 105 110
   Glu Leu Ala Asp Thr Val Phe Leu Val Leu Lys Lys Lys Pro Leu Glu
   115 120 125
   Phe Leu His Tyr Phe His His Ser Met Thr Met Val Leu Cys Phe Val
   130 135 140
   Gln Leu Gly Gly Tyr Thr Ser Val Ser Trp Val Pro Ile Thr Leu Asn
   145 150 155 160
   Leu Thr Val His Val Phe Met Tyr Tyr Tyr Tyr Met Arg Ser Ala Ala
   165 170 175
   Gly Val Arg Ile Trp Trp Lys Gln Tyr Leu Thr Thr Leu Gln Ile Val
   180 185 190
   Gln Phe Val Leu Asp Leu Gly Phe Ile Tyr Phe Cys Ala Tyr Thr Tyr
   195 200 205
   Phe Ala Phe Thr Tyr Phe Pro Trp Ala Pro Asn Val Gly Lys Cys Ala
   210 215 220
   Gly Thr Glu Gly Ala Ala Leu Phe Gly Cys Gly Leu Leu Ser Ser Tyr
   225 230 235 240
   Leu Leu Leu Phe Ile Asn Phe Tyr Arg Ile Thr Tyr Asn Ala Lys Ala
   245 250 255
   Lys Ala Ala Lys Glu Arg Gly Ser Asn Phe Thr Pro Lys Thr Val Lys
   260 265 270
   Ser Gly Gly Ser Pro Lys Lys Pro Ser Lys Ser Lys His Ile
   275 280 285
<210> 56
   <211> 261
   <212> PRT
   <213> Drosophila melanogaster
<400> 56
   Ile Phe Asp Phe Glu Asn Asp Phe Ile His Gln Arg Thr Arg Lys Trp
   1 5 10 15
   Met Leu Glu Asn Trp Thr Trp Val Phe Tyr Tyr Cys Gly Ile Tyr Met
   20 25 30
   Leu Val Ile Phe Gly Gly Gln His Phe Met Gln Asn Arg Pro Arg Phe
   35 40 45
   Gln Leu Arg Gly Pro Leu Ile Ile Trp Asn Thr Leu Leu Ala Met Phe
   50 55 60
   Ser Ile Met Gly Ala Ala Arg Thr Ala Pro Glu Leu Ile His Val Leu
   65 70 75 80
   Arg His Tyr Gly Leu Phe His Ser Val Cys Val Pro Ser Tyr Ile Glu
   85 90 95
   Gln Asp Arg Val Cys Gly Phe Trp Thr Trp Leu Phe Val Leu Ser Lys
   100 105 110
   Leu Pro Glu Leu Gly Asp Thr Ile Phe Ile Val Leu Arg Lys Gln Pro
   115 120 125
   Leu Ile Phe Leu His Trp Tyr His His Ile Thr Val Leu Ile Tyr Ser
   130 135 140
   Trp Phe Ser Tyr Thr Glu Tyr Thr Ser Ser Ala Arg Trp Phe Ile Val
   145 150 155 160
   Met Asn Tyr Cys Val His Ser Val Met Tyr Ser Tyr Tyr Ala Leu Lys
   165 170 175
   Ala Ala Arg Phe Asn Pro Pro Arg Phe Ile Ser Met Ile Ile Thr Ser
   180 185 190
   Leu Gln Leu Ala Gln Met Ile Ile Gly Cys Ala Ile Asn Val Trp Ala
   195 200 205
   Asn Gly Phe Leu Lys Thr His Gly Thr Xaa Ser Cys His Ile Ser Gln
   210 215 220
   Arg Asn Ile Asn Leu Ser Ile Ala Met Tyr Ser Ser Tyr Phe Val Leu
   225 230 235 240
   Phe Ala Arg Phe Phe Tyr Lys Ala Tyr Leu Ala Pro Gly Gly His Lys
   245 250 255
   Ser Arg Arg Met Ala
   260
<210> 57
   <211> 299
   <212> PRT
   <213> Human elongase
<400> 57
   Met Glu His Phe Asp Ala Ser Leu Ser Thr Tyr Phe Lys Ala Leu Leu
   1 5 10 15
   Gly Pro Arg Asp Thr Arg Val Lys Gly Trp Phe Leu Leu Asp Asn Tyr
   20 25 30
   Ile Pro Thr Phe Ile Cys Ser Val Ile Tyr Leu Leu Ile Val Trp Leu
   35 40 45
   Gly Pro Lys Tyr Met Arg Asn Lys Gln Pro Phe Ser Cys Arg Gly Ile
   50 55 60
   Leu Val Val Tyr Asn Leu Gly Leu Thr Leu Leu Ser Leu Tyr Met Phe
   65                  70                  75                  80
   Cys Glu Leu Val Thr Gly Val Trp Glu Gly Lys Tyr Asn Phe Phe Cys
                   85                  90                  95
   Gln Gly Thr Arg Thr Ala Gly Glu Ser Asp Met Lys Ile Ile Arg Val
               100                 105                 110
   Leu Trp Trp Tyr Tyr Phe Ser Lys Leu Ile Glu Phe Met Asp Thr Phe
           115                 120                 125
   Phe Phe Ile Leu Arg Lys Asn Asn His Gln Ile Thr Val Leu His Val
       130                 135                 140
   Tyr His His Ala Ser Met Leu Asn Ile Trp Trp Phe Val Met Asn Trp
   145                 150                 155                 160
   Val Pro Cys Gly His Ser Tyr Phe Gly Ala Thr Leu Asn Ser Phe Ile
                   165                 170                 175
   His Val Leu Met Tyr Ser Tyr Tyr Gly Leu Ser Ser Val Pro Ser Met
               180                 185                 190
   Arg Pro Tyr Leu Trp Trp Lys Lys Tyr Ile Thr Gln Gly Gln Leu Leu
           195                 200                 205
   Gln Phe Val Leu Thr Ile Ile Gln Thr Ser Cys Gly Val Ile Trp Pro
       210                 215                 220
   Cys Thr Phe Pro Leu Gly Trp Leu Tyr Phe Gln Ile Gly Tyr Met Ile
   225                 230                 235                 240
   Ser Leu Ile Ala Leu Phe Thr Asn Phe Tyr Ile Gln Thr Tyr Asn Lys
                   245                 250                 255
   Lys Gly Ala Ser Arg Arg Lys Asp His Leu Lys Asp His Gln Asn Gly
               260                 265                 270
   Ser Met Ala Ala Val Asn Gly His Thr Asn Ser Phe Ser Pro Leu Glu
           275                 280                 285
   Asn Asn Val Lys Pro Arg Lys Leu Arg Lys Asp
       290                 295
<210> 58
   <211> 288
   <212> PRT
   <213> Caenorhabditis elegans
<400> 58
   Met Ala Gln His Pro Leu Val Gln Arg Leu Leu Asp Val Lys Phe Asp
    1               5                  10                  15
   Thr Lys Arg Phe Val Ala Ile Ala Thr His Gly Pro Lys Asn Phe Pro
               20                  25                  30
   Asp Ala Glu Gly Arg Lys Phe Phe Ala Asp His Phe Asp Val Thr Ile
           35                  40                  45
   Gln Ala Ser Ile Leu Tyr Met Val Val Val Phe Gly Thr Lys Trp Phe
       50                  55                  60
   Met Arg Asn Arg Gln Pro Phe Gln Leu Thr Ile Pro Leu Asn Ile Trp
   65                  70                  75                  80
   Asn Phe Ile Leu Ala Ala Phe Ser Ile Ala Gly Ala Val Lys Met Thr
                   85                  90                  95
   Pro Glu Phe Phe Gly Thr Ile Ala Asn Lys Gly Ile Val Ala Ser Tyr
               100                 105                 110
   Cys Lys Val Phe Asp Phe Thr Lys Gly Glu Asn Gly Tyr Trp Val Trp
           115                 120                 125
   Leu Phe Met Ala Ser Lys Leu Phe Glu Leu Val Asp Thr Ile Phe Leu
       130                 135                 140
   Val Leu Arg Lys Arg Pro Leu Met Phe Leu His Trp Tyr His His Ile
   145                 150                 155                 160
   Leu Thr Met Ile Tyr Ala Trp Tyr Ser His Pro Leu Thr Pro Gly Phe
                   165                 170                 175
   Asn Arg Tyr Gly Ile Tyr Leu Asn Phe Val Val His Ala Phe Met Tyr
               180                 185                 190
   Ser Tyr Tyr Phe Leu Arg Ser Met Lys Ile Arg Val Pro Gly Phe Ile
           195                 200                 205
   Ala Gln Ala Ile Thr Ser Leu Gln Ile Val Gln Phe Ile Ile Ser Cys
       210                 215                 220
   Ala Val Leu Ala His Leu Gly Tyr Leu Met His Phe Thr Asn Ala Asn
   225                 230                 235                 240
   Cys Asp Phe Glu Pro Ser Val Phe Lys Leu Ala Val Phe Met Asp Thr
                   245                 250                 255
   Thr Tyr Leu Ala Leu Phe Val Asn Phe Phe Leu Gln Ser Tyr Val Leu
               260                 265                 270
   Arg Gly Gly Lys Asp Lys Tyr Lys Ala Val Pro Lys Lys Lys Asn Asn
           275                 280                 285
<210> 59
   <211> 798
   <212> DNA
   <213> Human elongase
<400> 59
   atgaacatgt cagtgttgac tttacaagaa tatgaattcg aaaagcagtt caacgagaat      60
   gaagccatcc aatggatgca ggaaaactgg aagaaatctt tcctgttttc tgctctgtat     120
   gctgccttta tattcggtgg tcggcaccta atgaataaac gagcaaagtt tgaactgagg     180
   aagccattag tgctctggtc tctgaccctt gcagtcttca gtatattcgg tgctcttcga     240
   actggtgctt atatggtgta cattttgatg accaaaggcc tgaagcagtc agtttgtgac     300
   cagggttttt acaatggacc tgtcagcaaa ttctgggctt atgcatttgt gctaagcaaa     360
   gcacccgaac taggagatac aatattcatt attctgagga agcagaagct gatcttcctg     420
   cactggtatc accacatcac tgtgctcctg tactcttggt actcctacaa agacatggtt     480
   gccgggggag gttggttcat gactatgaac tatggcgtgc acgccgtgat gtactcttac     540
   tatgccttgc gggcggcagg tttccgagtc tcccggaagt ttgccatgtt catcaccttg     600
   tcccagatca ctcagatgct gatgggctgt gtggttaact acctggtctt ctgctggatg     660
   cagcatgacc agtgtcactc tcactttcag aacatcttct ggtcctcact catgtacctc     720
   agctaccttg tgctcttctg ccatttcttc tttgaggcct acatcggcaa aatgaggaaa     780
   acaacgaaag ctgaatag                                                   798
<210> 60
   <211> 265
   <212> PRT
   <213> Human elongase
<400> 60
   Met Asn Met Ser Val Leu Thr Leu Gln Glu Tyr Glu Phe Glu Lys Gln
   1               5                   10                  15
   Phe Asn Glu Asn Glu Ala Ile Gln Trp Met Gln Glu Asn Trp Lys Lys
               20                  25                  30
   Ser Phe Leu Phe Ser Ala Leu Tyr Ala Ala Phe Ile Phe Gly Gly Arg
           35                  40                  45
   His Leu Met Asn Lys Arg Ala Lys Phe Glu Leu Arg Lys Pro Leu Val
       50                  55                  60
   Leu Trp Ser Leu Thr Leu Ala Val Phe Ser Ile Phe Gly Ala Leu Arg
   65                  70                  75                  80
   Thr Gly Ala Tyr Met Val Tyr Ile Leu Met Thr Lys Gly Leu Lys Gln
                   85                  90                  95
   Ser Val Cys Asp Gln Gly Phe Tyr Asn Gly Pro Val Ser Lys Phe Trp
               100                 105                 110
   Ala Tyr Ala Phe Val Leu Ser Lys Ala Pro Glu Leu Gly Asp Thr Ile
           115                 120                 125
   Phe Ile Ile Leu Arg Lys Gln Lys Leu Ile Phe Leu His Trp Tyr His
       130                 135                 140
   His Ile Thr Val Leu Leu Tyr Ser Trp Tyr Ser Tyr Lys Asp Met Val
   145                 150                 155                 160
   Ala Gly Gly Gly Trp Phe Met Thr Met Asn Tyr Gly Val His Ala Val
                           165                     170                     175
   Met Tyr Ser Tyr Tyr Ala Leu Arg Ala Ala Gly Phe Arg Val Ser Arg
                   180                     185                     190
   Lys Phe Ala Met Phe Ile Thr Leu Ser Gln Ile Thr Gln Met Leu Met
           195                     200                     205
   Gly Cys Val Val Asn Tyr Leu Val Phe Cys Trp Met Gln His Asp Gln
       210                     215                     220
   Cys His Ser His Phe Gln Asn Ile Phe Trp Ser Ser Leu Met Tyr Leu
   225                     230                     235                     240
   Ser Tyr Leu Val Leu Phe Cys His Phe Phe Phe Glu Ala Tyr Ile Gly
                   245                     250                     255
   Lys Met Arg Lys Thr Thr Lys Ala Glu
               260                     265
<210> 61
   <211> 82
   <212> PRT
   <213> Unknown
<220>
   <223> Description of Unknown sequence: jojoba KCS; Genbank Accession # U37088
<400> 61
   Ala Thr Leu Pro Asn Phe Lys Ser Ser Ile Asn Leu His His Val Lys
    1               5                  10                  15
   Leu Gly Tyr His Tyr Leu Ile Ser Asn Ala Leu Phe Leu Val Phe Ile
               20                  25                  30
   Pro Leu Leu Gly Leu Ala Ser Ala His Leu Ser Ser Phe Ser Ala His
           35                  40                  45
   Asp Ser Leu Leu Phe Asp Leu Leu Arg Arg Asn Leu Leu Pro Val Val
       50                  55                  60
   Val Cys Ser Phe Leu Phe Val Leu Leu Ala Thr Leu His Phe Leu Thr
   65                  70                  75                  80
   Arg Pro
<210> 62
   <211> 80
   <212> PRT
   <213> Saccharomyces cerevisiae
<400> 62
   Ser Thr Leu Pro Pro Val Leu Tyr Ala Ile Thr Ala Tyr Tyr Val Ile
    1               5                  10                  15
   Ile Phe Gly Gly Arg Phe Leu Leu Ser Lys Ser Lys Pro Phe Lys Leu
               20                  25                  30
   Asn Gly Leu Phe Gln Leu His Asn Leu Val Leu Thr Ser Leu Ser Leu
           35                  40                  45
   Thr Leu Leu Leu Leu Met Val Glu Gln Leu Val Pro Ile Ile Val Gln
       50                  55                  60
   His Gly Leu Tyr Phe Ala Ile Cys Asn Ile Gly Ala Trp Thr Gln Pro
   65                  70                  75                  80
<210> 63
   <211> 18
   <212> DNA
   <213> Artificial Sequence
<220>
   <223> Description of Artificial sequence: R0352 primer
<400> 63
   acgcgtacgt aaagcttg 18
<210> 64
   <211> 36
   <212> DNA
   <213> Artificial Sequence
<220>
   <223> Description of Artificial sequence: RO514 Primer
<400> 64
   ggctatggat ccatgaattc actcgttact caatat 36
<210> 65
   <211> 33
   <212> DNA
   <213> Artificial Sequence
<220>
   <223> Description of artificial sequence: RO 515 Primer
<400> 65
   cctgccaagc ttttaccttt ttcttctgtg ttg 33
<210> 66
   <211> 21
   <212> DNA
   <213> Artificial Sequence
<220>
   <223> Description of Artificial sequence: RO 541 Primer
<400> 66
   gattactagc agctgtaata c 21
<210> 67
   <211> 21
   <212> DNA
   <213> Artificial Sequence
<220>
   <223> Description of Artificial sequence: RO 540 Primer
<400> 67
   gtgaatgtaa cggtgacata a 21
<210> 68
   <211> 28
   <212> PRT
   <213> Homo sapiens
<400> 68
   Asp Thr Ile Phe Ile Ile Leu Arg Lys Gln Lys Leu Ile Phe Leu His
    1               5                  10                  15
   Trp Tyr His His Ile Thr Val Leu Leu Tyr Ser Trp
               20                  25
<210> 69
   <211> 29
   <212> DNA
   <213> Artificial Sequence
<220>
   <223> Description of Artificial sequence: RO 719 primer
<400> 69
   ggttctccca tggaacattt tgatgcatc 29
<210> 70
   <211> 30
   <212> DNA
   <213> Artificial Sequence
<220>
   <223> Description of Artificial sequence: RO 720 primer
<400> 70
   ggtttcaaag ctttgacttc aatccttccg 30
<210> 71
   <211> 36
   <212> DNA
   <213> Artificial Sequence
<220>
   <223> Description of Artificial sequence: RO 738 primer
<400> 71
   aatcaggaat tcatggctca gcatccgctc gttcaa 36
<210> 72
   <211> 36
   <212> DNA
   <213> Artificial Sequence
<220>
   <223> Description of Artificial sequence: RO 739 primer
<400> 72
   ccgcttgtcg acttagttgt tcttcttctt tggcac 36
<210> 73
   <211> 30
   <212> DNA
   <213> Artificial Sequence
<220>
   <223> Description of Artificial sequence: RP 735 primer
<400> 73
   cctcctgaat tccaacacta ttcagctttc 30
<210> 74
   <211> 20
   <212> DNA
   <213> Artificial Sequence
<220>
   <223> Description of Artificial sequence: RO 73 primer
<400> 74
   taatacgact cactataggg 20
<210> 75
   <211> 18
   <212> DNA
   <213> Artificial Sequence
<220>
   <223> Description of Artificial sequence: RO 728 primer
<400> 75
   gagactttga gcggttcg 18
<210> 76
   <211> 20
   <212> DNA
   <213> Artificial Sequence
<220>
   <223> Description of Artificial sequence: RO 730 primer
<400> 76
   tctctgctgc gttgaactcg 20
<210> 77
   <211> 19
   <212> DNA
   <213> Artificial Sequence
<220>
   <223> Description of Artificial sequence: RO 729 primer
<400> 77
   aaagctcttg acctcgaac 19
<210> 78
   <211> 20
   <212> DNA
   <213> Artificial Sequence
<220>
   <223> Description of Artificial sequence: RO 731 primer
<400> 78
   aacttgatga acgacacgtg 20